# EUROPEAN PATENT APPLICATION

(11) **EP 4 219 497 A1**
(43) Date of publication of application: **02.08.2023**
(21) Application number: 23150862.3
(22) Date of filing: 10.01.2023
(51) Int. Cl.: C07D 471/16, C07D 519/00, C07F 11/00, H10K 50/00

(54) **HETEROCYCLIC COMPOUND, LIGHT-EMITTING DEVICE INCLUDING THE HETEROCYCLIC COMPOUND AND ELECTRONIC APPARATUS INCLUDING THE LIGHT-EMITTING DEVICE**

(30) Priority: 26.01.2022 KR 20220011841
(71) Applicant: Samsung Display Co., Ltd., Yongin-si, Gyeonggi-do 17113 (KR); Industry-Academic Cooperation Foundation Gyeongsang National University, Jinju-si, Gyeongsangnam-do 52828 (KR)
(72) Inventor: CHO, Sejin, 17113 Yongin-si (KR); KIM, Yunhi, 52828 Jinju-si (KR); KWON, Soonki, 52828 Jinju-si (KR); KIM, Mikyung, 17113 Yongin-si (KR); LEE, Kwanhee, 17113 Yongin-si (KR); HA, Yeonhee, 17113 Yongin-si (KR); LEE, Gyeongseok, 52828 Jinju-si (KR)
(74) Representative: Russell, Tim

(57) **Abstract**

Embodiments provide a heterocyclic compound, a light-emitting device that includes the heterocyclic compound, and an electronic apparatus that includes the light-emitting device. The light-emitting device includes a first electrode, a second electrode facing the first electrode, an interlayer between the first electrode and the second electrode and including an emission layer, and at least one of the heterocyclic compound. The heterocyclic compound is represented by Formula 1, which is explained in the specification:

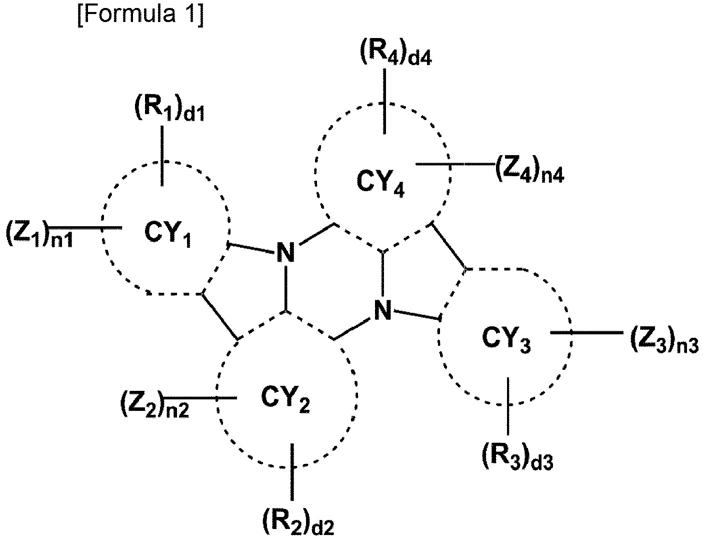

## Description

### CROSS-REFERENCE TO RELATED APPLICATION(S)

This application claims priority to and benefits of Korean Patent Application No. 10-2022-0011841 under 35 U.S.C. §119, filed on January 26, 2022, in the Korean Intellectual Property Office, the entire contents of which are incorporated herein by reference.

### BACKGROUND

### 1. Technical Field

Embodiments relate to a heterocyclic compound, a light-emitting device including the same, and an electronic apparatus including the light-emitting device.

### 2. Description of the Related Art

Organic light-emitting devices may include a first electrode located on a substrate, and a hole transport region, an emission layer, an electron transport region, and a second electrode sequentially stacked on the first electrode. Holes provided from the first electrode move toward the emission layer through the hole transport region, and electrons provided from the second electrode move toward the emission layer through the electron transport region. Carriers, such as holes and electrons, recombine in the emission layer to produce excitons. The excitons may transition from an excited state to a ground state, thus generating light.

It is to be understood that this background of the technology section is, in part, intended to provide useful background for understanding the technology. However, this background of the technology section may also include ideas, concepts, or recognitions that were not part of what was known or appreciated by those skilled in the pertinent art prior to a corresponding effective filing date of the subject matter disclosed herein.

### SUMMARY

The invention is defined by the claims.

Embodiments include a heterocyclic compound, a light-emitting device comprising the same, and an electronic apparatus comprising the light-emitting device.

Additional aspects will be set forth in part in the description which follows and, in part, will be apparent from the description, or may be learned by practice of the embodiments of the disclosure.

According to embodiments, a heterocyclic compound may be represented by Formula 1:

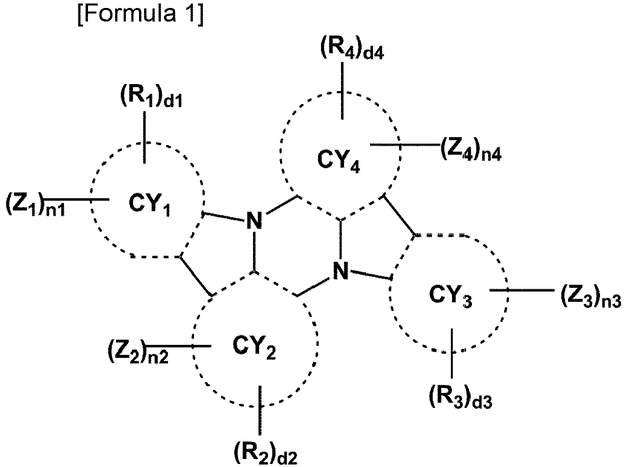

In Formula 1,
CY₁ to CY₄ are each independently a C₃-C₆₀ carbocyclic group or a C₁-C₆₀ heterocyclic group,
Z₁ is *-(L₁)ₐ₁-E₁,
Z₂ is *-(L₂)ₐ₂-E₂,
Z₃ is *-(L₃)ₐ₃-E₃,
Z₄ is *-(L₄)ₐ₄-E₄,
L₁ to L₄ are each independently a single bond, a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
a1 to a4 are each independently an integer from 1 to 3,
E₁ to E₄ are each independently (i) a C₃-C₆₀ carbocyclic group unsubstituted or substituted with at least one R₁₀ₐ or (ii) a group containing at least one heteroatom,
n1 to n4 are each independently an integer from 0 to 3,
the sum of n1 to n4 is 1 or greater,
when E₁ to E₄ are each a benzene group, the sum of n2 and n4 is 1 or 2,
* indicates a binding site to a neighboring atom,
R₁ to R₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂),
d1 to d4 are each independently an integer from 0 to 10,
two or more groups of R₁ to R₄ are optionally bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ,
each R₁₀ₐ is independently:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q31)(Q32),
wherein Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof; or
a C₇-C₆₀ arylalkyl group; or a C₂-C₆₀ heteroarylalkyl group.

In an embodiment, CY₁ to CY₄ may each independently be a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzotriazole group a benzoxazole group, a benzothiazole group, a benzoxadiazole group, a benzothiadiazole group, a 5,6,7,8-tetrahydroisoquinoline group, or a 5,6,7,8-tetrahydroquinoline group.

In an embodiment, L₁ to L₄ may each independently be a single bond, or a group represented by one of Formulae 2-1 to 2-40, which are explained below.

In an embodiment, in Formula 1, the group containing at least one heteroatom may be: a C₁-C₆₀ heterocyclic group unsubstituted or substituted with at least one R₁₀ₐ; or -Si(Ar₁)(Ar₂)(Ar₃), -N(Ar₁)(Ar₂), -B(Ar₁)(Ar₂), -C(=O)(Ar₁), -S(=O)₂(Ar₁), or - P(=O)(Ar₁)(Ar₂), wherein Ar₁ to Ar₃ may each independently be hydrogen, deuterium, - F, -Cl, -Br, -I, hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₅-C₃₀ carbocyclic group that is unsubstituted or substituted with at least one R₁₀ₐ, or a C₁-C₃₀ heterocyclic group that is unsubstituted or substituted with at least one R₁₀ₐ; and R₁₀ₐ is the same as described in Formula 1.

In an embodiment, E₁ to E₄ may each independently be: a T1 group, a cyclic group in which two or more T1 groups are condensed with each other, a T2 group, a cyclic group in which two or more T2 groups are condensed with each other, or a cyclic group in which at least one T2 group is condensed with at least one T1 group, each unsubstituted or substituted with at least one R₁₀ₐ; a group represented by one of Formulae 3-1 to 3-9; or -Si(Ar₁)(Ar₂)(Ar₃), -N(Ar₁)(Ar₂), -B(Ar₁)(Ar₂), -C(=O)(Ar₁), - S(=O)₂(Ar₁), or -P(=O)(Ar₁)(Ar₂), wherein the T1 group and the T2 group are each explained below, Formulae 3-1 to 3-9 are explained below, Ar₁ to Ar₃ may each independently be a C₃-C₆₀ carbocyclic group unsubstituted or substituted with at least one R₁₀ₐ, or a C₁-C₆₀ heterocyclic group unsubstituted or substituted with at least one R₁₀ₐ, and R₁₀ₐ is the same as described in Formula 1.

In an embodiment, E₁ to E₄ are each independently be: *-N(Ar₁)(Ar₂) or *-P(=O)(Ar₁)(Ar₂); or a group represented by one of Formulae 4-1 to 4-87, wherein Formulae 4-1 to 4-87 are explained below, Ar₁ and Ar₂ may each independently be hydrogen, deuterium, -F, -Cl, -Br, -I, hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₅-C₃₀ carbocyclic group that is unsubstituted or substituted with at least one R₁₀ₐ, or a C₁-C₃₀ heterocyclic group that is unsubstituted or substituted with at least one R₁₀ₐ, and R₁₀ₐ is the same as described in Formula 1.

In an embodiment, in Formula 1, a moiety represented by 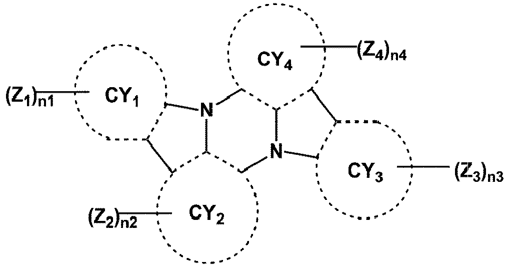 may be a moiety represented by one of Formulae 1-1 to 1-27, which are explained below.

In an embodiment, the sum of n1 to n4 may be an integer from 1 to 4.

In an embodiment, n1 to n4 may satisfy at least one of Condition 1 and Condition 2, which are explained below.

In an embodiment, n1 may be 0, n2 may be 0, n3 may be 0, and n4 may be an integer of 1 or greater; or n1 may be 0, n2 may be an integer of 1 or greater, n3 may be 0, and n4 may be 0; or n1 may be 0, n2 may be an integer of 1 or greater, n3 may be 0, and n4 may be an integer of 1 or greater; or n1 may be an integer of 1 or greater, n2 may be an integer of 1 or greater, n3 may be an integer of 1 or greater, and n4 may be an integer of 1 or greater; or n1 may be an integer of 1 or greater, n2 may be 0, n3 may be 0, and n4 may be 0; or n1 may be 0, n2 may be 0, n3 may be an integer of 1 or greater, and n4 may be 0; or n1 may be an integer of 1 or greater, n2 may be 0, n3 may be an integer of 1 or greater, and n4 may be 0; or n1 may be an integer of 1 or greater, n2 may be an integer of 1 or greater, n3 may be 0, and n4 may be 0; or n1 may be an integer of 1 or greater, n2 may be an integer of 1 or greater, n3 may be an integer of 1 or greater, and n4 may be 0.

In an embodiment, the heterocyclic compound may be one of Compounds 1 to 111, which are explained below.

According to embodiments, a light-emitting device comprises a first electrode, a second electrode facing the first electrode, an interlayer between the first electrode and the second electrode and comprising an emission layer, and at least one heterocyclic compound described herein.

In an embodiment, the first electrode may be an anode; the second electrode may be a cathode; the interlayer may comprise the at least one heterocyclic compound, a hole transport region between the first electrode and the emission layer, and an electron transport region between the emission layer and the second electrode; the hole transport region may comprise a hole injection layer, a hole transport layer, an emission auxiliary layer, an electron-blocking layer, or any combination thereof; and the electron transport region may comprise a hole-blocking layer, an electron transport layer, an electron injection layer, or any combination thereof.

In an embodiment, the emission layer may comprise the at least one heterocyclic compound.

In an embodiment, the emission layer may further comprise a host, and an amount of the at least one heterocyclic compound may be in a range of about 0.01 parts by weight to about 49.99 parts by weight, based on 100 parts by weight of the emission layer.

In an embodiment, the emission layer may further comprise an anthracene-based compound.

In an embodiment, the emission layer may be configured to emit light having a maximum emission wavelength in a range of about 400 nm to about 500 nm.

According to embodiments, an electronic apparatus may comprise the light-emitting device.

In an embodiment, the electronic apparatus may further comprise a thin-film transistor, wherein the thin-film transistor may comprise a source electrode and a drain electrode, and the first electrode of the light-emitting device may be electrically connected to at least one of the source electrode and the drain electrode.

In an embodiment, the electronic apparatus may further comprise a color filter, a quantum dot color conversion layer, a color conversion layer, a touch screen layer, a polarizing layer, or any combination thereof.

It is to be understood that the embodiments above are described in a generic and explanatory sense only and not for the purpose of limitation, and the disclosure is not limited to the embodiments described above.

### BRIEF DESCRIPTION OF THE DRAWINGS

The above and other aspects and features of the disclosure will be more apparent by describing in detail embodiments thereof with reference to the accompanying drawings, in which:
FIG. 1 is a schematic cross-sectional view of a light-emitting device according to an embodiment;
FIG. 2 is a schematic cross-sectional view of an electronic apparatus according to an embodiment; and
FIG. 3 is a schematic cross-sectional view of an electronic apparatus according to another embodiment.

### DETAILED DESCRIPTION OF THE EMBODIMENTS

The disclosure will now be described more fully hereinafter with reference to the accompanying drawings, in which embodiments are shown. This disclosure may, however, be embodied in different forms and should not be construed as limited to the embodiments set forth herein. Rather, these embodiments are provided so that this disclosure will be thorough and complete, and will fully convey the scope of the disclosure to those skilled in the art.

In the drawings, the sizes, thicknesses, ratios, and dimensions of the elements may be exaggerated for ease of description and for clarity. Like numbers refer to like elements throughout.

In the description, it will be understood that when an element (or region, layer, part, etc.) is referred to as being "on", "connected to", or "coupled to" another element, it can be directly on, connected to, or coupled to the other element, or one or more intervening elements may be present therebetween. In a similar sense, when an element (or region, layer, part, etc.) is described as "covering" another element, it can directly cover the other element, or one or more intervening elements may be present therebetween.

In the description, when an element is "directly on," "directly connected to," or "directly coupled to" another element, there are no intervening elements present. For example, "directly on" may mean that two layers or two elements are disposed without an additional element such as an adhesion element therebetween.

As used herein, the expressions used in the singular such as "a," "an," and "the," are intended to include the plural forms as well, unless the context clearly indicates otherwise.

As used herein, the term "and/or" includes any and all combinations of one or more of the associated listed items. For example, "A and/or B" may be understood to mean "A, B, or A and B." The terms "and" and "or" may be used in the conjunctive or disjunctive sense and may be understood to be equivalent to "and/or".

In the specification and the claims, the term "at least one of" is intended to include the meaning of "at least one selected from the group of" for the purpose of its meaning and interpretation. For example, "at least one of A and B" may be understood to mean "A, B, or A and B." When preceding a list of elements, the term, "at least one of," modifies the entire list of elements and does not modify the individual elements of the list.

It will be understood that, although the terms first, second, etc. may be used herein to describe various elements, these elements should not be limited by these terms. These terms are only used to distinguish one element from another element. Thus, a first element could be termed a second element without departing from the teachings of the disclosure. Similarly, a second element could be termed a first element, without departing from the scope of the disclosure.

The spatially relative terms "below", "beneath", "lower", "above", "upper", or the like, may be used herein for ease of description to describe the relations between one element or component and another element or component as illustrated in the drawings. It will be understood that the spatially relative terms are intended to encompass different orientations of the device in use or operation, in addition to the orientation depicted in the drawings. For example, in the case where a device illustrated in the drawing is turned over, the device positioned "below" or "beneath" another device may be placed "above" another device. Accordingly, the illustrative term "below" may include both the lower and upper positions. The device may also be oriented in other directions and thus the spatially relative terms may be interpreted differently depending on the orientations.

The terms "about" or "approximately" as used herein is inclusive of the stated value and means within an acceptable range of deviation for the recited value as determined by one of ordinary skill in the art, considering the measurement in question and the error associated with measurement of the recited quantity (i.e., the limitations of the measurement system). For example, "about" may mean within one or more standard deviations, or within ±20%, ±10%, or ±5% of the stated value.

It should be understood that the terms "comprises," "comprising," "includes," "including," "have," "having," "contains," "containing," and the like are intended to specify the presence of stated features, integers, steps, operations, elements, components, or combinations thereof in the disclosure, but do not preclude the presence or addition of one or more other features, integers, steps, operations, elements, components, or combinations thereof.

Unless otherwise defined or implied herein, all terms (including technical and scientific terms) used have the same meaning as commonly understood by those skilled in the art to which this disclosure pertains. It will be further understood that terms, such as those defined in commonly used dictionaries, should be interpreted as having a meaning that is consistent with their meaning in the context of the relevant art and should not be interpreted in an ideal or excessively formal sense unless clearly defined in the specification.

An aspect provides a heterocyclic compound represented by Formula 1:

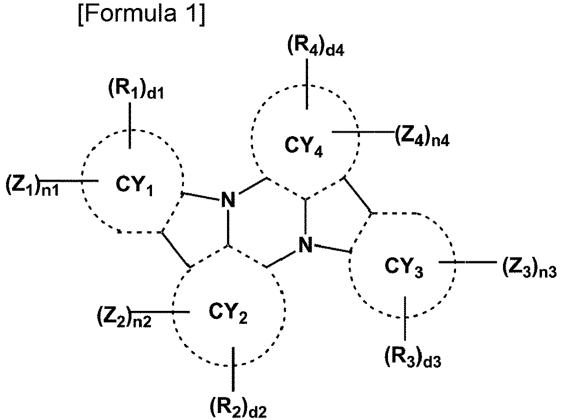

In Formula 1, CY₁ to CY₄ are each independently a C₃-C₆₀ carbocyclic group or a C₁-C₆₀ heterocyclic group.

In an embodiment, CY₁ to CY₄ may each independently be a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzotriazole group a benzoxazole group, a benzothiazole group, a benzoxadiazole group, a benzothiadiazole group, a 5,6,7,8-tetrahydroisoquinoline group, or a 5,6,7,8-tetrahydroquinoline group.

In Formula 1, Z₁ is *-(L₁)ₐ₁-E₁, Z₂ is *-(L₂)ₐ₂-E₂, Z₃ is *-(L₃)ₐ₃-E₃, and Z₄ is *-(L₄)ₐ₄-E4.

In Formula 1, L₁ to L₄ are each independently a single bond, a C₅-C₃₀ carbocyclic group unsubstituted or substituted with at least one R₁₀ₐ, or a C₁-C₃₀ heterocyclic group unsubstituted or substituted with at least one R₁₀ₐ.

In embodiments, L₁ to L₄ are each independently be:
a single bond; or
a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluoren-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzotriazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ.

In an embodiment, L₁ to L₄ may each independently be:
a single bond; or
a group represented by one of Formulae 2-1 to 2-40:

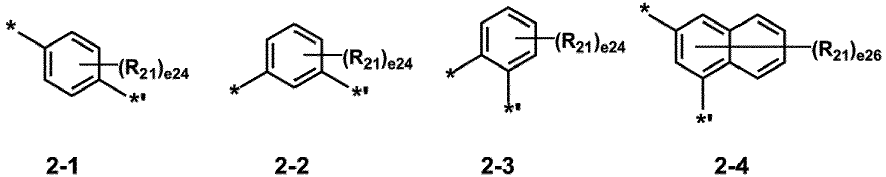

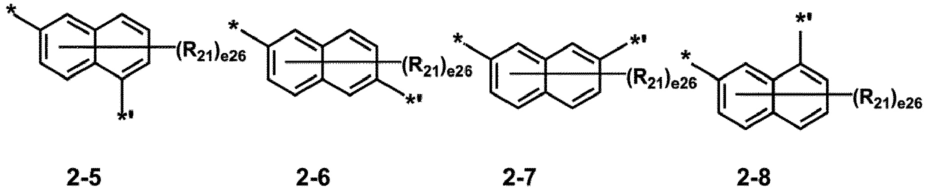

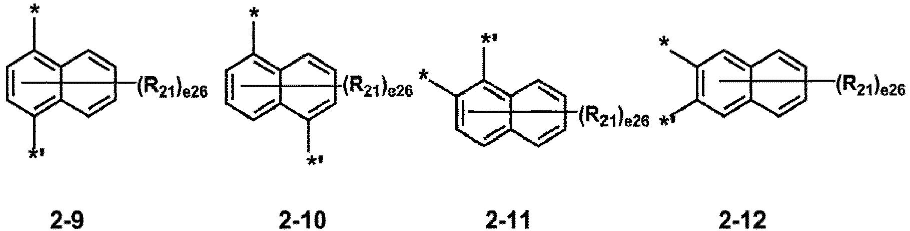

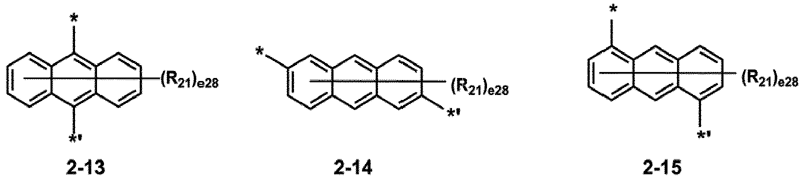

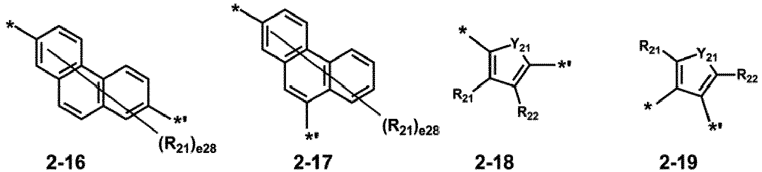

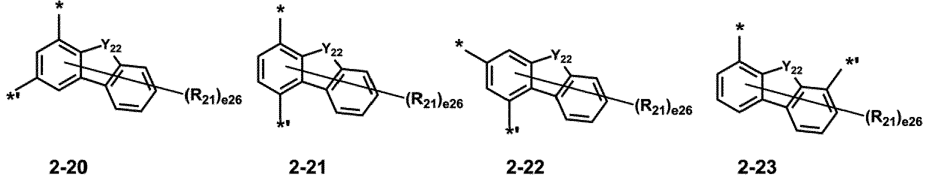

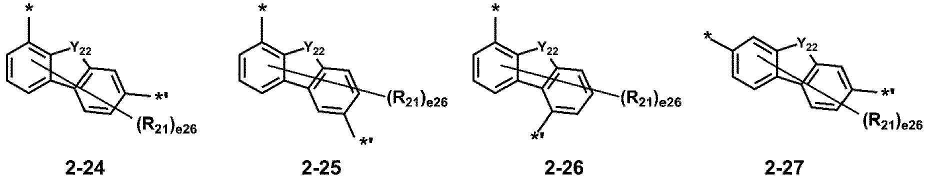

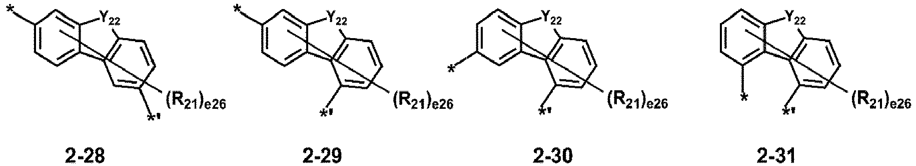

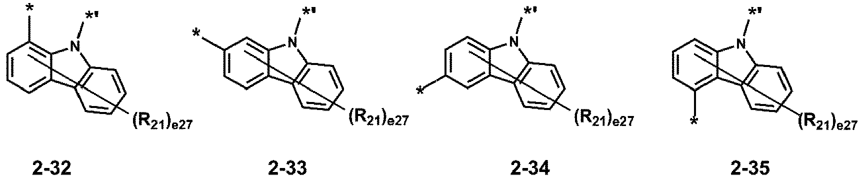

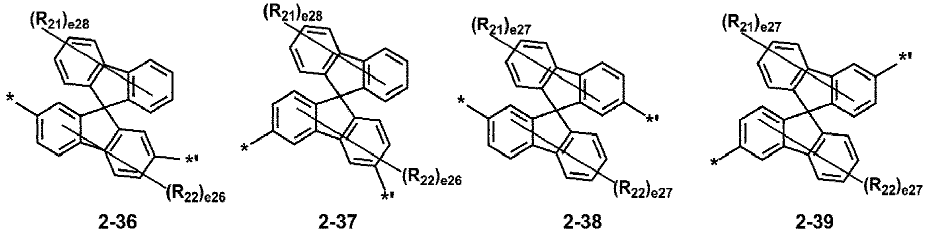

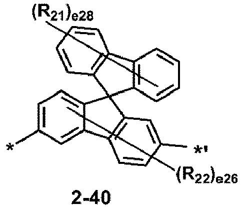

In Formulae 2-1 to 2-40 ,
Y₂₁ may be O or S,
Y₂₂ may be O, S, N(R₂₃), C(R₂₃)(R₂₄), or Si(R₂₃)(R₂₄),
R₂₁ to R₂₄ may each independently be hydrogen, deuterium, -F, -Cl, -Br, -I, a hydroxyl group, a cyano group, a nitro group, an amidino group, a hydrazino group, a hydrazono group, -CF₃, -CF₂H, -CFH₂, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilollyl group, -Si(Q₄₁)(Q₄₂)(Q₄₃), -N(Q₄₁)(Q₄₂), or -B(Q₄₁)(Q₄₂),
e24 may be an integer from 0 to 4,
e26 may be an integer from 0 to 6,
e27 may be an integer from 0 to 7,
e28 may be an integer from 0 to 8,
Q₄₁ to Q₄₃ may each independently be a C₁-C₁₀ alkyl group, a C₁-C₁₀ alkoxy group, a phenyl group, a biphenyl group, a terphenyl group, or a naphthyl group, and
* and *' each indicate a binding site to a neighboring atom.

In Formula 1, a1 to a4 are each independently an integer from 1 to 3.

When a1 is 2 or greater, L₁(s) in the number of a1 may be identical to or different from each other.

When a2 is 2 or greater, L₂(s) in the number of a2 may be identical to or different from each other.

When a3 is 2 or greater, L₃(s) in the number of a3 may be identical to or different from each other.

When a4 is 2 or greater, L₄(s) in the number of a4 may be identical to or different from each other.

In Formula 1, E₁ to E₄ are each independently (i) a C₃-C₆₀ carbocyclic group unsubstituted or substituted with at least one R₁₀ₐ or (ii) a group containing at least one heteroatom.

The term "heteroatom" as used herein may be any element other than a carbon atom or a hydrogen atom. For example, the heteroatom may be boron (B), nitrogen (N), phosphorus (P), oxygen (O), sulfur (S), selenium (Se), or silicon (Si).

In an embodiment, the group containing at least one heteroatom may include boron (B), nitrogen (N), phosphorus (P), oxygen (O), sulfur (S), selenium (Se), silicon (Si), or any combination thereof.

In Formula 1, n1 to n4 are each independently an integer from 0 to 3, and the sum of n1 to n4 is 1 or more.

In Formula 1, when E₁ to E₄ are each a benzene group, the sum of n2 and n4 is 1 or 2.

In an embodiment, n1 may be an integer of 2 or more, and Z₁(s) in the number of n1 may be identical to or different from each other;
n2 may be an integer of 2 or more, and Z₂(s) in the number of n2 may be identical to or different from each other;
n3 may be an integer of 2 or more, and Z₃ in the number of n3 may be identical to or different from each other; or
n4 may be an integer of 2 or more, and Z₄(s) in the number of n4 may be identical to or different from each other.

In an embodiment, the group containing at least one heteroatom may be: a C₁-C₆₀ heterocyclic group unsubstituted or substituted with at least one R₁₀ₐ; or
-Si(Ar₁)(Ar₂)(Ar₃), -N(Ar₁)(Ar₂), -B(Ar₁)(Ar₂), -C(=O)(Ar₁), -S(=O)₂(Ar₁), or - P(=O)(Ar₁)(Ar₂),
wherein Ar₁ to Ar₃ may each independently be: hydrogen, deuterium, -F, -Cl, - Br, -I, hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₅-C₃₀ carbocyclic group that is unsubstituted or substituted with at least one R₁₀ₐ, or a C₁-C₃₀ heterocyclic group that is unsubstituted or substituted with at least one R₁₀ₐ, and
R₁₀ₐ is the same as described herein.

In embodiments, E₁ to E₄ may each independently be:
a T1 group, a cyclic group in which two or more T1 groups are condensed with each other, a T2 group, a cyclic group in which two or more T2 groups are condensed with each other, or a cyclic group in which at least one T2 group is condensed with at least one T1 group, each unsubstituted or substituted with at least one R₁₀ₐ;
a group represented by one of Formulae 3-1 to 3-9; or
-Si(Ar₁)(Ar₂)(Ar₃), -N(Ar₁)(Ar₂), -B(Ar₁)(Ar₂), -C(=O)(Ar₁), -S(=O)₂(Ar₁), or - P(=O)(Ar₁)(Ar₂),
wherein the T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or a bicyclo[2.2.1]heptane) group, a norbornene group, a bicyclo[1.1.1]pentane group, a bicyclo[2.1.1]hexane group, a bicyclo[2.2.2]octane group, or a benzene group,
the T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group, and
Ar₁ to Ar₃ may each independently be a C₃-C₆₀ carbocyclic group unsubstituted or substituted with at least one R₁₀ₐ, or a C₁-C₆₀ heterocyclic group unsubstituted or substituted with at least one R₁₀ₐ:

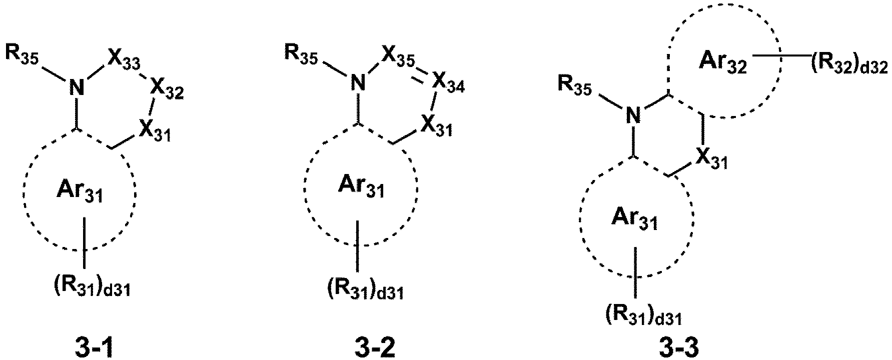

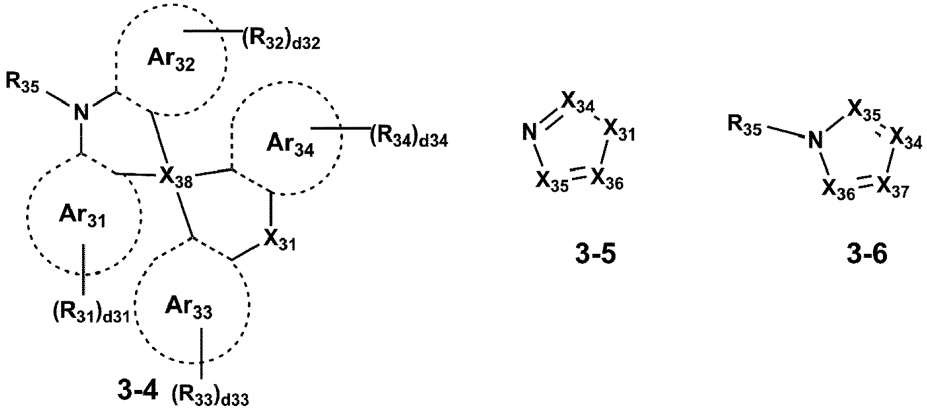

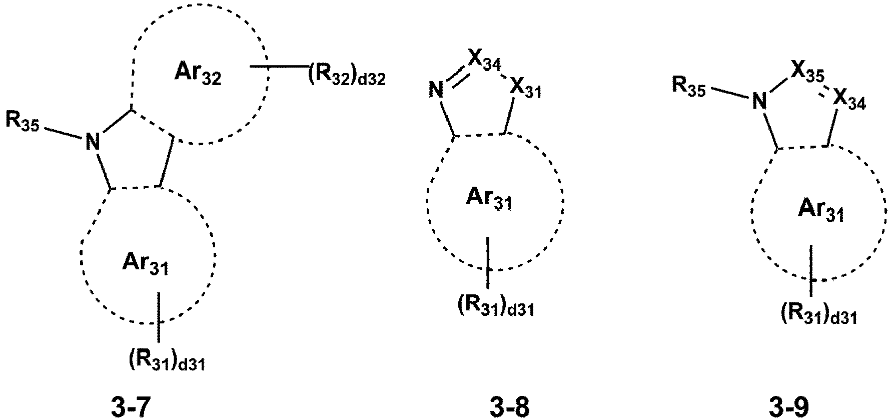

In Formulae 3-1 to 3-9,
Ar₃₁ to Ar₃₄ may each independently be a C₃-C₆₀ carbocyclic group or a C₁-C₆₀ heterocyclic group,
X₃₁ may be N(R₃₁ₐ), C(R₃₁ₐ)(R_{31b}), Si(R₃₁ₐ)(R_{31b}), O, S, or Se,
X₃₂ may be N(R₃₂ₐ), C(R₃₂ₐ)(R_{32b}), Si(R₃₂ₐ)(R_{32b}), O, S, or Se,
X₃₃ may be N(R₃₃ₐ), C(R₃₃ₐ)(R_{33b}), Si(R₃₃ₐ)(R_{33b}), O, S, or Se,
X₃₄ may be N or C(R₃₄ₐ),
X₃₅ may be N or C(R₃₅ₐ),
X₃₆ may be N or C(R₃₆ₐ),
X₃₇ may be N or C(R₃₇ₐ),
X₃₈ may be C or Si,
R₃₁, R₃₂, R₃₃, R₃₄, R₃₅, R₃₁ₐ, R_{31b}, R₃₂ₐ, R_{32b}, R₃₃ₐ, R_{33b}, R₃₄ₐ, R₃₅ₐ, R₃₆ₐ, and R₃₇ₐ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂),
d31 to d34 may each independently be an integer from 0 to 10,
one of R₃₁(s) in the number of d31, R₃₂(s) in the number of d32, R₃₃(s) in the number of d33, R₃₄(s) in the number of d34, R₃₅, R₃₁ₐ, R_{31b}, R₃₂ₐ, R_{32b}, R₃₃ₐ, R_{33b}, and R₃₄ₐ to R₃₇ₐ indicates a binding site to a neighboring atom,
two or more neighboring groups of R₃₁, R₃₂, R₃₃, R₃₄, R₃₅, R₃₁ₐ, R_{31b}, R₃₂ₐ, R_{32b}, R₃₃ₐ, R_{33b}, R₃₄ₐ, R₃₅ₐ, R₃₆ₐ and R₃₇ₐ may optionally be bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ, and
R₁₀ₐ is the same as described herein.

In embodiments, E₁ to E₄ may each independently be:
a cyclopropane group, a cyclobutane group, a cyclopentane group, a cyclohexane group, a cycloheptane group, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, a 1,2,3,4-tetrahydronaphthalene group, a pyrrole group, a thiophene group, a furan group, an indole group, a benzoindole group, a naphthoindole group, an isoindole group, a benzoisoindole group, a naphthoisoindole group, a benzosilole group, a benzogermole group, a benzothiophene group, a benzofuran group, a benzoborole group, a benzophosphine group, a benzoselenophene group, a carbazole group, a dibenzosilole group, a dibenzogermole group, a dibenzothiophene group, a dibenzofuran group, a dibenzoselenophene group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzotriazole group, a benzoxazole group, a benzoisoxazole group, a benzothiazole group, a benzoisothiazole group, a benzoxadiazole group, a benzothiadiazole, a 5,6,7,8-tetrahydroisoquinoline group, a 5,6,7,8-tetrahydroquinoline, a dithianopyrrole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, or an indenoanthracene group, each unsubstituted or substituted with at least one R₁₀ₐ;
a group represented by one of Formulae 3-1 to 3-8; or
-N(Ar₁)(Ar₂) or -P(=O)(Ar₁)(Ar₂),
wherein Formulae 3-1 to 3-8 and Ar₁ and Ar₂ are each the same as described herein.

In embodiments, E₁ to E₄ may each independently be:
*-N(Ar₁)(Ar₂) or *-P(=O)(Ar₁)(Ar₂); or
a group represented by one of Formulae 4-1 to 4-87,
wherein Ar₁ and Ar₂ may each independently be hydrogen, deuterium, -F, -Cl, - Br, -I, hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₅-C₃₀ carbocyclic group that is unsubstituted or substituted with at least one R₁₀ₐ, or a C₁-C₃₀ heterocyclic group that is unsubstituted or substituted with at least one R₁₀ₐ, and
R₁₀ₐ is the same as described herein:

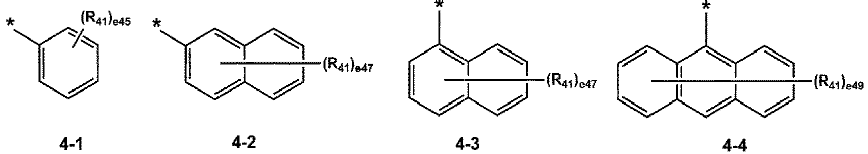

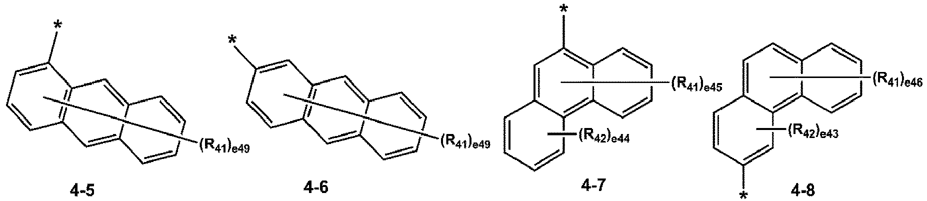

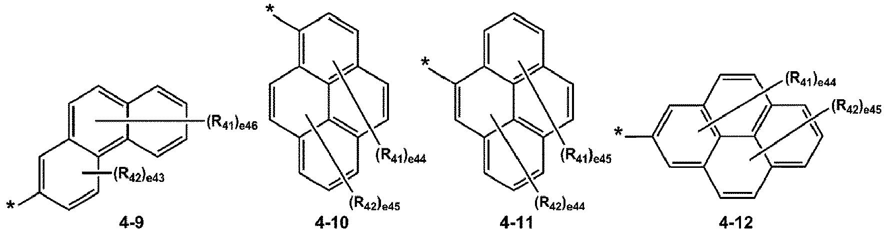

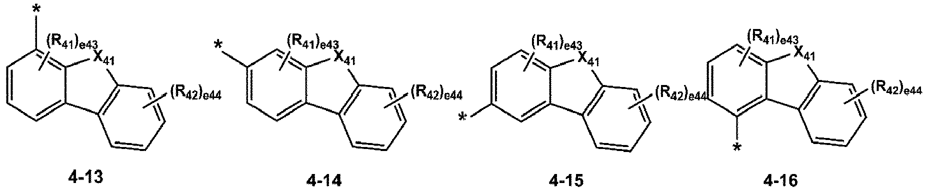

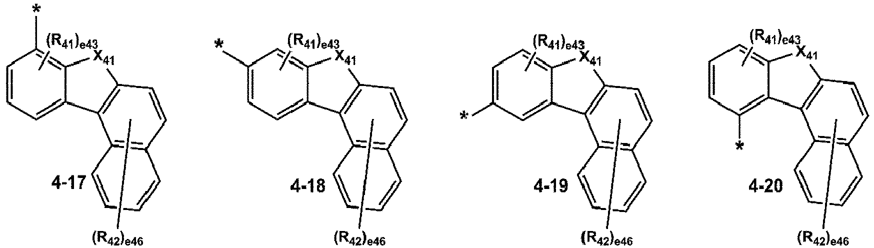

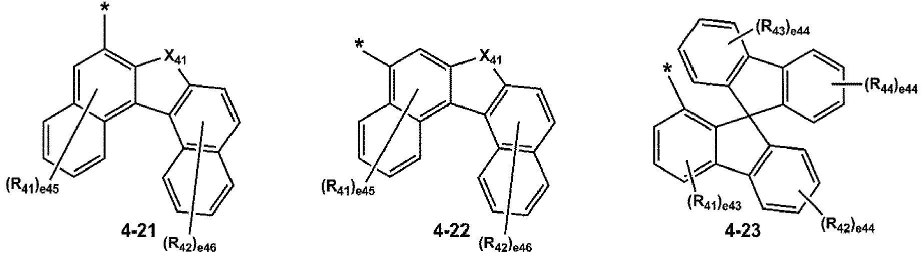

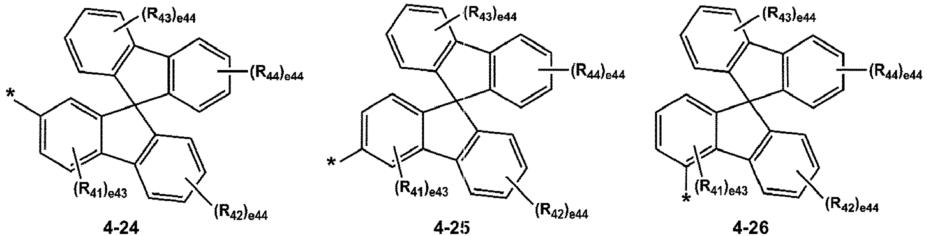

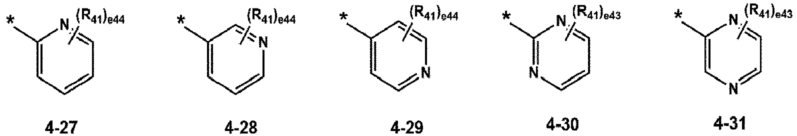

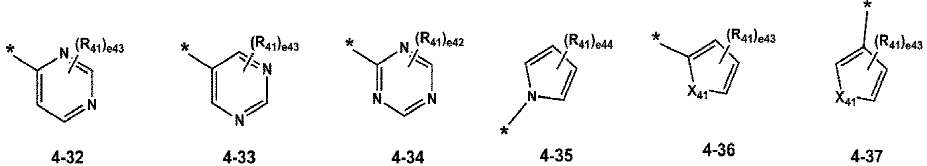

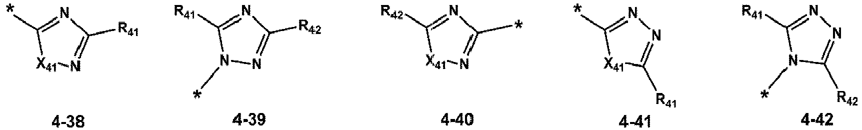

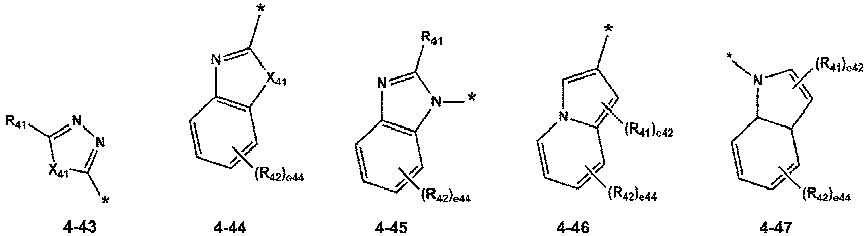

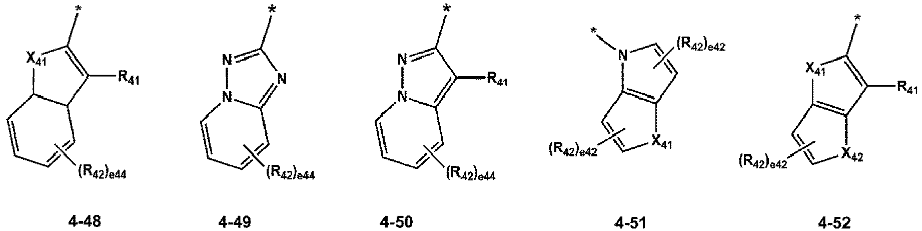

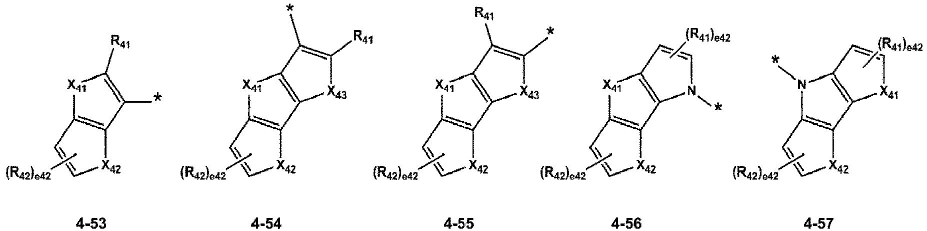

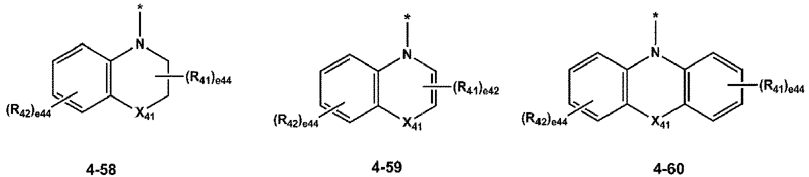

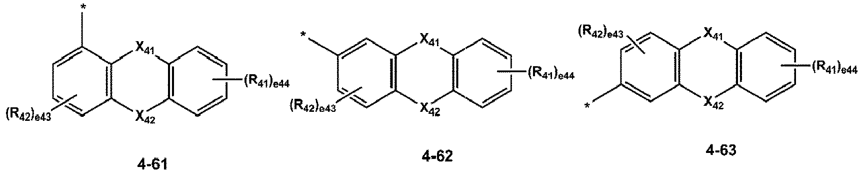

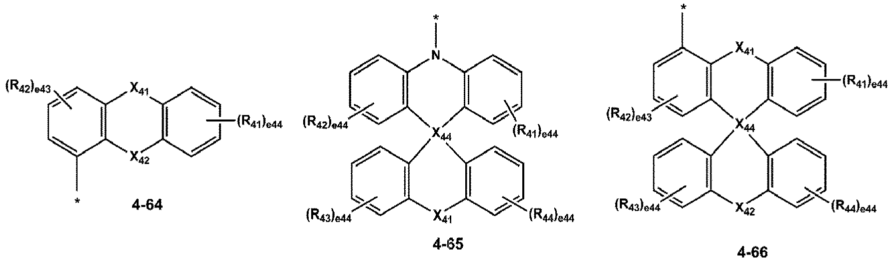

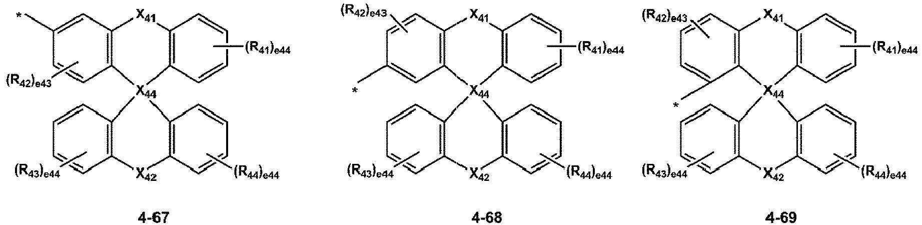

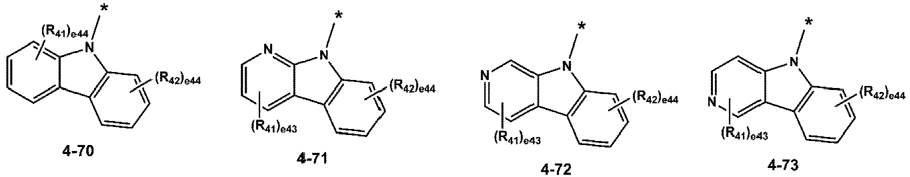

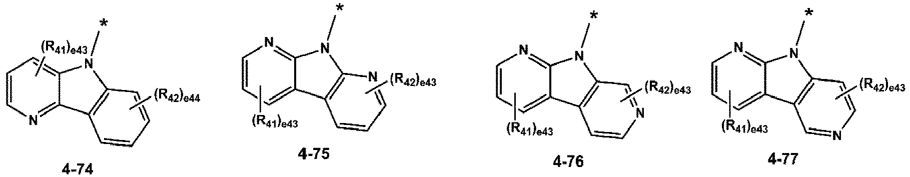

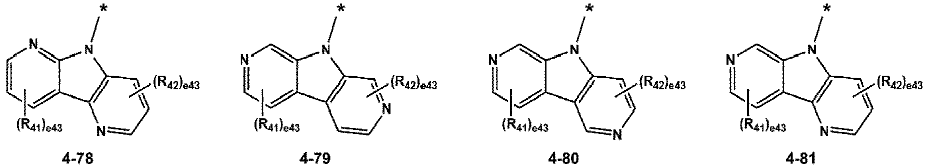

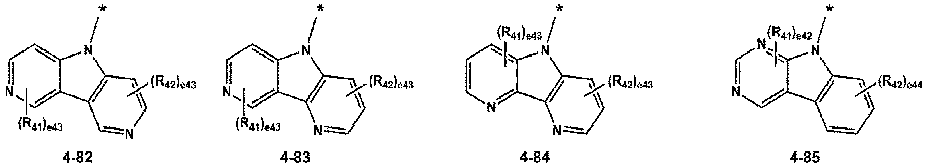

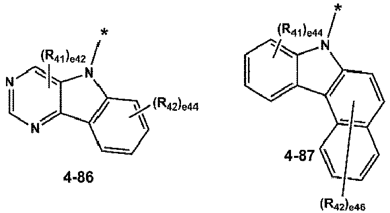

In Formulae 4-1 to 4-87,
X₄₁ may be O, S, Se, N(R₄₅), C(R₄₅)(R₄₆), or Si(R₄₅)(R₄₆),
X₄₂ may be O, S, Se, N(R₄₇), C(R₄₇)(R₄₈), or Si(R₄₇)(R₄₈),
X₄₃ may be O, S, Se, N(R₄₉), C(R₄₉)(R₅₀), or Si(R₄₉)(R₅₀),
X₄₄ may be C or Si,
R₄₁ to R₅₀ may each independently be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂),
two or more groups of R₄₁ to R₅₀ may optionally be bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ,
e42 may be 1 or 2,
e43 may be an integer from 1 to 3,
e44 may be an integer from 1 to 4,
e45 may be an integer from 1 to 5,
e46 may be an integer from 1 to 6,
e47 may be an integer from 1 to 7,
e49 may be an integer from 1 to 9,
Q₁ to Q₃ and R₁₀ₐ are each the same as described herein, and
* indicates a binding site to a neighboring atom.

In Formula 1, R₁ to R₄ are each independently hydrogen, deuterium, -F, -Cl, - Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂).

In Formula 1, d1 to d4 are each independently an integer from 0 to 10.

In Formula 1, two or more groups of R₁ to R₄ are optionally bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ.

In an embodiment, Z₁ to Z₄ may be all the same; or
Z₁ and Z₃ may be identical to each other, and Z₂ and Z₄ may be different from each other; or
Z₁ and Z₃ may be different from each other, and Z₂ and Z₄ may be identical to each other; or
Z₁ and Z₂ may be different from each other, and Z₃ and Z₄ may be identical to each other; or
Z₁ and Z₂ may be identical to each other, and Z₃ and Z₄ may be different from each other; or
Z₁ to Z₄ may all be different from each other.

In an embodiment, the sum of n1 to n4 may be an integer from 1 to 4, but is not limited thereto.

In an embodiment, n1 to n4 may satisfy at least one of Condition 1 and Condition 2:
[Condition 1]
   The sum of n2 and n4 is 1 or 2
[Condition 2]
   The sum of n1 and n3 is 0 or 2.

In an embodiment, when each of E₁ to E₄ is a C₃-C₆₀ carbocyclic group unsubstituted or substituted with at least one R₁₀ₐ, at least one of Condition 1 and Condition 2 may be satisfied.

In an embodiment, n1 may be 0, n2 may be 0, n3 may be 0, and n4 may be an integer of 1 or greater; or
n1 may be 0, n2 may be an integer of 1 or greater, n3 may be 0, and n4 may be 0; or
n1 may be 0, n2 may be an integer of 1 or greater, n3 may be 0, and n4 may be an integer of 1 or greater; or
n1 may be an integer of 1 or greater, n2 may be an integer of 1 or greater, n3 may be an integer of 1 or greater, and n4 may be an integer of 1 or greater; or
n1 may be an integer of 1 or greater, n2 may be 0, n3 may be 0, and n4 may be 0; or
n1 may be 0, n2 may be 0, n3 may be an integer of 1 or greater, and n4 may be 0; or
n1 may be an integer of 1 or greater, n2 may be 0, n3 may be an integer of 1 or greater, and n4 may be 0; or
n1 may be an integer of 1 or greater, n2 may be an integer of 1 or greater, n3 may be 0, and n4 may be 0; or
n1 may be an integer of 1 or greater, n2 may be an integer of 1 or greater, n3 may be an integer of 1 or greater, and n4 may be 0, but embodiments are not limited thereto.

In an embodiment, n1 may be 0, n2 may be 0, n3 may be 0, and n4 may be 1; or
n1 may be 0, n2 may be 1, n3 may be 0, and n4 may be 0; or
n1 may be 0, n2 may be 1, n3 may be 0, and n4 may be 1; or
n1 may be 1, n2 may be 1, n3 may be 1, and n4 may be 1; or
n1 may be 1, n2 may be 0, n3 may be 0, and n4 may be 0; or
n1 may be 0, n2 may be 0, n3 may be 1, and n4 may be 0; or
n1 may be 1, n2 may be 0, n3 may be 1, and n4 may be 0; or
n1 may be 1, n2 may be 1, n3 may be 0, and n4 may be 0; or
n1 may be 1, n2 may be 1, n3 may be 1, and n4 may be 0.

In an embodiment, at least two of n1 to n4 may each independently be an integer of 1 or greater, but embodiments are not limited thereto.

In an embodiment, in Formula 1, a moiety represented by 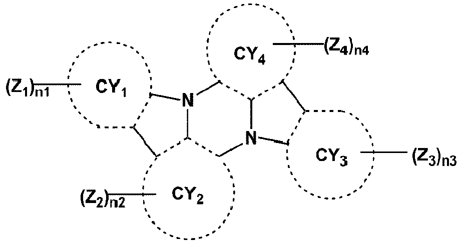 may be a moiety represented by one of Formulae 1-1 to 1-27:

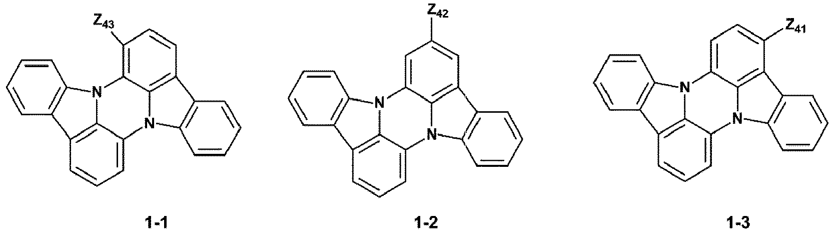

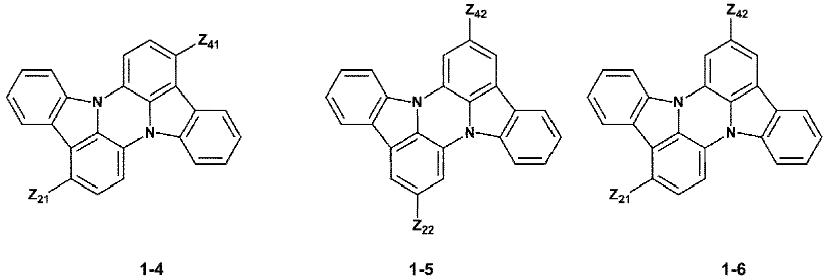

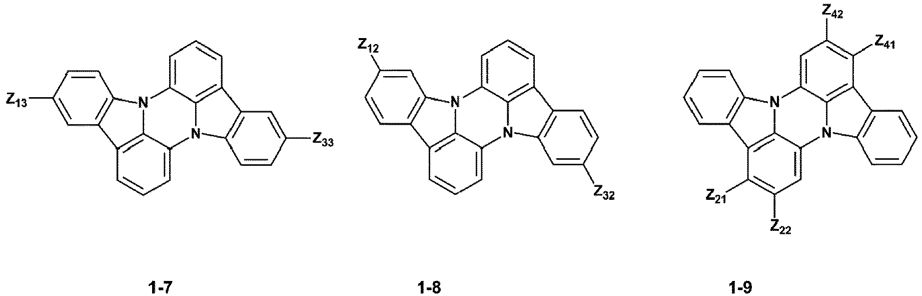

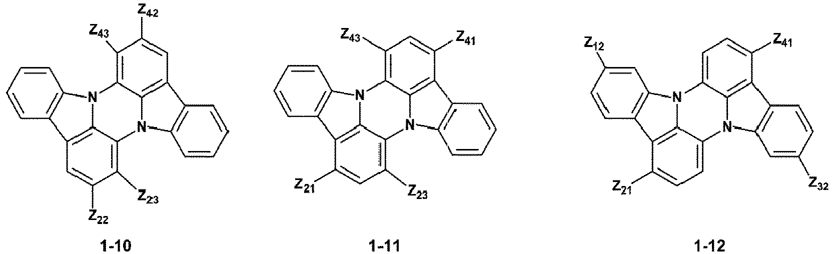

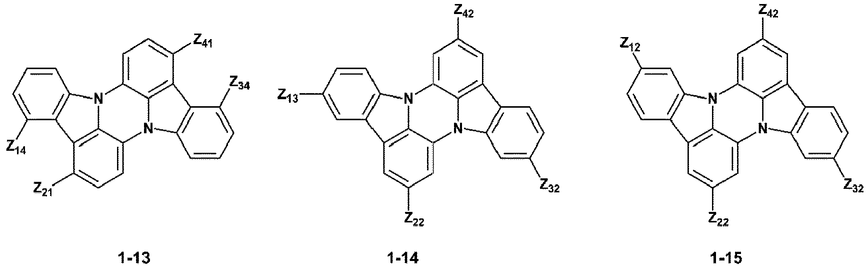

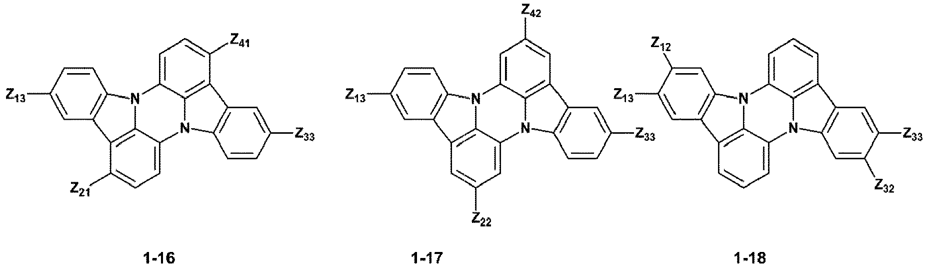

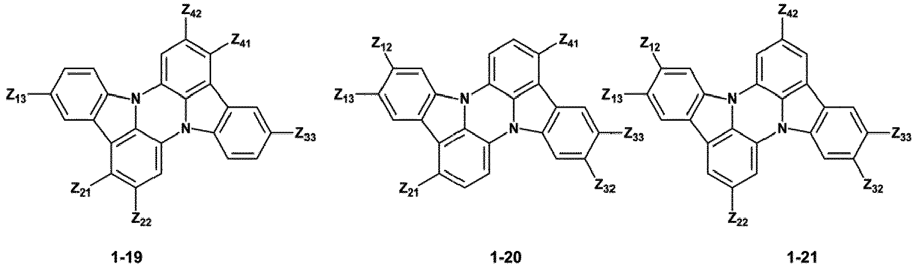

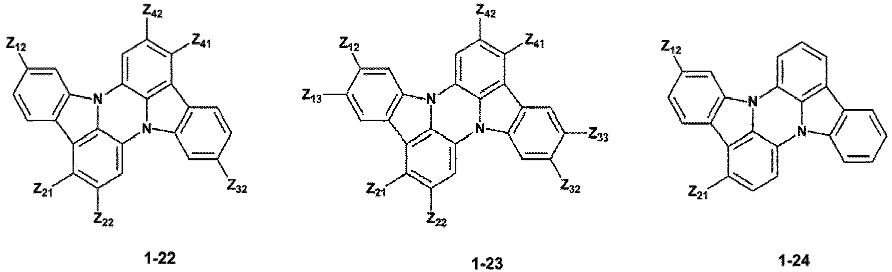

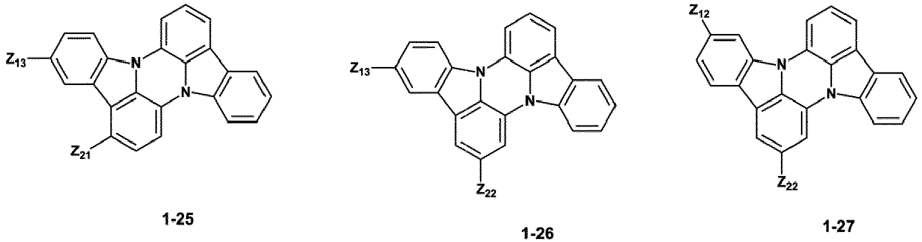

In Formulae 1-1 to 1-27,
Z₁₂ to Z₁₄ may each independently be the same as described herein in connection with Z₁,
Z₂₁ to Z₂₃ may each independently be the same as described herein in connection with Z₂,
Z₃₂ to Z₃₄ may each independently be the same as described herein in connection with Z₃, and
Z₄₁ to Z₄₃ may each independently be the same as described herein in connection with Z₄.

Each group "R₁₀ₐ" as used herein independently is:
deuterium (-D), -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), - C(=O)(Qn), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇₋C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q31)(Q32),
wherein Q₁ to Q₃, Q₁₁ to Q₁₃, Q₂₁ to Q₂₃ and Q₃₁ to Q₃₃ are each independently: hydrogen, deuterium, -F, -Cl, -Br, -I, a hydroxyl group, a cyano group, or a nitro group; a C₁-C₆₀ alkyl group, a C₂-C₆₀ alkenyl group, a C₂-C₆₀ alkynyl group, a C₁-C₆₀ alkoxy group, a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof; or a C₇-C₆₀ aryl alkyl group; or a C₂-C₆₀ heteroaryl alkyl group.

In embodiments, the heterocyclic compound represented by Formula 1 may be one of Compounds 1 to 111, but embodiments are not limited thereto:

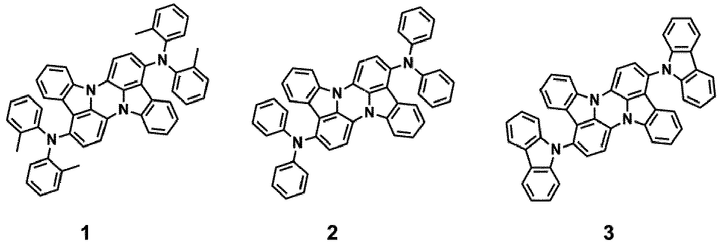

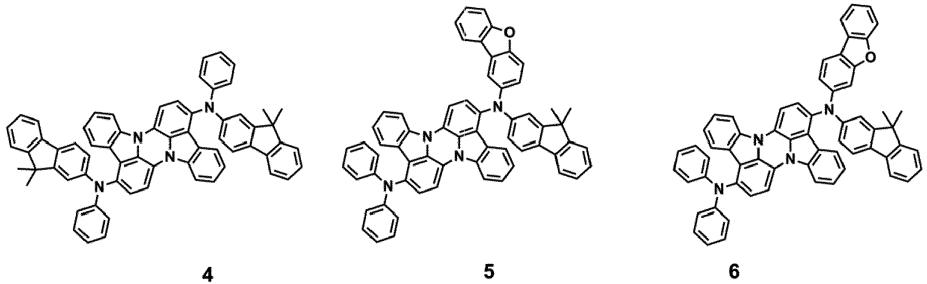

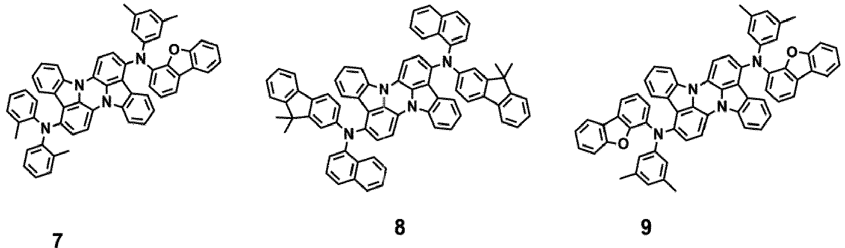

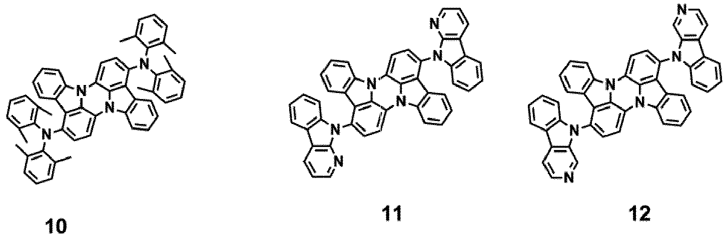

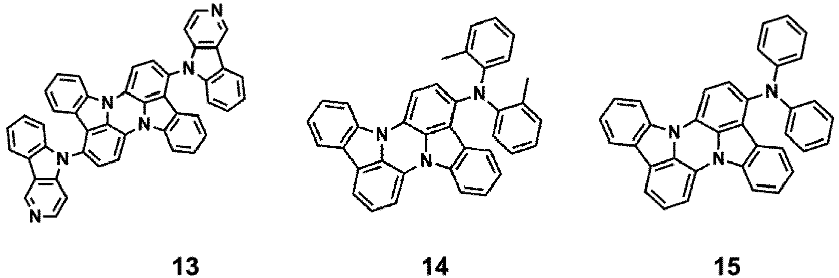

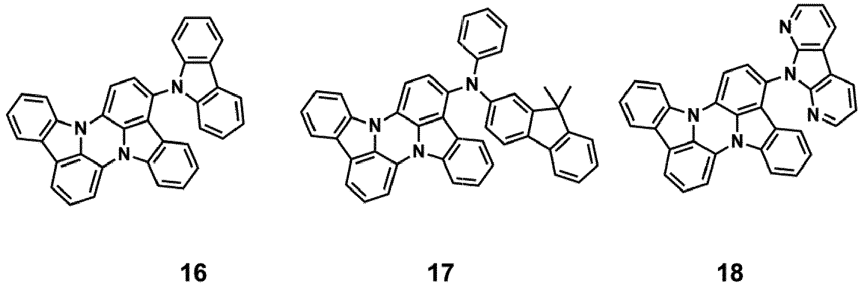

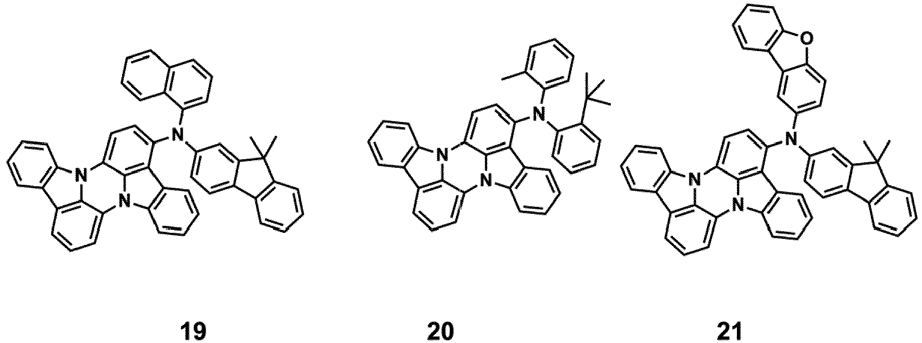

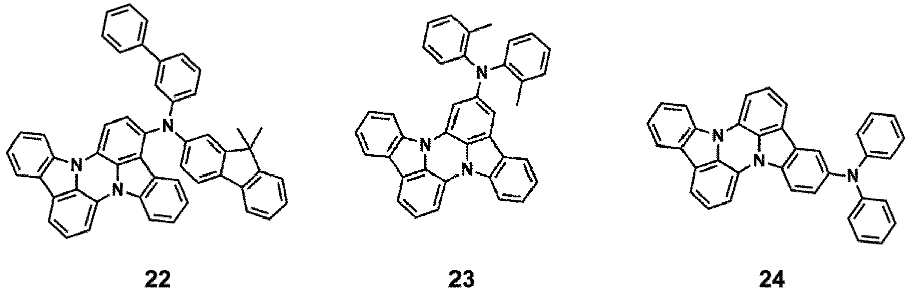

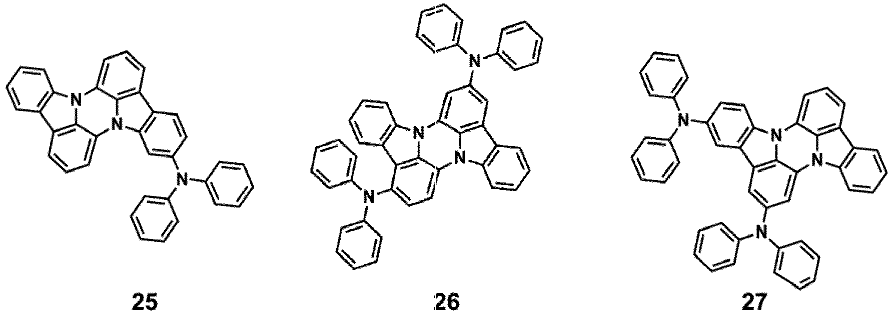

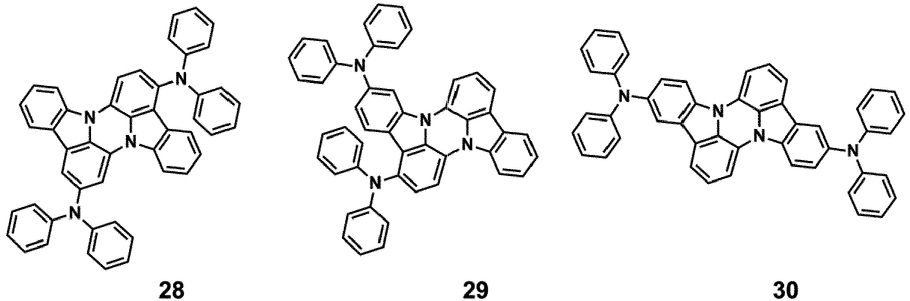

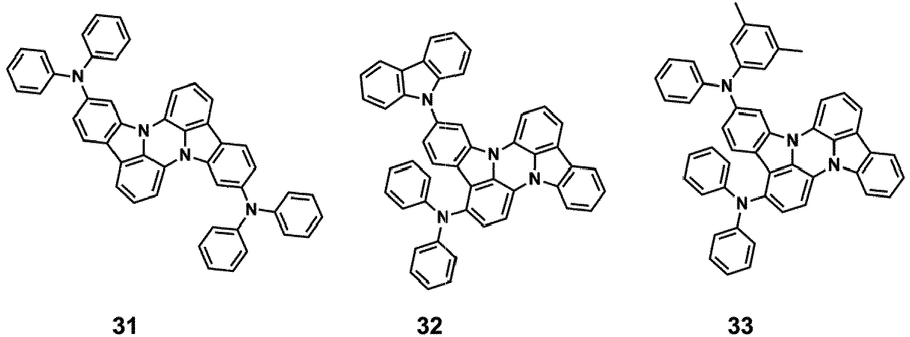

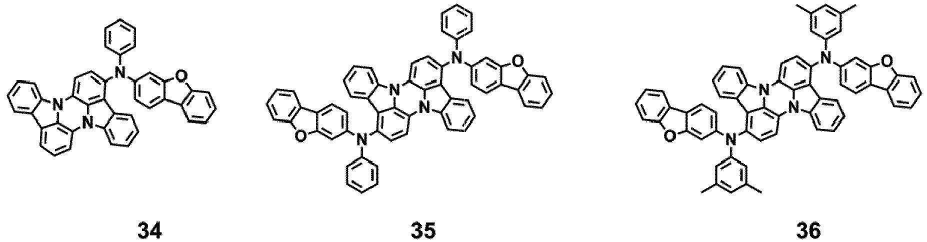

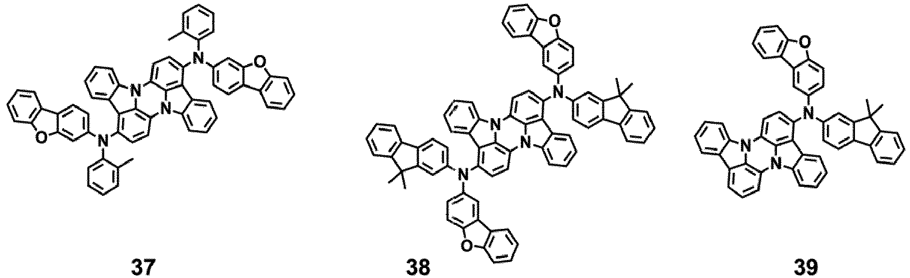

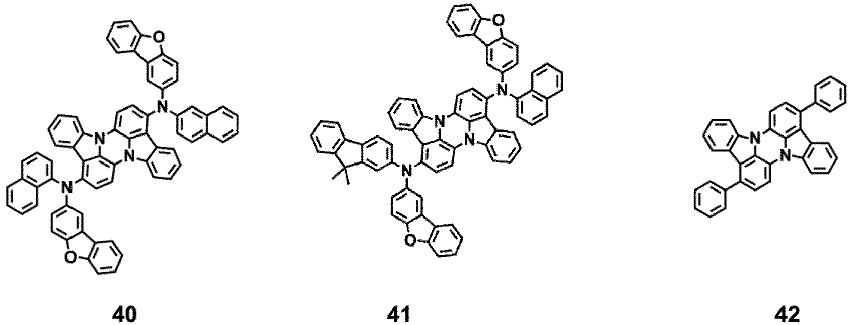

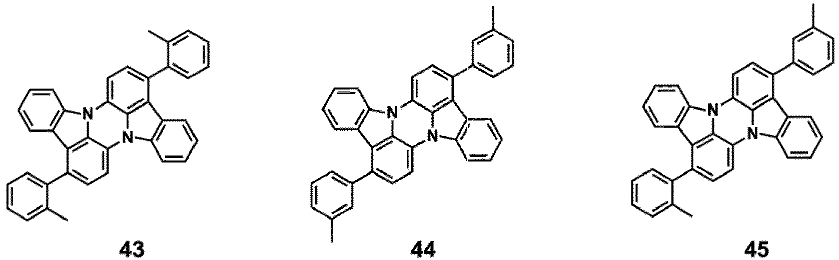

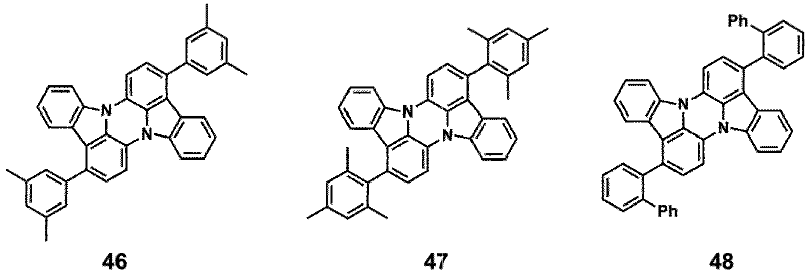

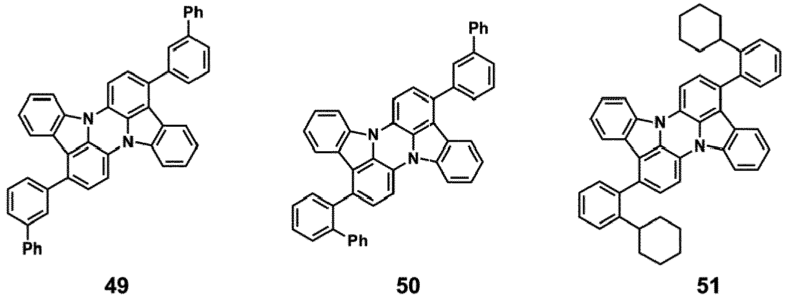

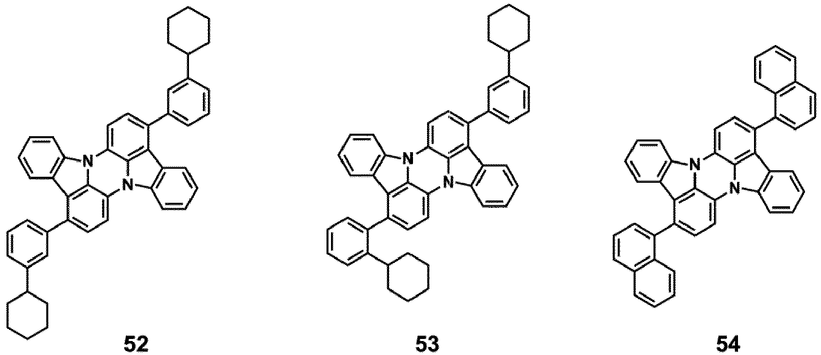

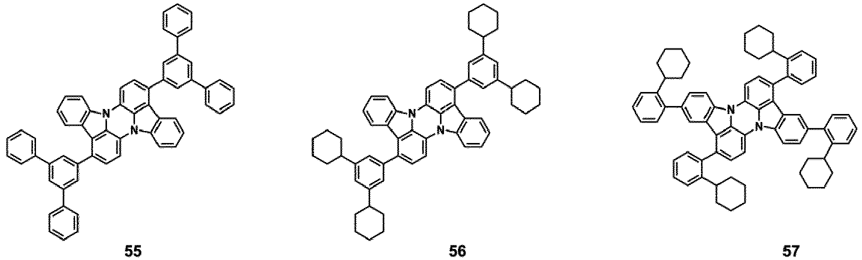

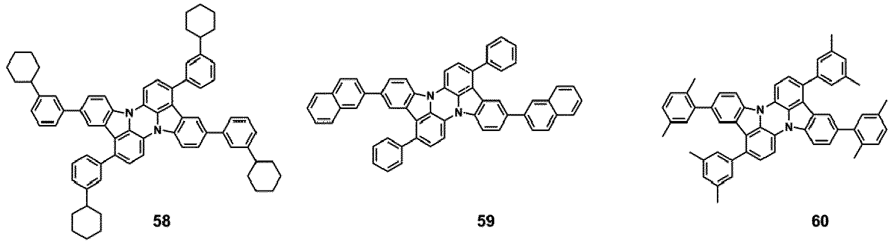

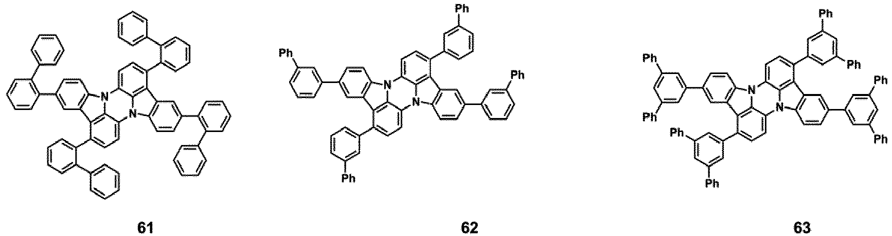

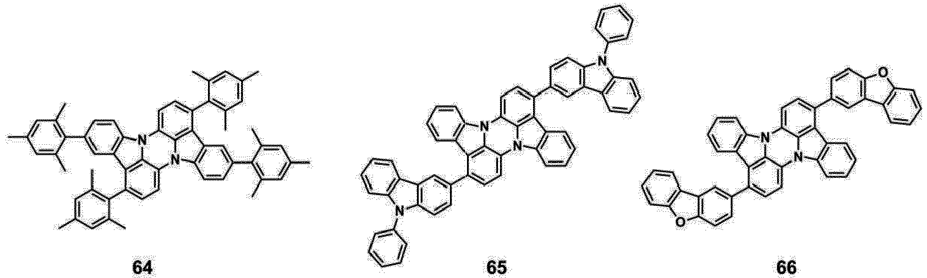

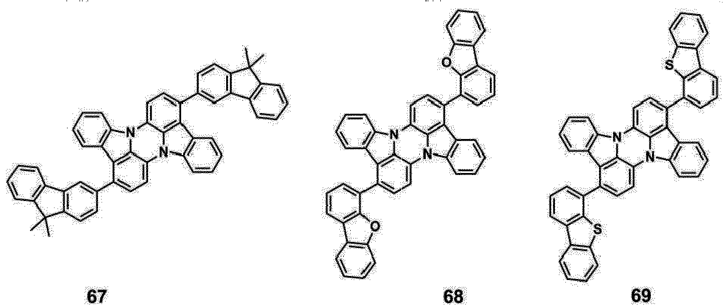

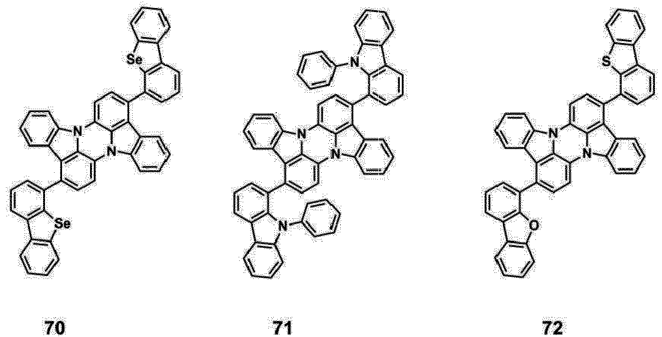

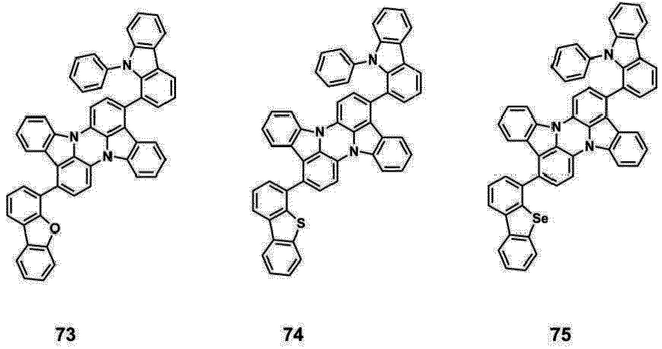

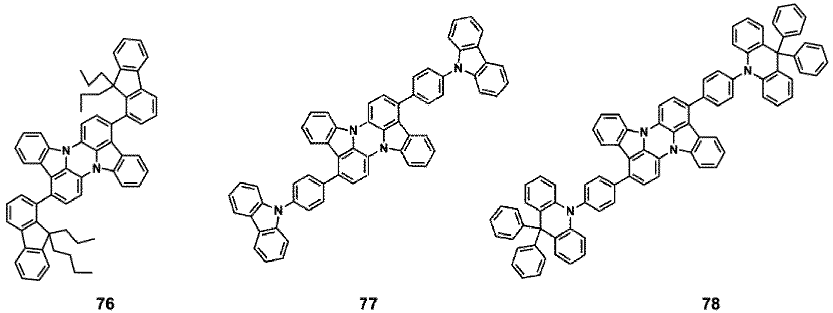

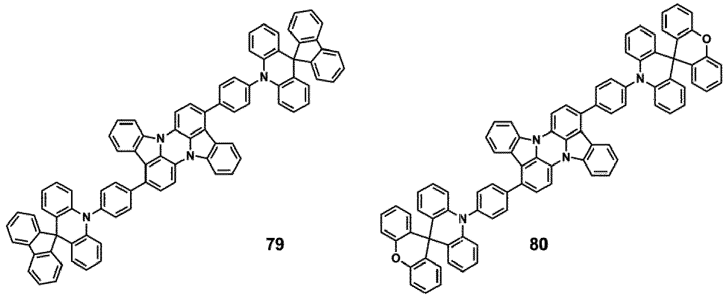

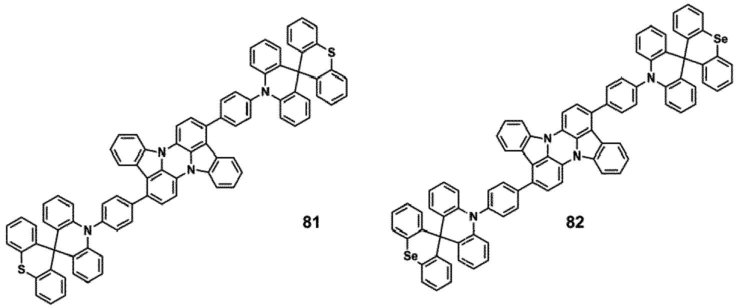

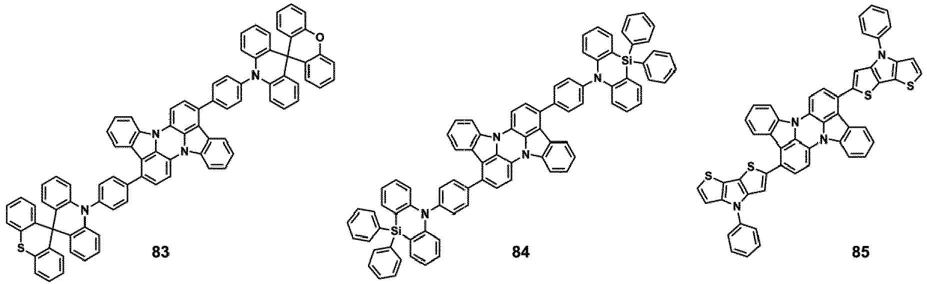

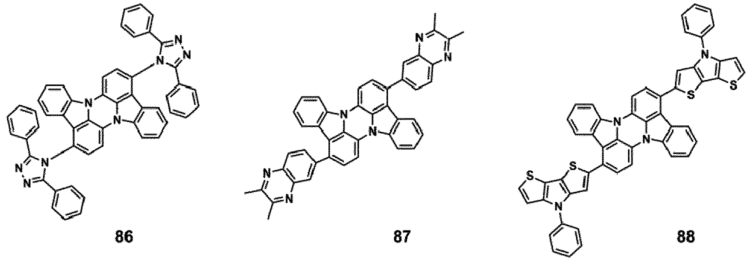

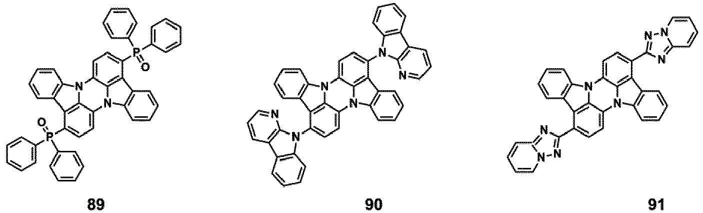

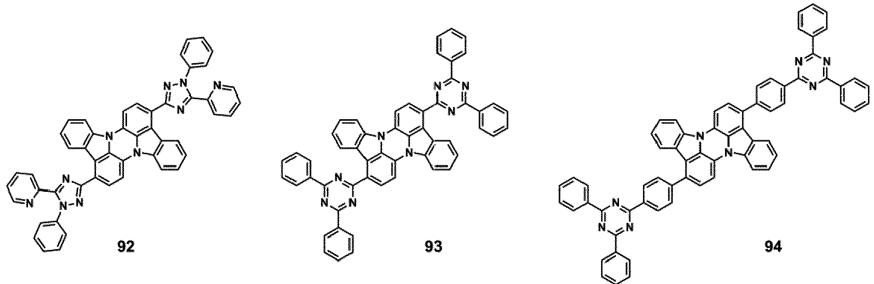

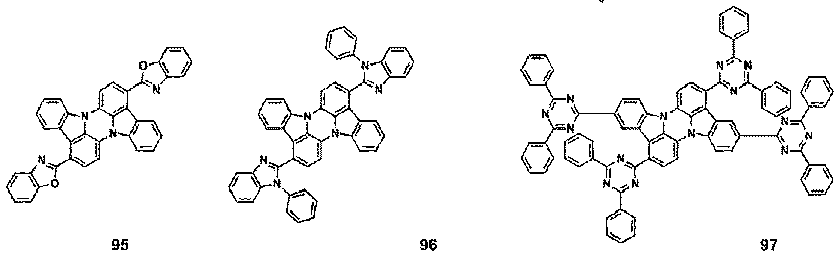

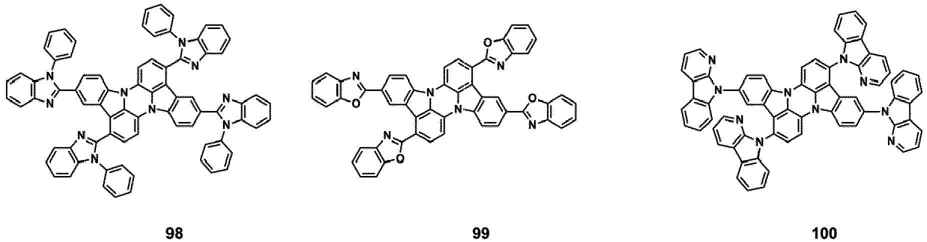

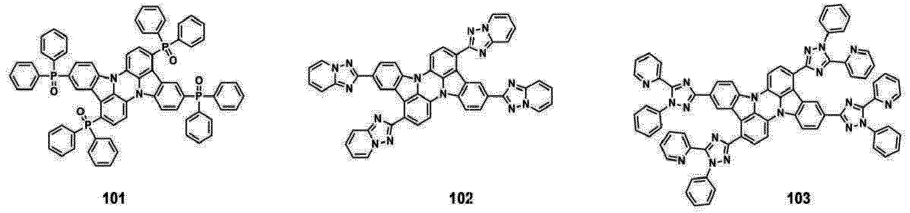

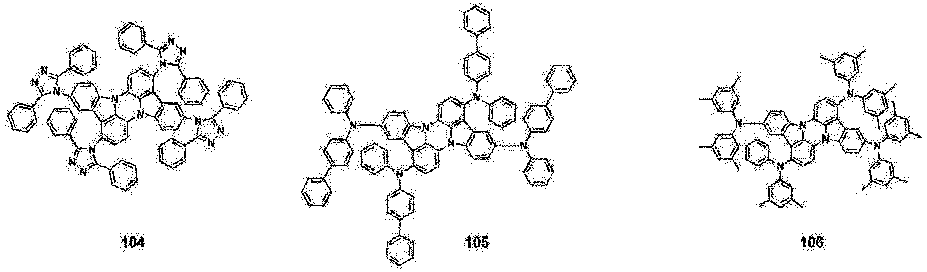

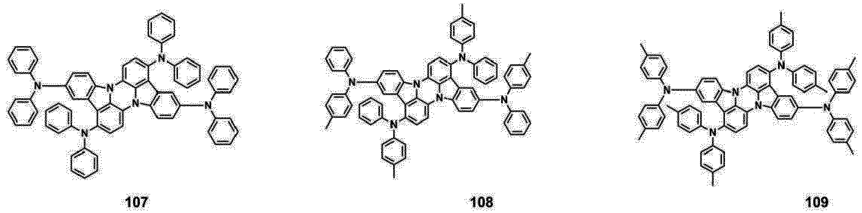

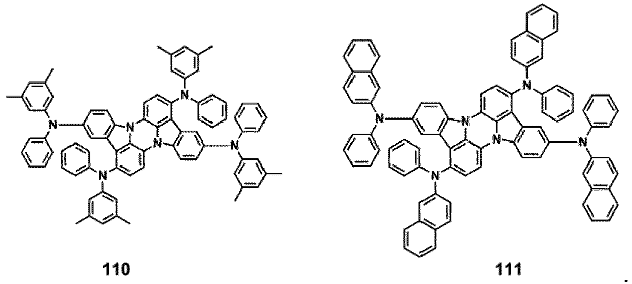

The heterocyclic compound represented by Formula 1 includes a bicarbazolebased core to which at least one carbocyclic group or at least one heteroatom-containing group is substituted.

The heterocyclic compound has a planar structure including a bicarbazolebased core. Due to the inclusion of the large planar structure having the condensed ring, multiple resonance is further activated and the delocalization of electrons in the molecule is expanded, and the polarizability is increased, resulting in a higher f value. Thus, the heterocyclic compound can be used as a high-efficiency delayedfluorescence material.

Since the heterocyclic compound is substituted with at least one carbocyclic group or at least one heteroatom-containing group, the molecule may retain rigidity in terms of molecular binding energy (BDE), and as a whole, multiple resonance can be achieved.

Therefore, an electronic device, e.g., an organic light-emitting device, using the heterocyclic compound represented by Formula 1 may have a low driving voltage, high maximum quantum yield, high efficiency, and long lifespan.

According to embodiments, a light-emitting device comprises a first electrode, a second electrode facing the first electrode, and an interlayer between the first electrode and the second electrode and including an emission layer, and at least one heterocyclic compound as described herein.

In an embodiment, the first electrode may be an anode;
the second electrode may be a cathode;
the interlayer may include the at least one heterocyclic compound, a hole transport region between the emission layer and the first electrode, and an electron transport region between the emission layer and the second electrode;
the hole transport region may include a hole injection layer, a hole transport layer, an emission auxiliary layer, an electron-blocking layer, or any combination thereof, and
the electron transport region may include a buffer layer, a hole-blocking layer, an electron control layer, an electron transport layer, an electron injection layer, or any combination thereof.

In an embodiment, the emission layer may include the at least one heterocyclic compound represented by Formula 1.

In an embodiment, the emission layer may further include a host, and an amount of the heterocyclic compound may be in a range of about 0.01 parts by weight to about 49.99 parts by weight, based on 100 parts by weight of the emission layer.

In an embodiment, the emission layer may further include an anthracenecontaining compound.

The term "anthracene-based compound" as used herein may be a compound containing an anthracene group.

In an embodiment, the anthracene-based compound may be included as a host in the emission layer.

In an embodiment, the anthracene-based compound may be a compound represented by Formula 5:

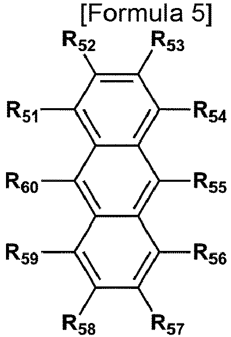

In Formula 5,
R₅₁ to R₆₀ may each independently be *-(L₅₁)ₐ₅₁-A₅₁, hydrogen, deuterium, -F, - Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or-P(=O)(Q₁)(Q₂),
L₅₁ may be a single bond, a C₅-C₃₀ carbocyclic group unsubstituted or substituted with at least one R₁₀ₐ, or a C₁-C₃₀ heterocyclic group unsubstituted or substituted with at least one R₁₀ₐ,
a51 may be an integer from 1 to 3,
A₅₁ may be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
two or more groups of R₅₁ to R₆₀ may optionally be bound to each other to form a C₅-C₃₀ carbocyclic group that is unsubstituted or substituted with at least one R₁₀ₐ or a C₂-C₃₀ heterocyclic group unsubstituted or substituted with at least one R₁₀ₐ, and
R₁₀ₐ is the same as described herein.

In an embodiment, the emission layer may emit blue light or blue-green light.

In an embodiment, the emission layer may be configured to emit light having a maximum emission wavelength in a range of about 400 nm to about 500 nm.

According to an embodiment, provided is an electronic apparatus which comprises the light-emitting device.

In an embodiment, the electronic apparatus may further include a thin-film transistor, wherein the thin-film transistor may include a source electrode and a drain electrode, and the first electrode of the light-emitting device may be electrically connected to at least one of the source electrode or the drain electrode.

In an embodiment, the electronic apparatus may further include a color filter, a quantum dot color conversion layer, a color conversion layer, a touch screen layer, a polarizing layer, or any combination thereof. For example, the electronic apparatus may be a flat panel display apparatus, but embodiments are not limited thereto.

The electronic apparatus may be the same as described herein.

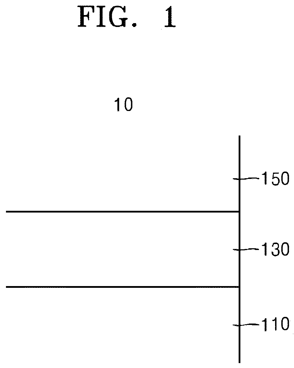

### [Description of FIG. 1]

FIG. 1 is a schematic cross-sectional view of a light-emitting device 10 according to an embodiment. The light-emitting device 10 includes a first electrode 110, an interlayer 130, and a second electrode 150.

Hereinafter, the structure of the light-emitting device 10 according to an embodiment and a method of manufacturing the light-emitting device 10 will be described with reference to FIG. 1.

### [First electrode 110]

In FIG. 1, a substrate may be further included under the first electrode 110 or on the second electrode 150. The substrate may be a glass substrate or a plastic substrate. In embodiments, the substrate may be a flexible substrate, and may include plastics with excellent heat resistance and durability, such as polyimide, polyethylene terephthalate (PET), polycarbonate, polyethylene naphthalate, polyarylate (PAR), polyetherimide, or any combination thereof.

The first electrode 110 may be formed by, for example, depositing or sputtering a material for forming the first electrode 110 on the substrate. When the first electrode 110 is an anode, a material for forming the first electrode 110 may be a high-work function material that facilitates injection of holes.

The first electrode 110 may be a reflective electrode, a semi-transmissive electrode, or a transmissive electrode. When the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. In embodiments, when the first electrode 110 is a semi-transmissive electrode or a reflective electrode, a material for forming the first electrode 110 may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 110 may have a structure consisting of a single layer or a structure including multiple layers. For example, the first electrode 110 may have a three-layered structure of ITO/Ag/ITO.

### [Interlayer 130]

The interlayer 130 may be located on the first electrode 110. The interlayer 130 may include an emission layer.

The interlayer 130 may further include a hole transport region between the first electrode 110 and the emission layer, and an electron transport region between the emission layer and the second electrode 150.

The interlayer 130 may further include, in addition to various organic materials, a metal-containing compound such as an organometallic compound, an inorganic material such as quantum dots, or the like.

In embodiments, the interlayer 130 may include two or more emitting units stacked between the first electrode 110 and the second electrode 150, and at least one charge generation layer located between the two or more emitting units. When the interlayer 130 includes the two or more emitting units and the at least one charge generation layer as described above, the light-emitting device 10 may be a tandem light-emitting device.

### [Hole transport region in interlayer 130]

The hole transport region may have a structure consisting of a layer consisting of a single material, a structure consisting of a layer consisting of different materials, or a structure including multiple layers including different materials.

The hole transport region may include a hole injection layer, a hole transport layer, an emission auxiliary layer, an electron-blocking layer, or any combination thereof.

For example, the hole transport region may have a multi-layered structure including a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/ellectron-blocking layer structure, wherein the layers of each structure may be stacked from the first electrode 110 in its respective stated order, but the structure of the hole transport region is not limited thereto.

The hole transport region may include a compound represented by Formula 201, a compound represented by Formula 202, or any combination thereof:

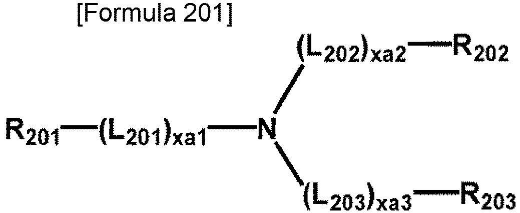

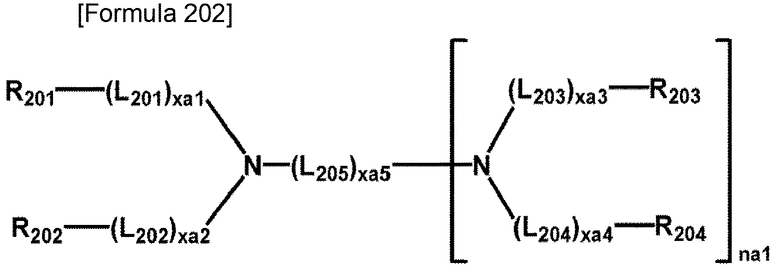

In Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ, or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group (for example, a carbazole group or the like) unsubstituted or substituted with at least one R₁₀ₐ (for example, Compound HT16),
R₂₀₃ and R₂₀₄ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₆-C₆₀ polycyclic group unsubstituted or substituted with at least one R₁₀ₐ, and
na1 may be an integer from 1 to 4.

In embodiments, each of Formulae 201 and 202 may include at least one of groups represented by Formulae CY201 to CY217:

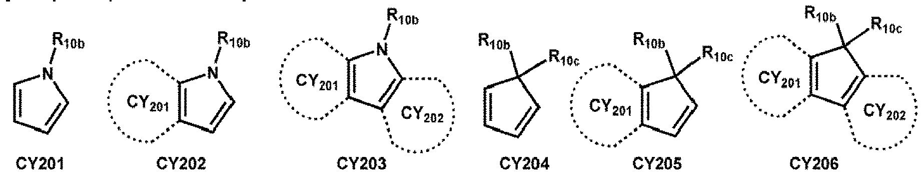

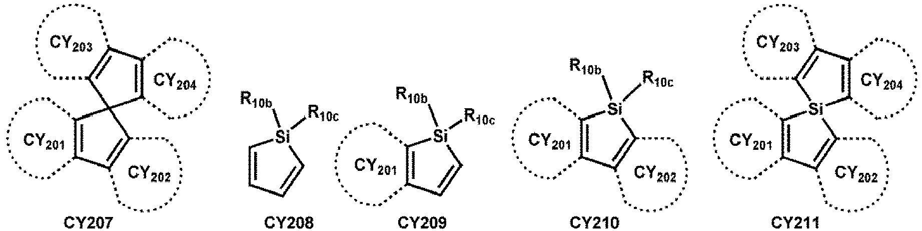

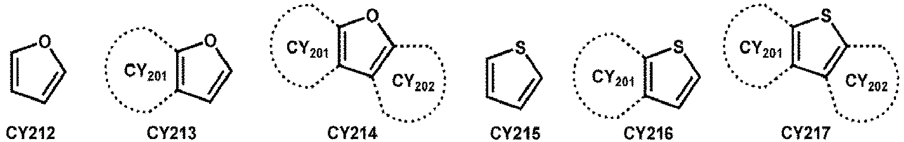

In Formulae CY201 to CY217, R_{10b} and R_{10c} may each independently be the same as described with respect to R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ as described herein.

In an embodiment, in Formulae CY201 to CY217, ring CY₂₀₁ to ring CY₂₀₄ may each independently be a benzene group, a naphthalene group, a phenanthrene group, or an anthracene group.

In embodiments, each of Formulae 201 and 202 may include at least one of groups represented by Formulae CY201 to CY203.

In embodiments, a compound represented by Formula 201 may include at least one of groups represented by Formulae CY201 to CY203 and at least one of groups represented by Formulae CY204 to CY217.

In embodiments, in Formula 201, xa1 may be 1, R₂₀₁ may be a group represented by one of Formulae CY201 to CY203, xa2 may be 0, and R₂₀₂ may be a group represented by one of Formulae CY204 to CY207.

In embodiments, each of Formulae 201 and 202 may not include a group represented by one of Formulae CY201 to CY203.

In embodiments, each of Formulae 201 and 202 may not include a group represented by one of Formulae CY201 to CY203, and may include at least one of groups represented by Formulae CY204 to CY217.

In embodiments, each of Formulae 201 and 202 may not include a group represented by one of Formulae CY201 to CY217.

In an embodiment, the hole transport region may include one of Compounds HT1 to HT46, m-MTDATA, TDATA, 2-TNATA, NPB(NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), or any combination thereof:

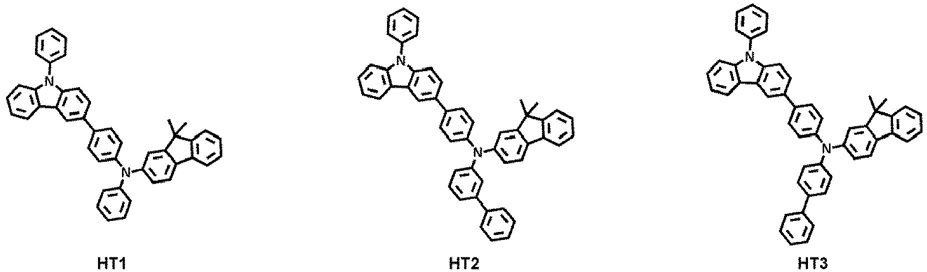

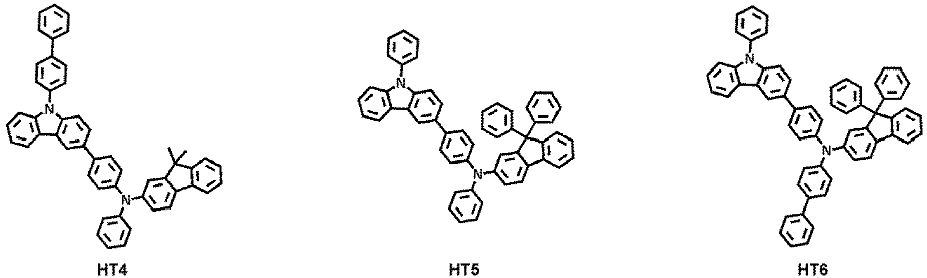

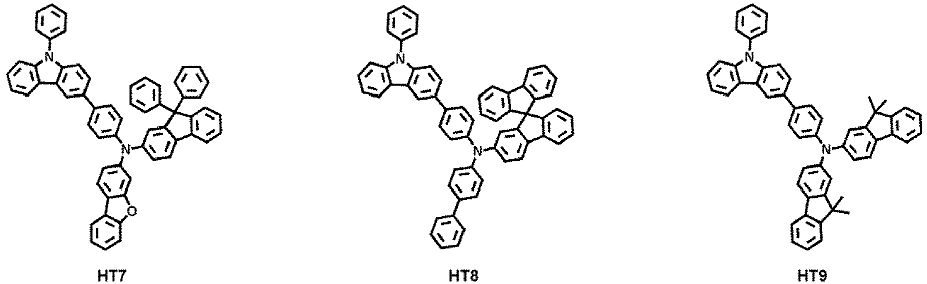

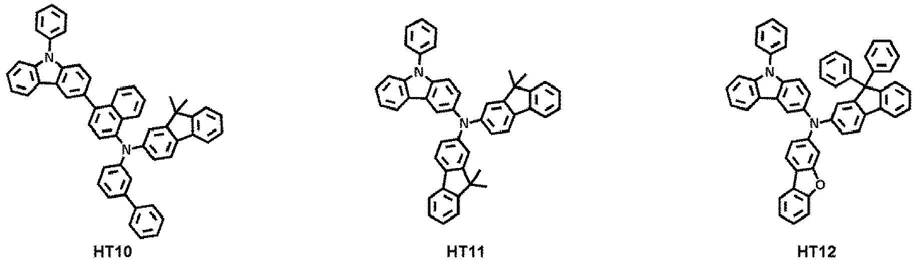

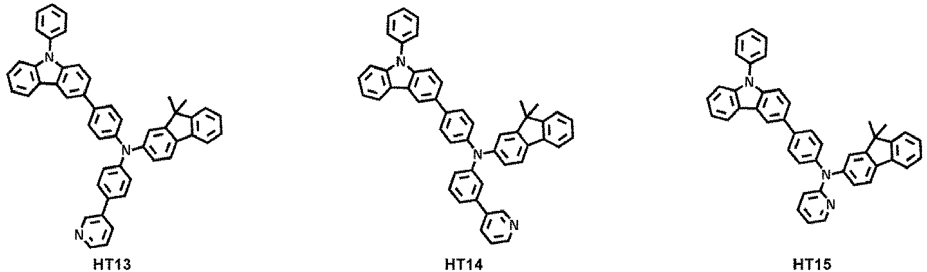

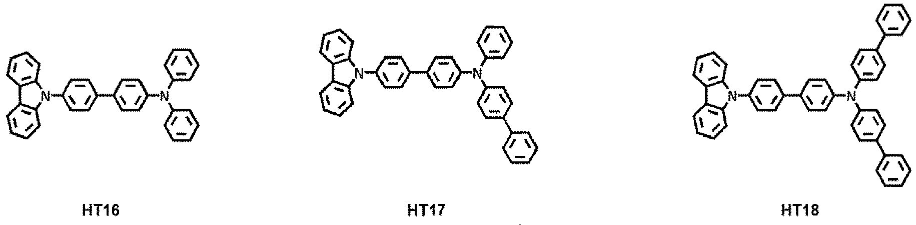

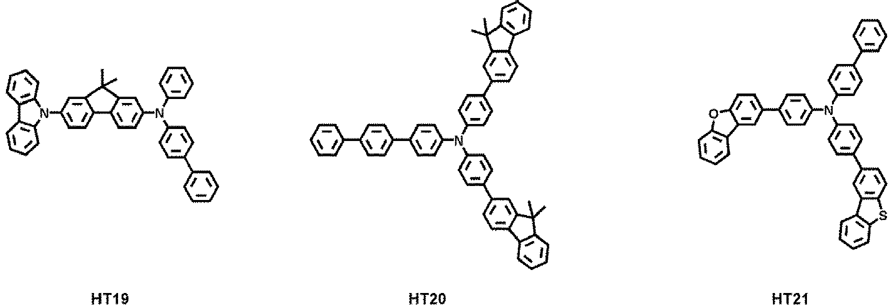

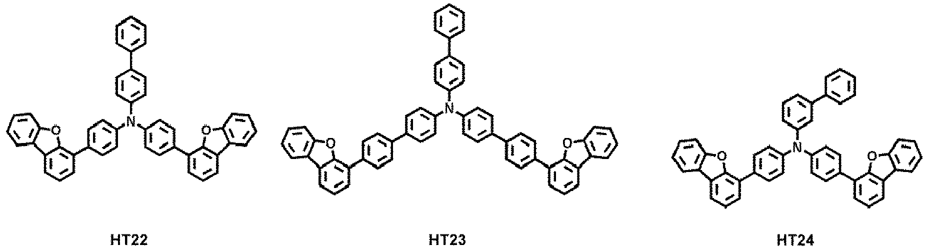

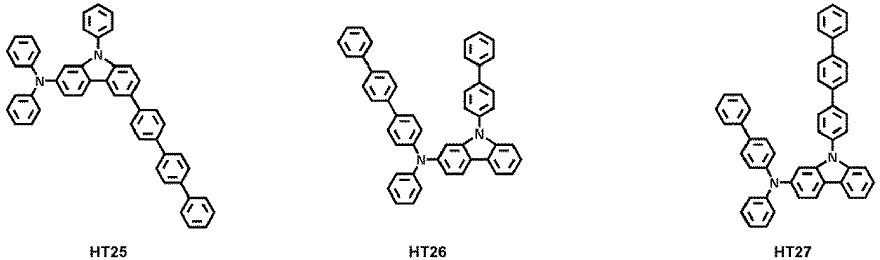

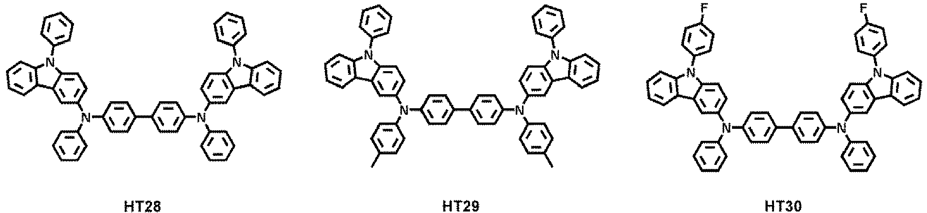

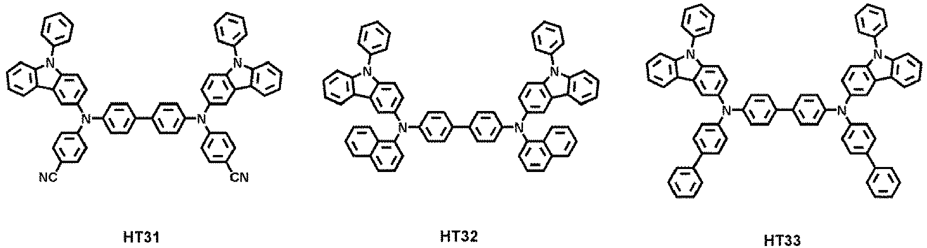

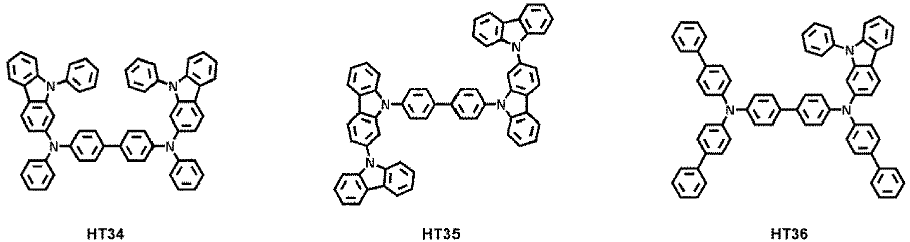

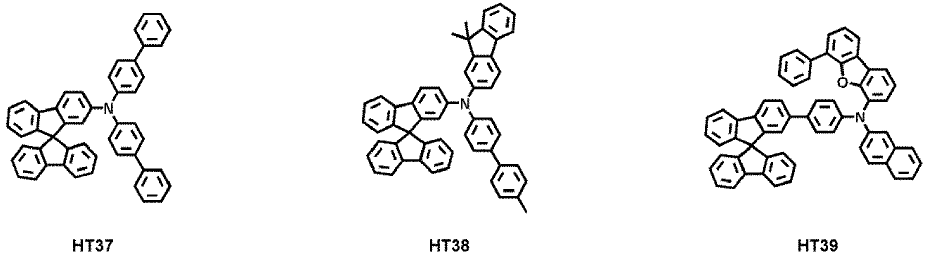

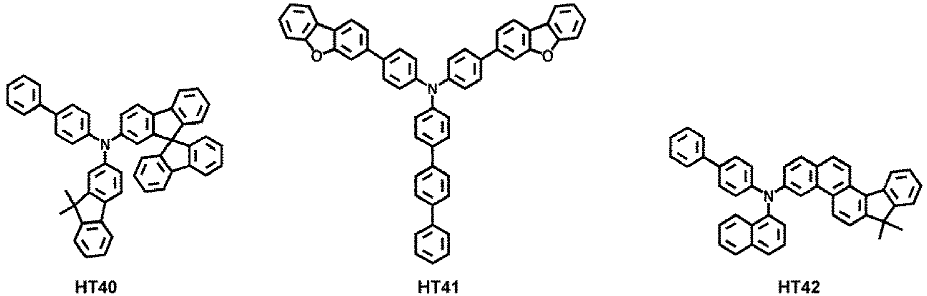

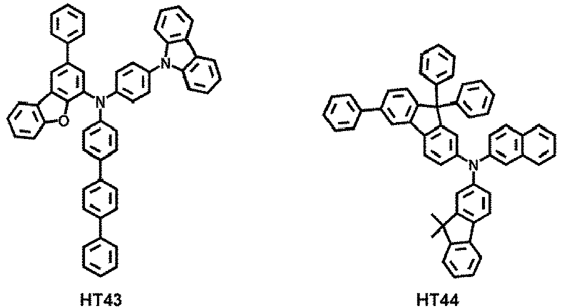

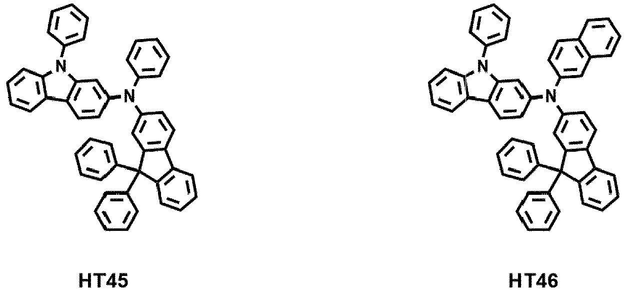

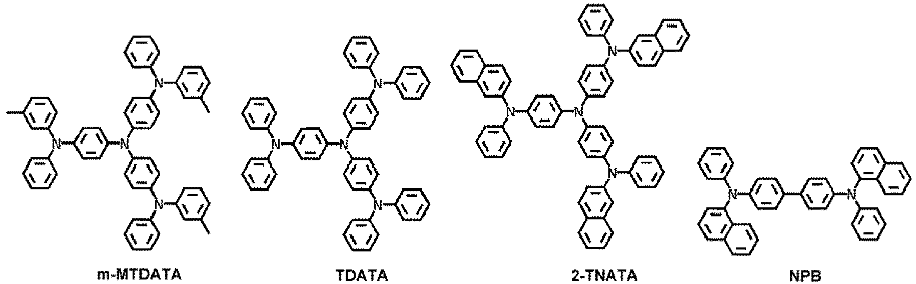

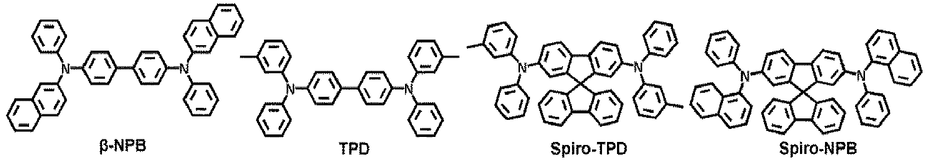

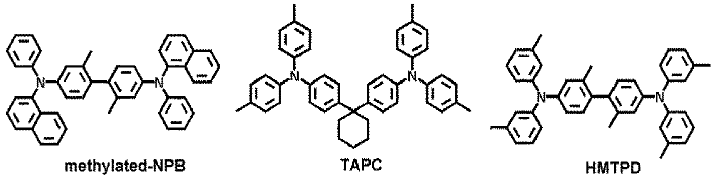

A thickness of the hole transport region may be in a range of about 50 Å to about 10,000 Å. For example, the thickness of the hole transport region may be in a range of about 100 Å to about 4,000 Å. When the hole transport region includes a hole injection layer, a hole transport layer, or any combination thereof, a thickness of the hole injection layer may be in a range of about 100 Å to about 9,000 Å, and a thickness of the hole transport layer may be in a range of about 50 Å to about 2,000 Å. For example, the thickness of the hole injection layer may be in a range of about 100 Å to about 1,000 Å. For example, the thickness of the hole transport layer may be in a range of about 100 Å to about 1,500 Å. When the thicknesses of the hole transport region, the hole injection layer, and the hole transport layer are within these ranges, satisfactory hole transporting characteristics may be obtained without a substantial increase in driving voltage.

The emission auxiliary layer may increase light-emission efficiency by compensating for an optical resonance distance according to a wavelength of light emitted by an emission layer, and the electron-blocking layer may block the leakage of electrons from an emission layer to a hole transport region. Materials that may be included in the hole transport region may be included in the emission auxiliary layer and the electron-blocking layer.

### [p-dopant]

The hole transport region may further include, in addition to these materials, a charge-generation material for the improvement of conductive properties. The charge-generation material may be uniformly or non-uniformly dispersed in the hole transport region (for example, in the form of a single layer consisting of a charge-generation material).

The charge-generation material may be, for example, a p-dopant.

For example, a lowest unoccupied molecular orbital (LUMO) energy level of the p-dopant may be equal to or less than about -3.5 eV.

In embodiments, the p-dopant may include a quinone derivative, a cyano group-containing compound, a compound including element EL1 and element EL2, or any combination thereof.

Examples of the quinone derivative may include TCNQ, F4-TCNQ, etc.

Examples of the cyano group-containing compound may include HAT-CN, and a compound represented by Formula 221.

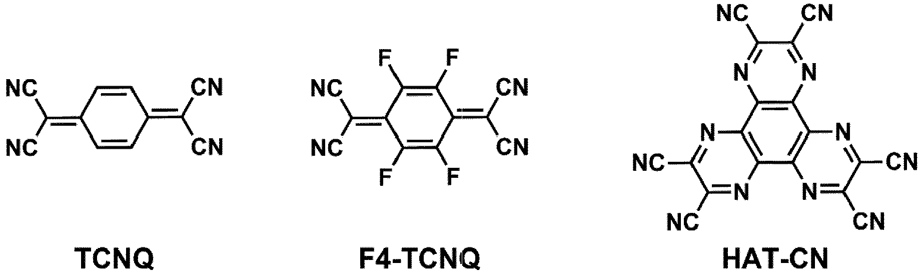

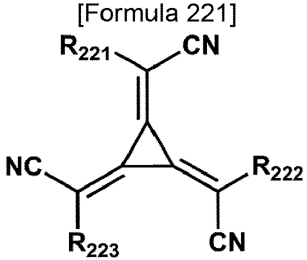

In Formula 221,
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with a cyano group; -F; -Cl; -Br; -I; a C₁-C₂₀ alkyl group substituted with a cyano group, -F, -Cl, -Br, -I, or any combination thereof; or any combination thereof.

In the compound including element EL1 and element EL2, element EL1 may be a metal, a metalloid, or any combination thereof, and element EL2 may be a non-metal, a metalloid, or any combination thereof.

Examples of the metal may include an alkali metal (for example, lithium (Li), sodium (Na), potassium (K), rubidium (Rb), cesium (Cs), etc.); an alkaline earth metal (for example, beryllium (Be), magnesium (Mg), calcium (Ca), strontium (Sr), barium (Ba), etc.); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); a post-transition metal (for example, zinc (Zn), indium (In), tin (Sn), etc.); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.).

Examples of the metalloid may include silicon (Si), antimony (Sb), and tellurium (Te).

Examples of the non-metal may include oxygen (O) and a halogen (for example, F, Cl, Br, I, etc.).

Examples of the compound including element EL1 and element EL2 may include a metal oxide, a metal halide (for example, a metal fluoride, a metal chloride, a metal bromide, or a metal iodide), a metalloid halide (for example, a metalloid fluoride, a metalloid chloride, a metalloid bromide, or a metalloid iodide), a metal telluride, or any combination thereof.

Examples of the metal oxide may include tungsten oxide (for example, WO, W₂O₃, WO₂, WO₃, W₂O₅, etc.), vanadium oxide (for example, VO, V₂O₃, VO₂, V₂O₅, etc.), molybdenum oxide (MoO, Mo₂O₃, MoO₂, MoOs, Mo₂O₅, etc.), and rhenium oxide (for example, ReOs, etc.).

Examples of the metal halide may include an alkali metal halide, an alkaline earth metal halide, a transition metal halide, a post-transition metal halide, and a lanthanide metal halide.

Examples of the alkali metal halide may include LiF, NaF, KF, RbF, CsF, LiCI, NaCl, KCI, RbCI, CsCI, LiBr, NaBr, KBr, RbBr, CsBr, Lil, Nal, KI, Rbl, and Csl.

Examples of the alkaline earth metal halide may include BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, Bel₂, Mgl₂, Cal₂, SrI₂, and BaI₂.

Examples of the transition metal halide may include a titanium halide (for example, TiF₄, TiCl₄, TiBr₄, TiI₄, etc.), a zirconium halide (for example, ZrF₄, ZrCl₄, ZrBr₄, ZrI₄, etc.), a hafnium halide (for example, HfF₄, HfCl₄, HfBr₄, Hfl₄, etc.), a vanadium halide (for example, VF₃, VCl₃, VBr₃, VI₃, etc.), a niobium halide (for example, NbF₃, NbCl₃, NbBr₃, Nbl₃, etc.), a tantalum halide (for example, TaF₃, TaCl₃, TaBr₃, TaI₃, etc.), a chromium halide (for example, CrF₃, CrCl₃, CrBr₃, CrI₃, etc.), a molybdenum halide (for example, MoF₃, MoCl₃, MoBr₃, MoI₃, etc.), a tungsten halide (for example, WF₃, WCl₃, WBr₃, WI₃, etc.), a manganese halide (for example, MnF₂, MnCl₂, MnBr₂, MnI₂, etc.), a technetium halide (for example, TcF₂, TcCl₂, TcBr₂, TcI₂, etc.), a rhenium halide (for example, ReF₂, ReCl₂, ReBr₂, ReI₂, etc.), an iron halide (for example, FeF₂, FeCl₂, FeBr₂, FeI₂, etc.), a ruthenium halide (for example, RuF₂, RuCl₂, RuBr₂, RuI₂, etc.), an osmium halide (for example, OsF₂, OsCl₂, OsBr₂, OsI₂, etc.), a cobalt halide (for example, CoF₂, CoCl₂, CoBr₂, CoI₂, etc.), a rhodium halide (for example, RhF₂, RhCl₂, RhBr₂, RhI₂, etc.), an iridium halide (for example, IrF₂, IrCl₂, IrBr₂, IrI₂, etc.), a nickel halide (for example, NiF₂, NiCl₂, NiBr₂, NiI₂, etc.), a palladium halide (for example, PdF₂, PdCl₂, PdBr₂, PdI₂, etc.), a platinum halide (for example, PtF₂, PtCl₂, PtBr₂, PtI₂, etc.), a copper halide (for example, CuF, CuCI, CuBr, Cul, etc.), a silver halide (for example, AgF, AgCl, AgBr, Agl, etc.), and a gold halide (for example, AuF, AuCI, AuBr, Aul, etc.).

Examples of the post-transition metal halide may include a zinc halide (for example, ZnF₂, ZnCl₂, ZnBr₂, ZnI₂, etc.), an indium halide (for example, InI₃, etc.), and a tin halide (for example, SnI₂, etc.).

Examples of the lanthanide metal halide may include YbF, YbF₂, YbF₃, SmF₃, YbCl, YbCl₂, YbCl₃ SmCl₃, YbBr, YbBr₂, YbBr₃ SmBr₃, Ybl, YbI₂, YbI₃, and SmI₃.

Examples of the metalloid halide may include an antimony halide (for example, SbCl₅, etc.).

Examples of the metal telluride may include an alkali metal telluride (for example, Li₂Te, Na₂Te, K₂Te, Rb₂Te, Cs2Te, etc.), an alkaline earth metal telluride (for example, BeTe, MgTe, CaTe, SrTe, BaTe, etc.), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), a post-transition metal telluride (for example, ZnTe, etc.), and a lanthanide metal telluride (for example, LaTe, CeTe, PrTe, NdTe, PmTe, EuTe, GdTe, TbTe, DyTe, HoTe, ErTe, TmTe, YbTe, LuTe, etc.).

### [Emission layer in interlayer 130]

When the light-emitting device 10 is a full-color light-emitting device, the emission layer may be patterned into a red emission layer, a green emission layer, and/or a blue emission layer, according to a subpixel. In embodiments, the emission layer may have a stacked structure of two or more layers of a red emission layer, a green emission layer, and a blue emission layer, in which the two or more layers may contact each other or may be separated from each other to emit white light. In embodiments, the emission layer may include two or more materials of a red light-emitting material, a green light-emitting material, and a blue light-emitting material, in which the two or more materials are mixed with each other in a single layer to emit white light.

The emission layer may include a host and a dopant. The dopant may include a phosphorescent dopant, a fluorescent dopant, or any combination thereof.

An amount of the dopant in the emission layer may be in a range of about 0.01 parts by weight to about 15 parts by weight, based on 100 parts by weight of the host.

In embodiments, the emission layer may include a quantum dot.

In embodiments, the emission layer may include a delayed fluorescence material. The delayed fluorescence material may serve as a host or as a dopant in the emission layer.

A thickness of the emission layer may be in a range of about 100 Å to about 1,000 Å. For example, the thickness of the emission layer may be in a range of about 200 Å to about 600 Å. When the thickness of the emission layer is within these ranges, excellent light-emission characteristics may be obtained without a substantial increase in driving voltage.

### [Host]

In embodiments, the host may include a compound represented by Formula 301:

[Formula 301] [Ar₃₀₁]_{xb11}-[(L₃₀₁)_{xb21}

In Formula 301,
Ar₃₀₁ and L₃₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ,-Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or -P(=O)(Q₃₀₁)(Q₃₀₂),
xb21 may be an integer from 1 to 5, and
Q₃₀₁ to Q₃₀₃ are each independently the same as described herein with respect to Q₁.

In an embodiment, in Formula 301, when xb11 is 2 or more, two or more of Ar₃₀₁(s) may be linked to each other via a single bond.

In embodiments, the host may include a compound represented by Formula 301-1, a compound represented by Formula 301-2, or any combination thereof:

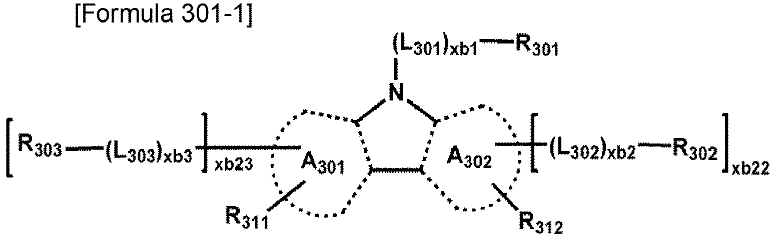

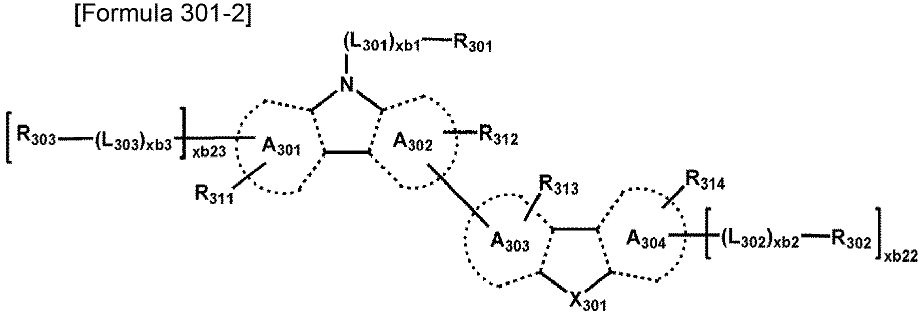

In Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N-[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each be the same as described herein,
L₃₀₂ to L₃₀₄ may each independently be the same as described herein with respect to with L₃₀₁,
xb2 to xb4 may each independently be the same as described herein with respect to xb1, and
R₃₀₂ to R₃₀₅ and R₃₁₁ to R₃₁₄ may each independently be the same as described herein with respect to R₃₀₁.

In embodiments, the host may include an alkali earth metal complex, a post-transition metal complex, or any combination thereof. For example, the host may include a Be complex (for example, Compound H55), an Mg complex, a Zn complex, or any combination thereof.

In an embodiment, the host may include one of Compounds H1 to H125, 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), or any combination thereof:

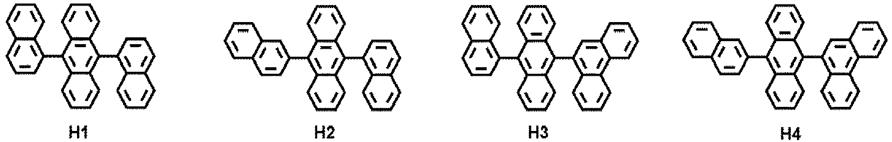

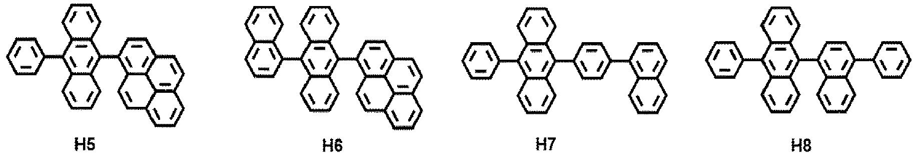

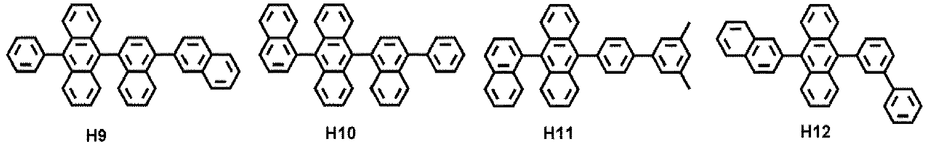

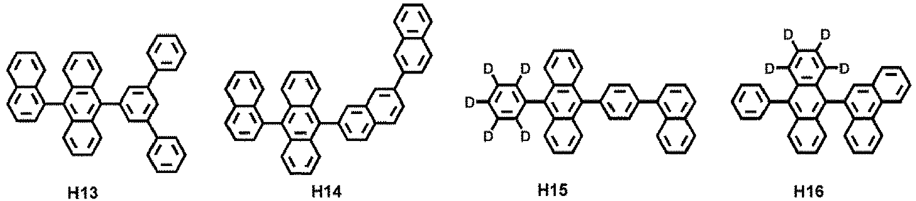

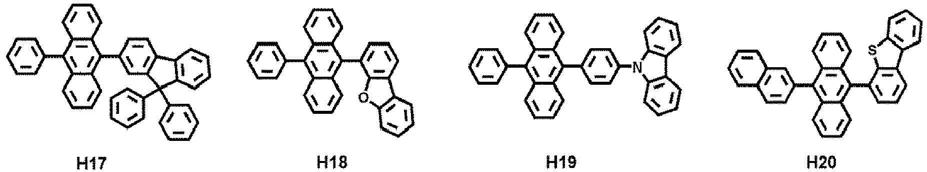

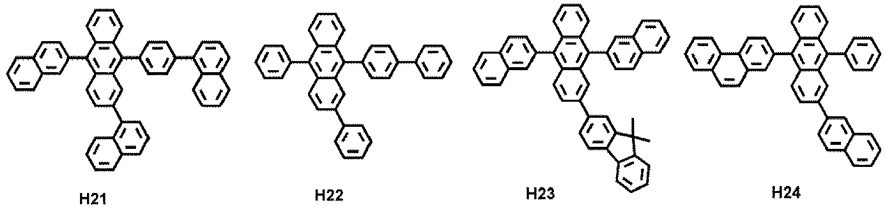

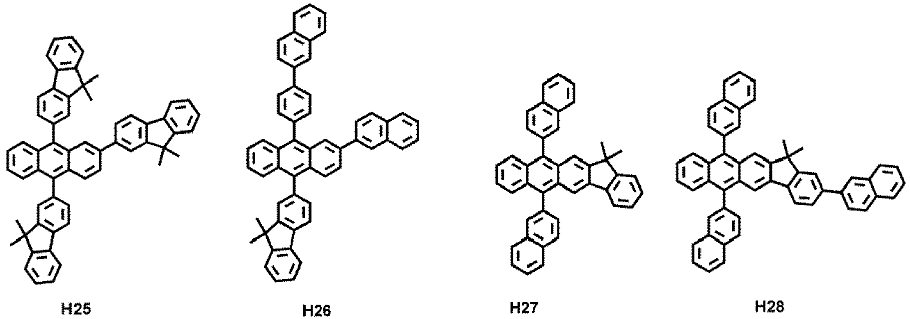

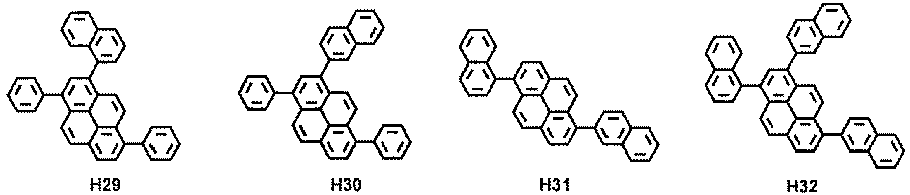

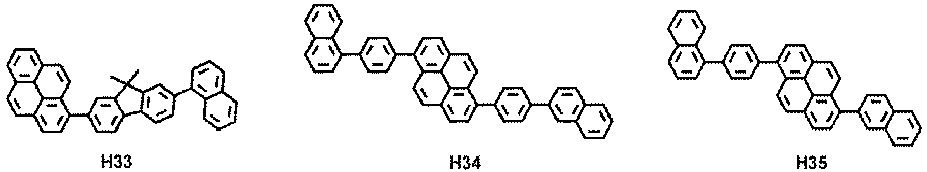

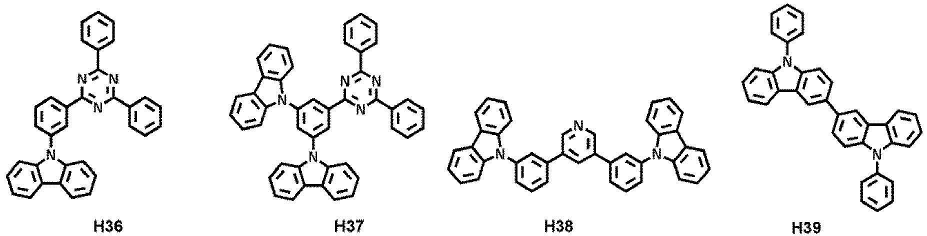

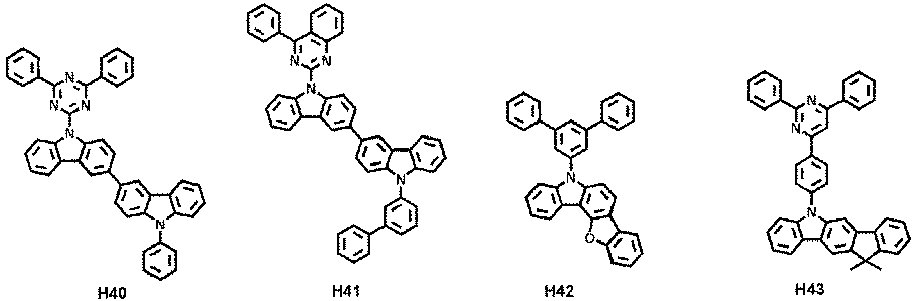

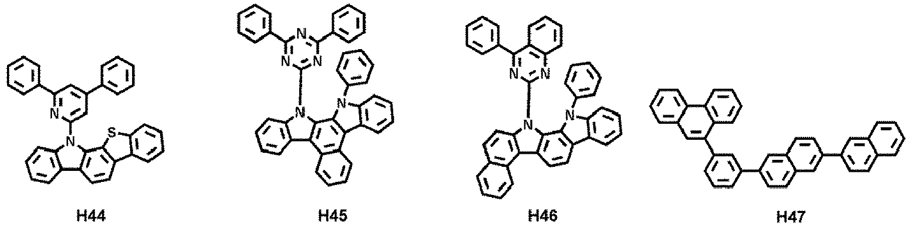

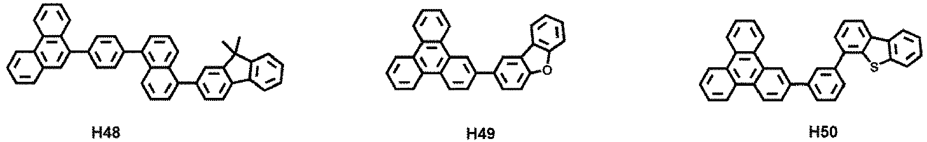

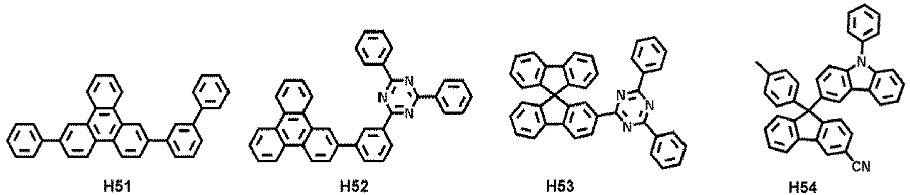

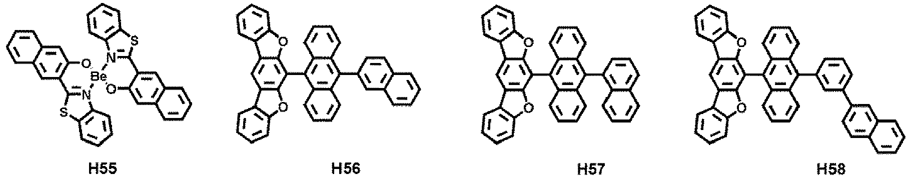

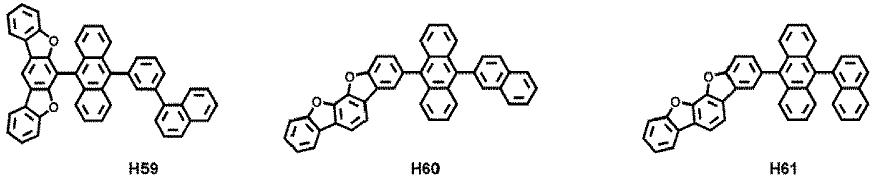

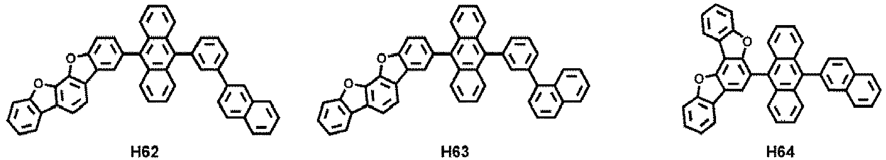

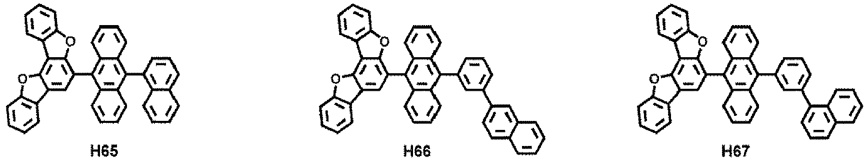

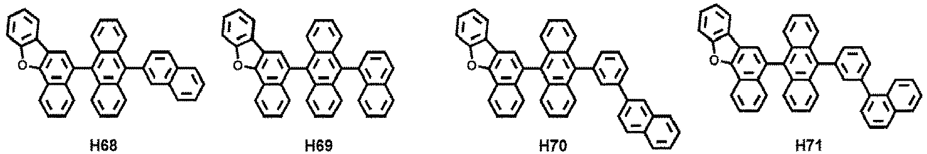

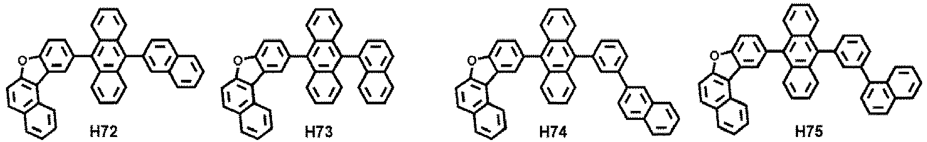

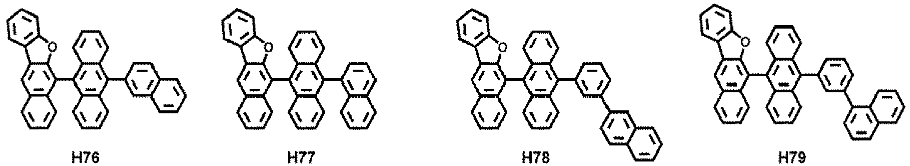

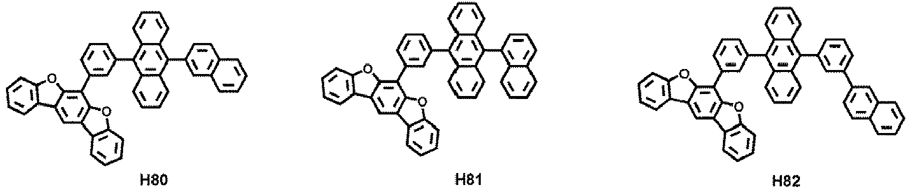

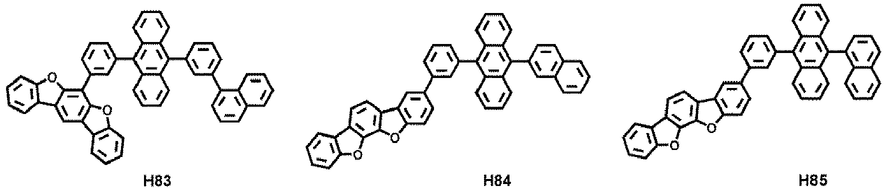

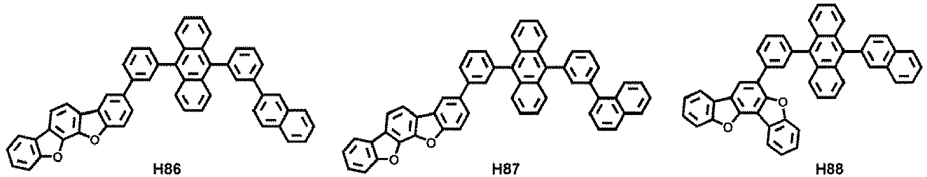

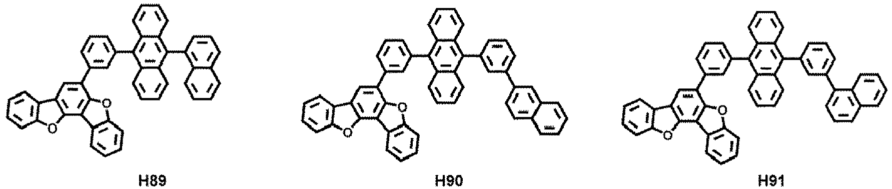

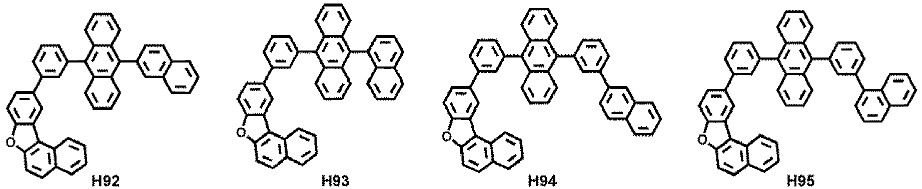

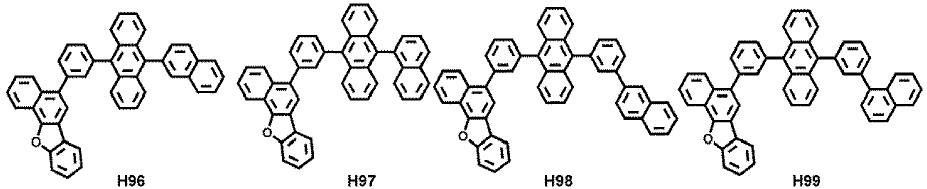

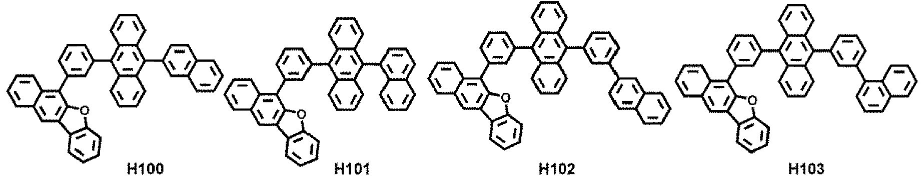

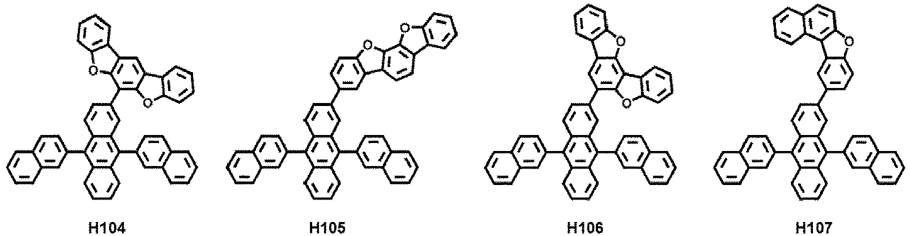

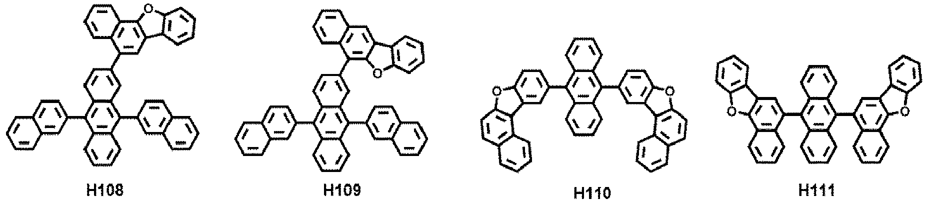

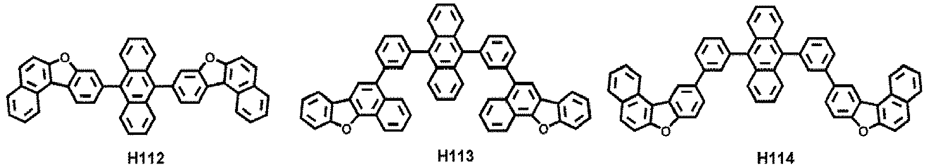

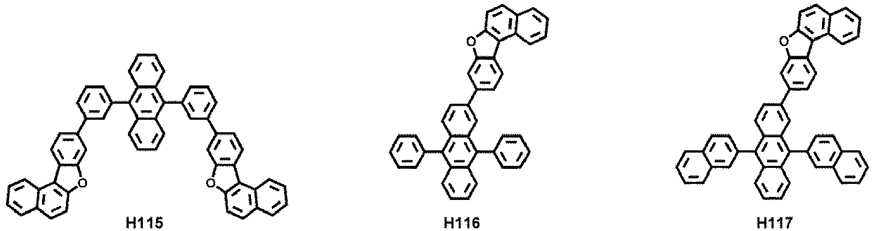

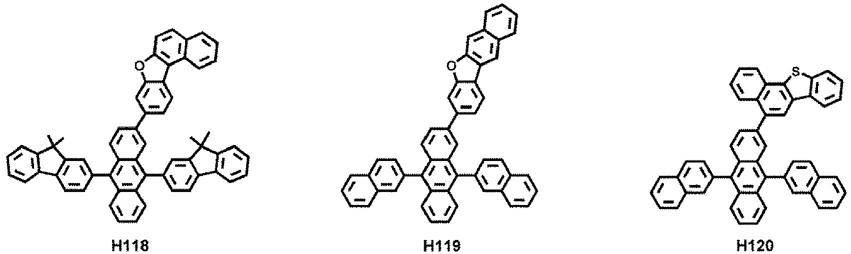

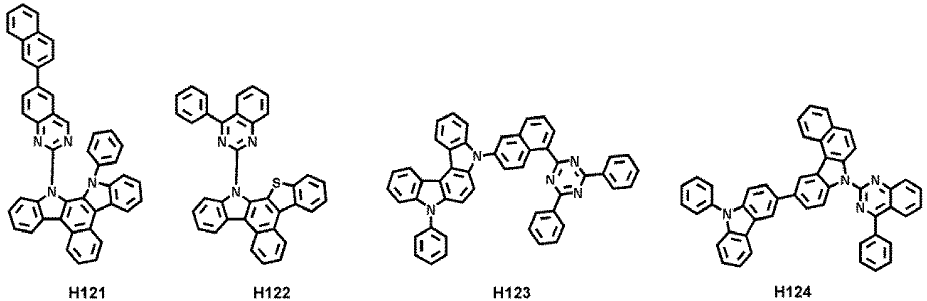

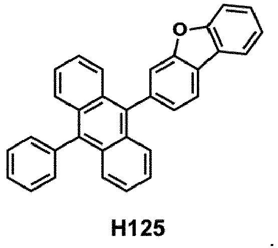

### [Phosphorescent dopant]

In embodiments, the phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In embodiments, the phosphorescent dopant may include an organometallic compound represented by Formula 401:

[Formula 401] M(L₄₀₁)_{xc1}(L₄₀₂)_{xc2}

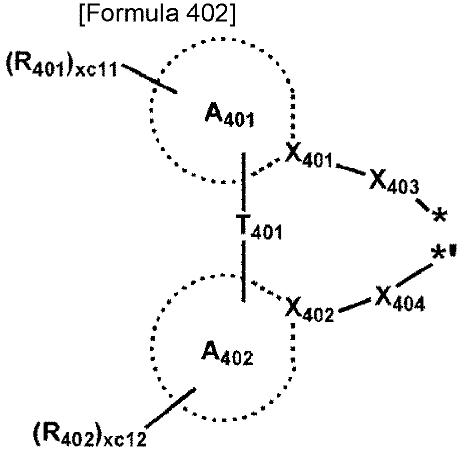

In Formulae 401 and 402,
M may be a transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 402, and xc1 may be 1, 2, or 3, wherein when xc1 is 2 or greater, two or more of L₄₀₁(s) may be identical to or different from each other,
L₄₀₂ may be an organic ligand, and xc2 may be 0, 1, 2, 3, or 4, wherein when xc2 is 2 or more, two or more of L₄₀₂(s) may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *.-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond (for example, a covalent bond or a coordination bond), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ may each independently be the same as described herein with respect to Q₁,
R₄₀₁ and R₄₀₂ may each independently be hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), or -P(=O)(Q₄₀₁)(Q₄₀₂),
Q₄₀₁ to Q₄₀₃ may each independently be the same as described herein with respect to Q₁,
xc11 and xc12 may each independently be an integer from 0 to 10, and
* and *' in Formula 402 each indicate a binding site to M in Formula 401.

For example, in Formula 402, X₄₀₁ may be nitrogen and X₄₀₂ may be carbon, or each of X₄₀₁ and X₄₀₂ may be nitrogen.

In embodiments, in Formula 401, when xc1 is 2 or more, two ring A₄₀₁(s) in two or more of L₄₀₁(s) may be optionally linked to each other via T₄₀₂, which is a linking group, or two ring A₄₀₂(s) may be optionally linked to each other via T₄₀₃, which is a linking group (see Compounds PD1 to PD4 and PD7). T₄₀₂ and T₄₀₃ may each independently be the same as described herein with respect to T₄₀₁.

In Formula 401, L₄₀₂ may be an organic ligand. For example, L₄₀₂ may include a halogen group, a diketone group (for example, an acetylacetonate group), a carboxylic acid group (for example, a picolinate group), -C(=O), an isonitrile group, - CN group, a phosphorus group (for example, a phosphine group, a phosphite group, etc.), or any combination thereof.

The phosphorescent dopant may include, for example, one of Compounds PD1 to PD39, or any combination thereof:

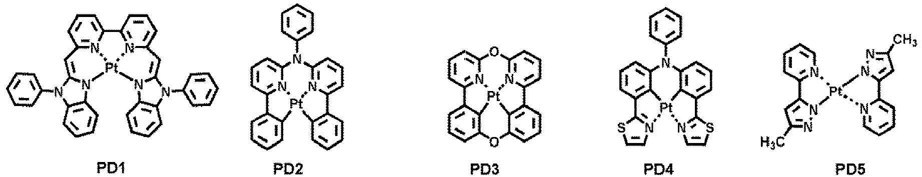

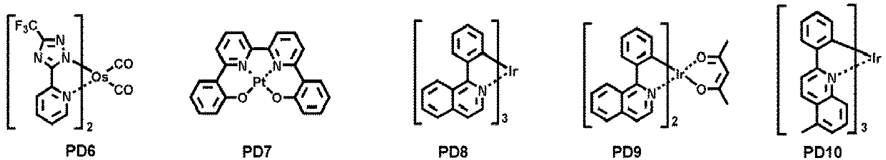

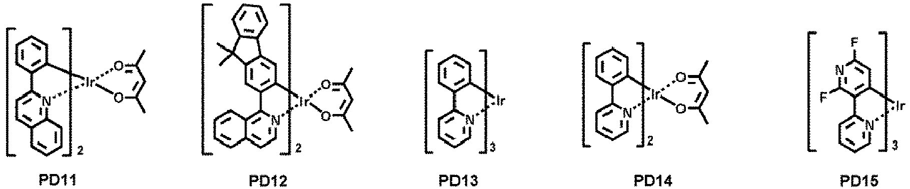

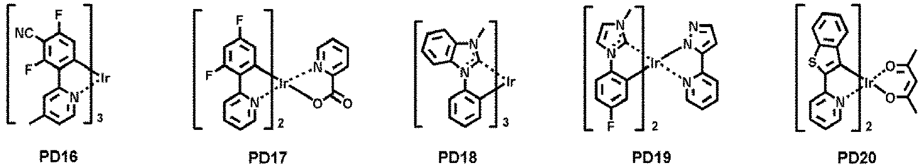

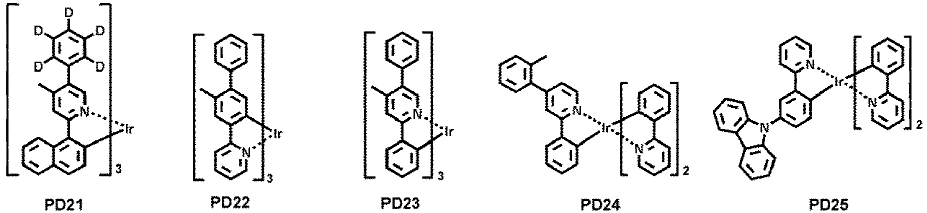

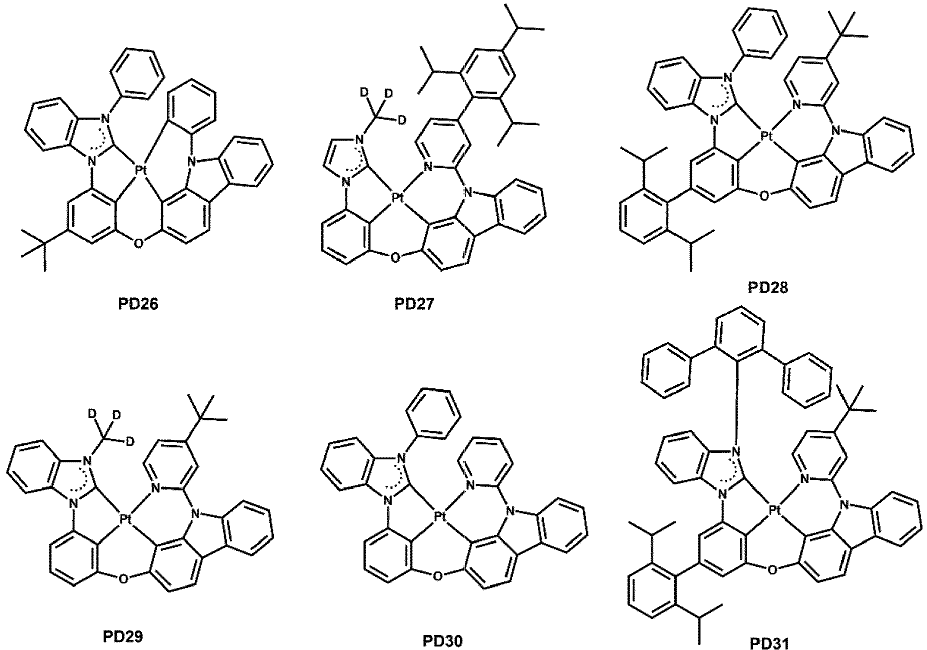

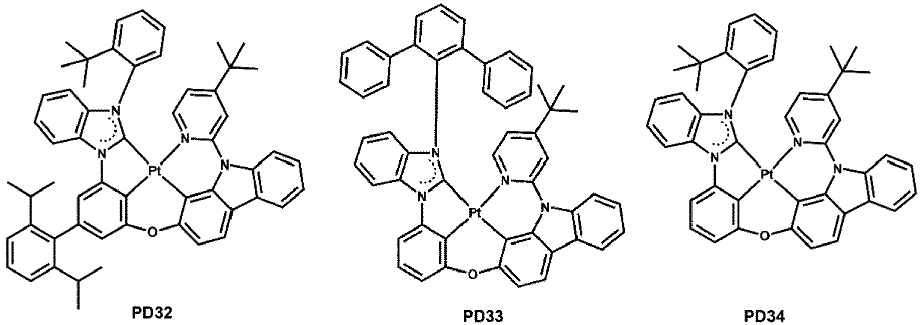

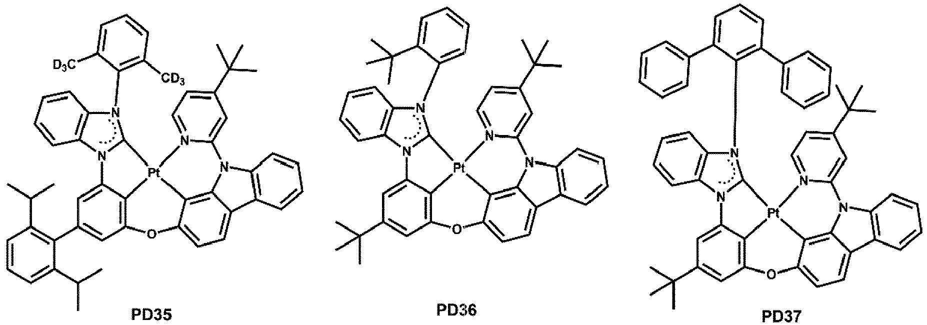

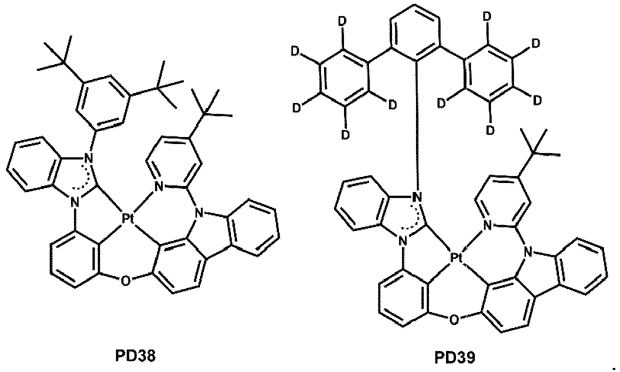

### [Fluorescent dopant]

The fluorescent dopant may include an amine group-containing compound, a styryl group-containing compound, or any combination thereof.

In embodiments, the fluorescent dopant may include a compound represented by Formula 501:

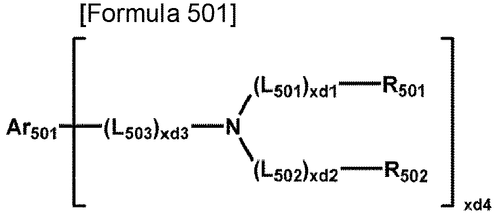

In Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

In an embodiment, in Formula 501, Ar₅₀₁ may be a condensed cyclic group (for example, an anthracene group, a chrysene group, or a pyrene group) in which three or more monocyclic groups are condensed together.

In embodiments, in Formula 501, xd4 may be 2.

For example, the fluorescent dopant may include one of Compounds FD1 to FD36, DPVBi, DPAVBi, or any combination thereof:

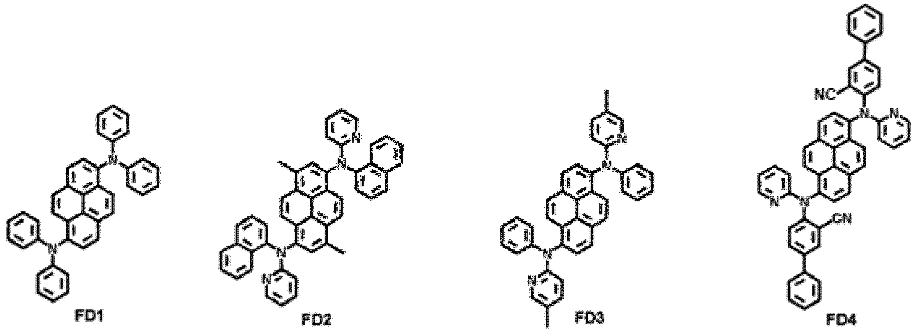

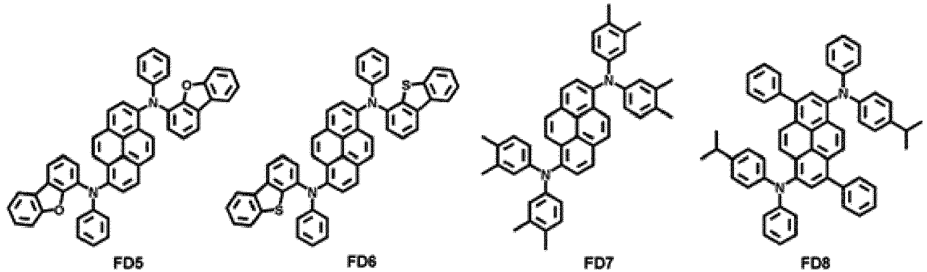

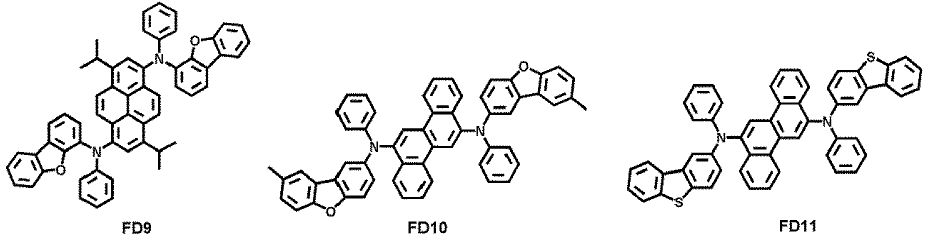

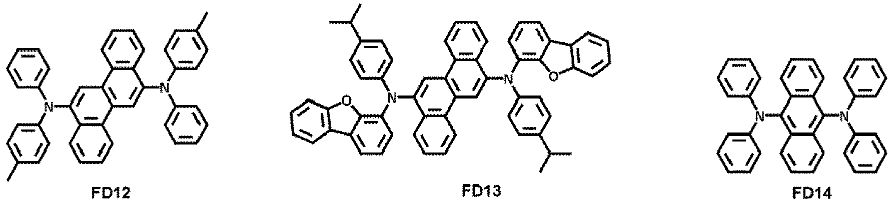

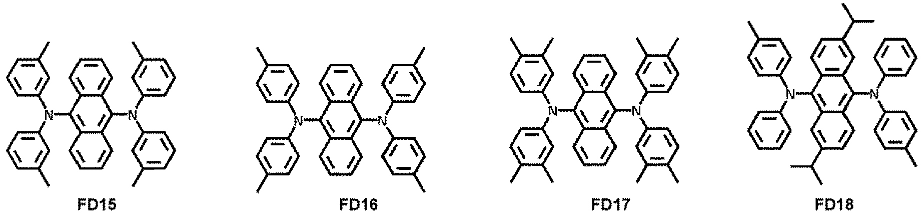

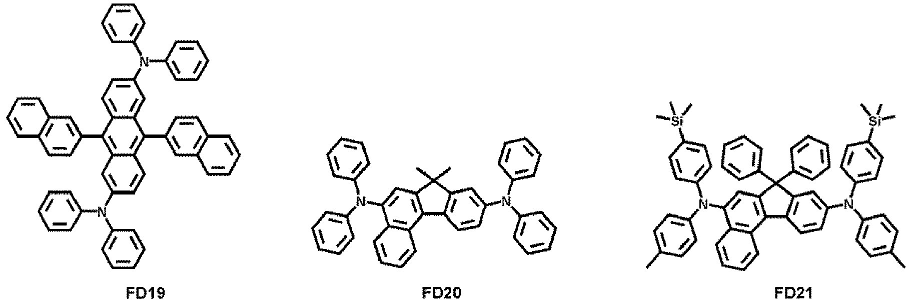

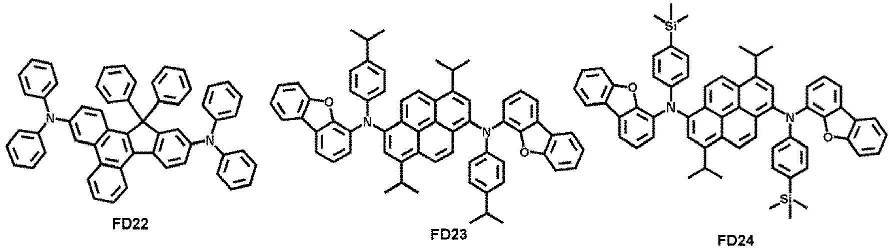

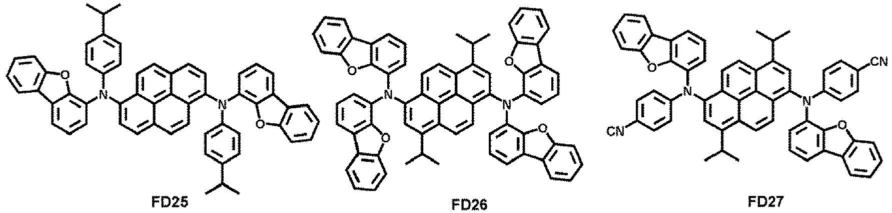

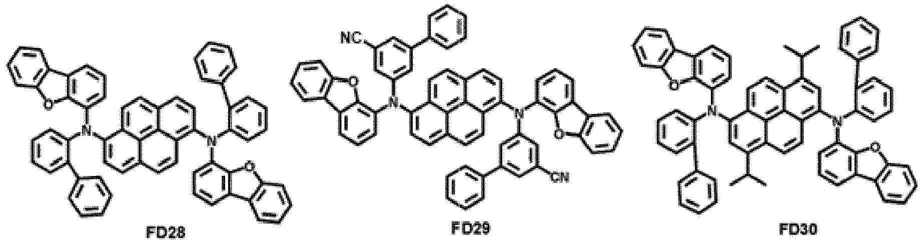

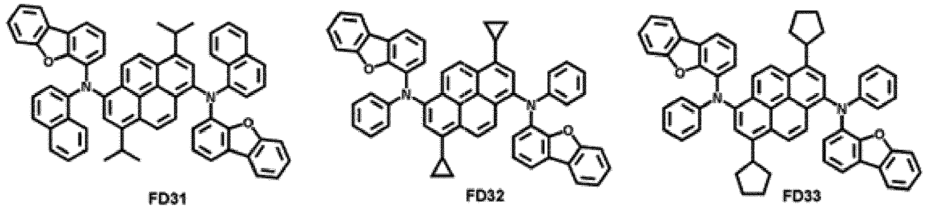

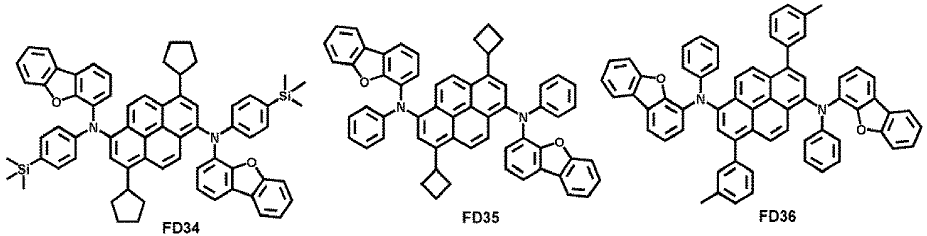

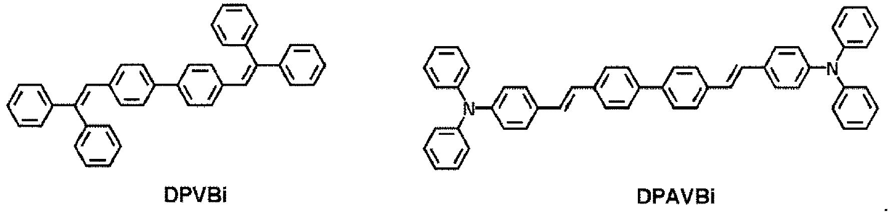

### [Delayed fluorescence material]

The emission layer may include a delayed fluorescence material.

In the specification, the delayed fluorescence material may be selected from compounds capable of emitting delayed fluorescent light based on a delayed fluorescence emission mechanism.

The delayed fluorescence material included in the emission layer may serve as a host or as a dopant, depending on the type of other materials included in the emission layer.

In embodiments, a difference between a triplet energy level (eV) of the delayed fluorescence material and a singlet energy level (eV) of the delayed fluorescence material may be greater than or equal to about 0 eV and less than or equal to about 0.5 eV. When the difference between the triplet energy level (eV) of the delayed fluorescence material and the singlet energy level (eV) of the delayed fluorescence material satisfies the above-described range, up-conversion from the triplet state to the singlet state of the delayed fluorescence materials may effectively occur, and thus, the luminescence efficiency of the light-emitting device 10 may be improved.

In embodiments, the delayed fluorescence material may include: a material including at least one electron donor (for example, a π electron-rich C₃-C₆₀ cyclic group, such as a carbazole group) and at least one electron acceptor (for example, a sulfoxide group, a cyano group, or a π electron-deficient nitrogen-containing C₁-C₆₀ cyclic group); or a material including a C₈-C₆₀ polycyclic group in which two or more cyclic groups are condensed while sharing boron (B).

Examples of the delayed fluorescence material may include at least one of Compounds DF1 to DF9:

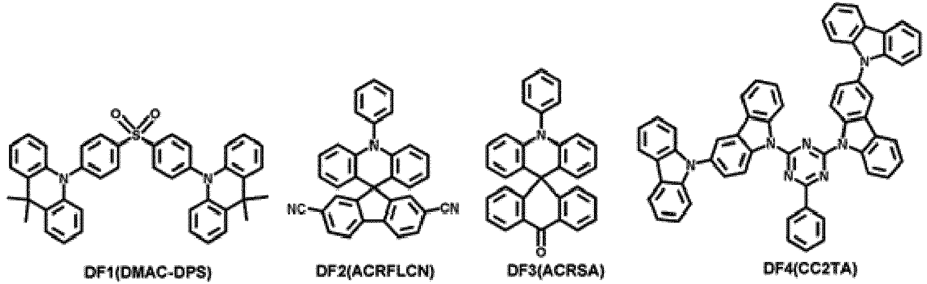

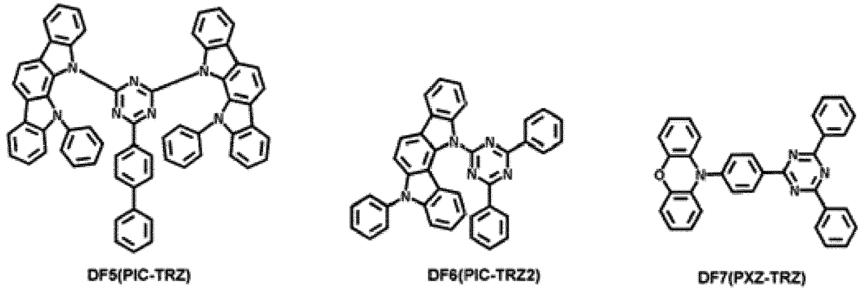

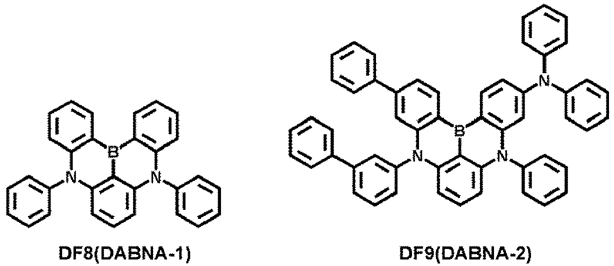

### [Quantum dot]

The emission layer may include a quantum dot.

In the specification, a quantum may be a crystal of a semiconductor compound, and may include any material capable of emitting light of various emission wavelengths according to a size of the crystal.

A diameter of the quantum dot may be, for example, in a range of about 1 nm to about 10 nm.

The quantum dot may be synthesized by a wet chemical process, a metal organic chemical vapor deposition process, a molecular beam epitaxy process, or any process similar thereto.

The wet chemical process is a method including mixing a precursor material with an organic solvent and growing a quantum dot particle crystal. When the crystal grows, the organic solvent naturally acts as a dispersant coordinated on the surface of the quantum dot crystal and controls the growth of the crystal so that the growth of quantum dot particles can be controlled through a process which costs lower, and may be more readily performed than vapor deposition methods, such as metal organic chemical vapor deposition (MOCVD) or molecular beam epitaxy (MBE),

The quantum dot may include Group II-VI semiconductor compounds, Group III-V semiconductor compounds, Group III-VI semiconductor compounds, Group I-III-VI semiconductor compounds, Group IV-VI semiconductor compounds, a Group IV element or compound, or any combination thereof.

Examples of the Group II-VI semiconductor compound may include: a binary compound, such as CdS, CdSe, CdTe, ZnS, ZnSe, ZnTe, ZnO, HgS, HgSe, HgTe, MgSe, or MgS; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, or MgZnS; a quaternary compound, such as CdZnSeS, CdZnSeTe, CdZnSTe, CdHgSeS, CdHgSeTe, CdHgSTe, HgZnSeS, HgZnSeTe, or HgZnSTe; or any combination thereof.

Examples of the Group III-V semiconductor compound may include: a binary compound, such as GaN, GaP, GaAs, GaSb, AIN, AIP, AlAs, AlSb, InN, InP, InAs, or InSb; a ternary compound, such as GaNP, GaNAs, GaNSb, GaPAs, GaPSb, AINP, AINAs, AINSb, AIPAs, AlPSb, InGaP, InNP, InAlP, InNAs, InNSb, InPAs, or InPSb; a quaternary compound, such as GaAINP, GaAINAs, GaAINSb, GaAIPAs, GaAIPSb, GalnNP, GalnNAs, GaInNSb, GaInPAs, GalnPSb, InAINP, InAINAs, InAINSb, InAIPAs, or InAIPSb; or any combination thereof. In an embodiment, the Group III-V semiconductor compound may further include a Group II element. Examples of the Group III-V semiconductor compound further including a Group II element may include InZnP, InGaZnP, InAIZnP, etc.

Examples of the Group III-VI semiconductor compound may include: a binary compound, such as GaS, GaSe, Ga₂Se₃, GaTe, InS, InSe, In₂S₃, ln₂Se₃, or InTe; a ternary compound, such as InGaS₃, or InGaSe₃; and any combination thereof.

Examples of the Group I-III-VI semiconductor compound may include: a ternary compound, such as AglnS, AgInS₂, CulnS, CuInS₂, CuGaO₂, AgGaO₂, or AgAlO₂; or any combination thereof.

Examples of the Group IV-VI semiconductor compound may include: a binary compound, such as SnS, SnSe, SnTe, PbS, PbSe, or PbTe; a ternary compound, such as SnSeS, SnSeTe, SnSTe, PbSeS, PbSeTe, PbSTe, SnPbS, SnPbSe, or SnPbTe; a quaternary compound, such as SnPbSSe, SnPbSeTe, or SnPbSTe; or any combination thereof.

Examples of the Group IV element or compound may include: a single element material, such as Si or Ge; a binary compound, such as SiC or SiGe; or any combination thereof.

Each element included in a multi-element compound such as a binary compound, a ternary compound, or a quaternary compound may be present in a particle at a uniform concentration or at a non-uniform concentration.

In embodiments, the quantum dot may have a single structure in which the concentration of each element in the quantum dot may be uniform, or the quantum dot may have a core-shell structure. For example, when the quantum dot has a core-shell structure, a material included in the core and a material included in the shell may be different from each other.

The shell of the quantum dot may serve as a protective layer that prevents chemical degeneration of the core to maintain semiconductor characteristics, and/or may serve as a charging layer that imparts electrophoretic characteristics to the quantum dot. The shell may be a single layer or a multi-layer. An interface between the core and the shell may have a concentration gradient in which the concentration of a material that is present in the shell decreases toward the core.

Examples of the shell of the quantum dot may include a metal oxide, a metalloid oxide, a non-metal oxide, a semiconductor compound, or any combination thereof. Examples of the metal oxide, the metalloid oxide, or the non-metal oxide may include: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, or NiO; a ternary compound, such as MgAl₂O₄, CoFe₂O₄, NiFe₂O₄, or CoMn₂O₄; or any combination thereof. Examples of the semiconductor compound may include, as described herein, a Group II-VI semiconductor compound, a Group III-V semiconductor compound, a Group III-VI semiconductor compound, a Group I-III-VI semiconductor compound, a Group IV-VI semiconductor compound, or any combination thereof. For example, the semiconductor compound may include CdS, CdSe, CdTe, ZnS, ZnSe, ZnTe, ZnSeS, ZinTeS, GaAs, GaP, GaSb, HgS, HgSe, HgTe, InAs, InP, InGaP, InSb, AlAs, AIP, AlSb, or any combination thereof.

A full width at half maximum (FWHM) of an emission wavelength spectrum of the quantum dot may be equal to or less than about 45 nm. For example, a FWHM of an emission wavelength spectrum of the quantum dot may be equal to or less than about 40 nm. For example, a FWHM of an emission wavelength spectrum of the quantum dot may be equal to or less than about 30 nm. Within these ranges, color purity or color reproducibility may be increased. Light emitted through the quantum dot may be emitted in all directions, so that a wide viewing angle may be improved.

The quantum dot may be in the form of a spherical particle, a pyramidal particle, a multi-arm particle, a cubic nanoparticle, a nanotube particle, a nanowire particle, a nanofiber particle, or a nanoplate particle.

Since the energy band gap may be adjusted by controlling the size of the quantum dot, light having various wavelength bands may be obtained from the quantum dot emission layer. Accordingly, by using quantum dots of different sizes, a light-emitting device that emits light of various wavelengths may be implemented. In embodiments, the size of the quantum dot may be selected to emit red, green, and/or blue light. For example, the size of the quantum dot may be configured to emit white light by combination of light of various colors.

### [Electron transport region in interlayer 130]

The electron transport region may have a structure consisting of a layer consisting of a single material, a structure consisting of a layer consisting of different materials, or a structure including multiple layers including different materials.

The electron transport region may include a buffer layer, a hole-blocking layer, an electron control layer, an electron transport layer, an electron injection layer, or any combination thereof.

For example, the electron transport region may have an electron transport layer/electron injection layer structure, a hole-blocking layer/electron transport layer/electron injection layer structure, an electron control layer/electron transport layer/electron injection layer structure, or a buffer layer/electron transport layer/electron injection layer structure, wherein the layers of each structure may be stacked from an emission layer in its respective stated order, but the structure of the electron transport region is not limited thereto.

In an embodiment, the electron transport region (for example, the buffer layer, the hole-blocking layer, the electron control layer, or the electron transport layer in the electron transport region) may include a metal-free compound including at least one π electron-deficient nitrogen-containing C₁-C₆₀ cyclic group.

For example, the electron transport region may include a compound represented by Formula 601:

[Formula 601] [Ar₆₀₁]ₓₑ₁₁-[(L₆₀₁)ₓₑ₁-R₆₀₁]ₓₑ₂₁

In Formula 601,
Ar₆₀₁ and L₆₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or-P(=O)(Q₆₀₁)(Q₆₀₂),
Q₆₀₁ to Q₆₀₃ may each independently be the same as described herein with respect to Q₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group unsubstituted or substituted with at least one R₁₀ₐ.

In an embodiment, in Formula 601, when xe11 is 2 or more, two or more of Ar₆₀₁(s) may be linked to each other via a single bond.

In embodiments, in Formula 601, Ar₆₀₁ may be a substituted or unsubstituted anthracene group.

In embodiments, the electron transport region may include a compound represented by Formula 601-1:

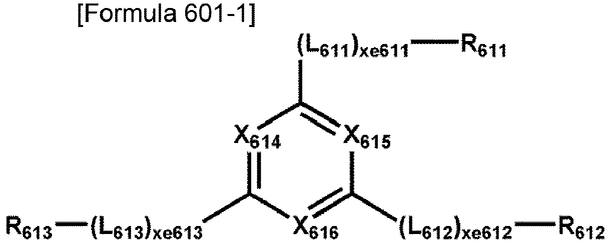

In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may be N,
L₆₁₁ to L₆₁₃ may each independently be the same as described herein with respect to L₆₀₁,
xe611 to xe613 may each independently be the same as described herein with respect to xe1,
R₆₁₁ to R₆₁₃ may each independently be the same as described herein with respect to R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group unsubstituted or substituted with at least one R₁₀ₐ, or a C₁-C₆₀ heterocyclic group unsubstituted or substituted with at least one R₁₀ₐ.

In an embodiment, in Formulae 601 and 601-1, xe1 and xe611 to xe613 may each independently be 0, 1, or 2.

The electron transport region may include one of Compounds ET1 to ET45, 2,9-dimethyl-4,7-diphenyl-1,10-phenanthroline (BCP), 4,7-diphenyl-1,10-phenanthroline (Bphen), Alq₃, BAlq, TAZ, NTAZ, or any combination thereof:

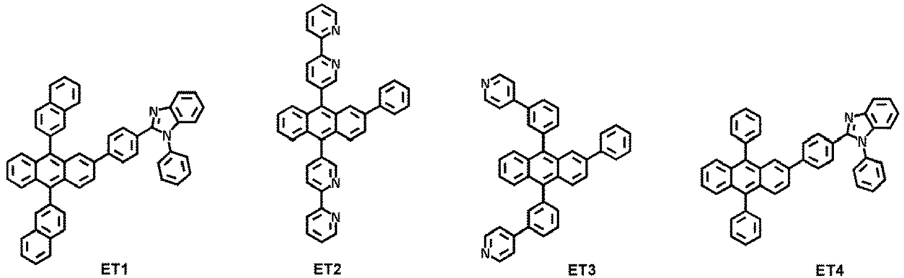

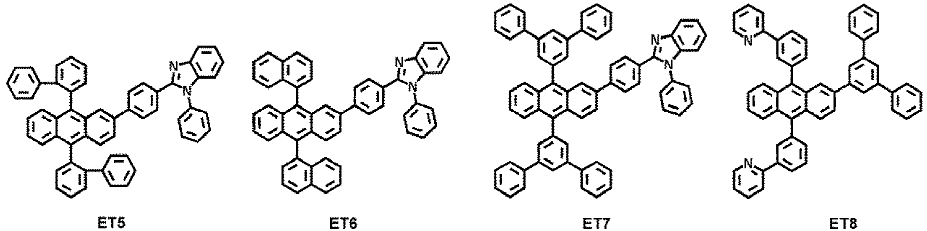

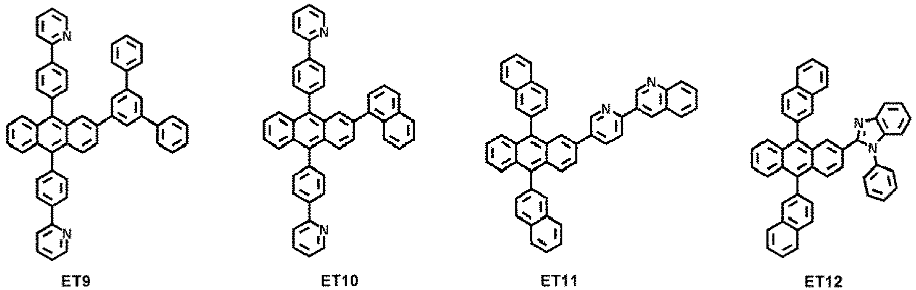

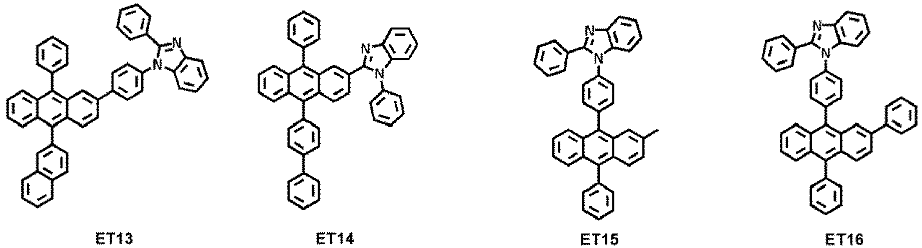

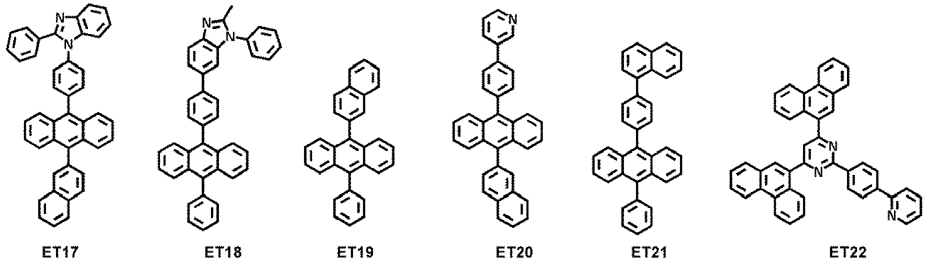

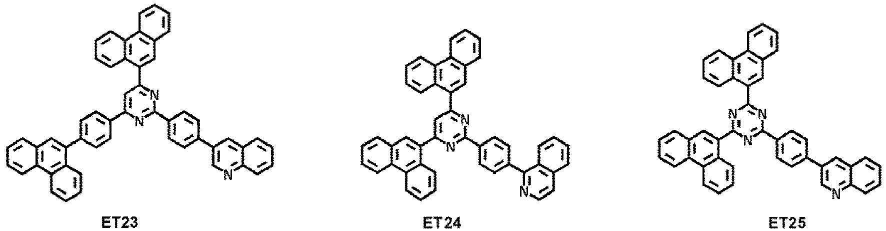

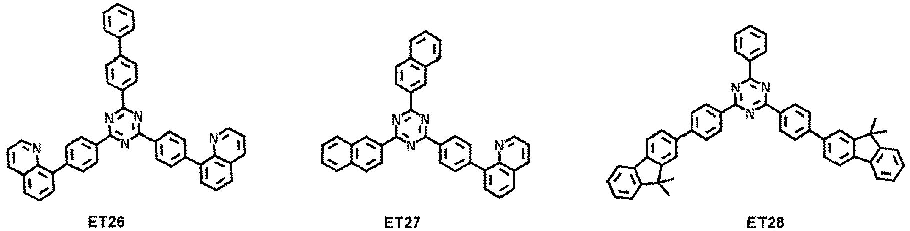

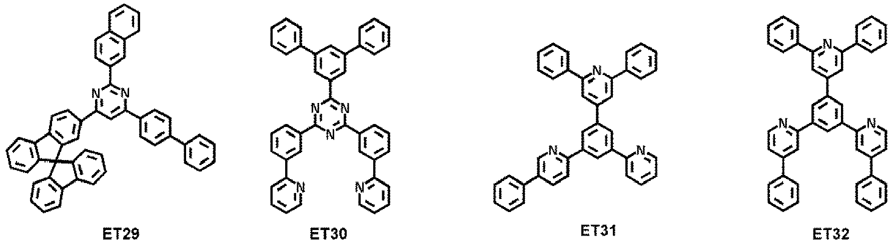

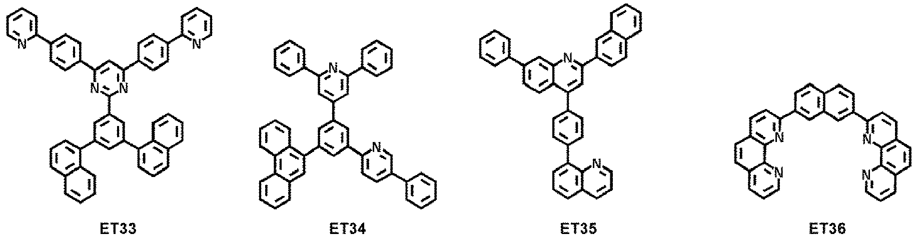

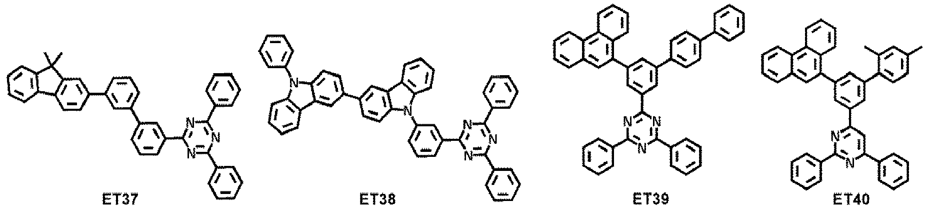

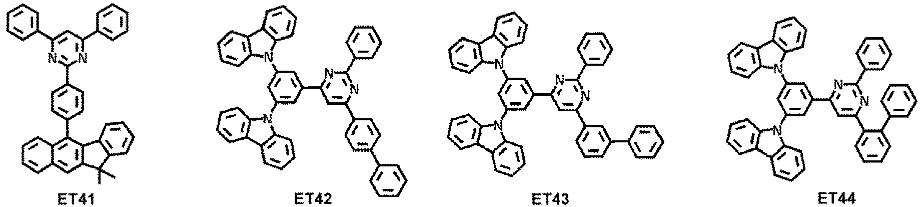

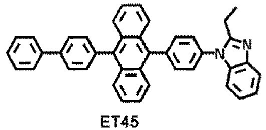

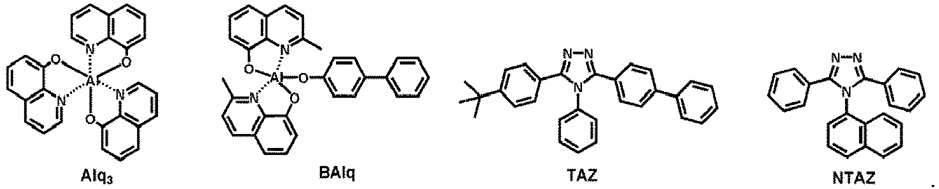

A thickness of the electron transport region may be in a range of about 100 Å to about 5,000 Å. For example, the thickness of the electron transport region may be in a range of about 160 Å to about 4,000 Å. When the electron transport region includes a buffer layer, a hole-blocking layer, an electron control layer, an electron transport layer, or any combination thereof, a thickness of the buffer layer, the hole-blocking layer, or the electron control layer may each independently be in a range of about 20 Å to about 1,000 Å, and a thickness of the electron transport layer may be in a range of about 100 Å to about 1,000 Å. For example, the thickness of the buffer layer, the hole-blocking layer, or the electron control layer may each independently be in a range of about 30 Å to about 300 Å. For example, the thickness of the electron transport layer may be in a range of about 150 Å to about 500 Å. When the thicknesses of the buffer layer, the hole-blocking layer, the electron control layer, the electron transport layer, and/or the electron transport region are within these ranges, satisfactory electron transporting characteristics may be obtained without a substantial increase in driving voltage.

The electron transport region (for example, the electron transport layer in the electron transport region) may further include, in addition to the materials described above, a metal-containing material.

The metal-containing material may include an alkali metal complex, an alkaline earth metal complex, or any combination thereof. A metal ion of an alkali metal complex may be a Li ion, a Na ion, a K ion, a Rb ion, or a Cs ion, and a metal ion of an alkaline earth metal complex may be a Be ion, a Mg ion, a Ca ion, a Sr ion, or a Ba ion. A ligand coordinated with the metal ion of the alkali metal complex or with the metal ion of the alkaline earth-metal complex may each independently include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

In an embodiment, the metal-containing material may include a Li complex. The Li complex may include, for example, Compound ET-D1 (LiQ) or Compound ET-D2:

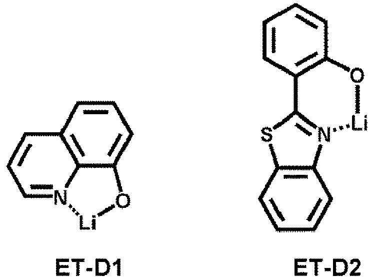

The electron transport region may include an electron injection layer that facilitates the injection of electrons from the second electrode 150. The electron injection layer may directly contact the second electrode 150.

The electron injection layer may have a structure consisting of a layer consisting of a single material, a structure consisting of a layer consisting of different materials, or a structure including multiple layers including different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may include oxides, halides (for example, fluorides, chlorides, bromides, or iodides), or tellurides of the alkali metal, the alkaline earth metal, and the rare earth metal, or any combination thereof.

The alkali metal-containing compound may include: alkali metal oxides, such as Li₂O, Cs₂O, or K₂O; alkali metal halides, such as LiF, NaF, CsF, KF, Lil, Nal, Csl, or KI; or any combination thereof. The alkaline earth metal-containing compound may include an alkaline earth metal compound, such as BaO, SrO, CaO, BaₓSr₁₋ₓO (wherein x is a real number satisfying the condition of 0<x<1), BaₓCa₁₋ₓO (wherein x is a real number satisfying the condition of 0<x<1), or the like. The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In embodiments, the rare earth metal-containing compound may include a lanthanide metal telluride. Examples of the lanthanide metal telluride may include LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, and Lu₂Te₃.

The alkali metal complex, the alkaline earth-metal complex, and the rare earth metal complex may include one of ions of the alkali metal, ions of the alkaline earth metal, and ions of the rare earth metal, and a ligand bound to the metal ion (for example, hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenyl benzimidazole, hydroxyphenylbenzothiazole, bipyridine, phenanthroline, cyclopentadiene, or any combination thereof).

The electron injection layer may consist of an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In embodiments, the electron injection layer may further include an organic material (for example, a compound represented by Formula 601).

In embodiments, the electron injection layer may consist of an alkali metal-containing compound (for example, an alkali metal halide); or the electron injection layer may consist of an alkali metal-containing compound (for example, an alkali metal halide), and an alkali metal, an alkaline earth metal, a rare earth metal, or any combination thereof. For example, the electron injection layer may be a KI:Yb co-deposited layer, an RbI:Yb co-deposited layer, a LiF:Yb co-deposited layer, or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 1 Å to about 100 Å. For example, the thickness of the electron injection layer may be in a range of about 3 Å to about 90 Å. When the thickness of the electron injection layer is within the ranges described above, satisfactory electron injection characteristics may be obtained without a substantial increase in driving voltage.

### [Second electrode 150]

The second electrode 150 may be located on the interlayer 130 having a structure as described herein. The second electrode 150 may be a cathode, which is an electron injection electrode. The second electrode 150 may include a material having a low-work function, for example, a metal, an alloy, an electrically conductive compound, or any combination thereof.

In embodiments, the second electrode 150 may include lithium (Li), silver (Ag), magnesium (Mg), aluminum (Al), aluminum-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a semi-transmissive electrode, or a reflective electrode.

The second electrode 150 may have a single-layered structure or a multilayered structure.

### [Capping layer]

The light-emitting device 10 may include a first capping layer located outside the first electrode 110, and/or a second capping layer located outside the second electrode 150. For example, the light-emitting device 10 may have a structure in which the first capping layer, the first electrode 110, the interlayer 130, and the second electrode 150 are stacked in this stated order, a structure in which the first electrode 110, the interlayer 130, the second electrode 150, and the second capping layer are stacked in this stated order, or a structure in which the first capping layer, the first electrode 110, the interlayer 130, the second electrode 150, and the second capping layer are stacked in this stated order.

Light generated in an emission layer of the interlayer 130 of the light-emitting device 10 may be extracted toward the outside through the first electrode 110, which may be a semi-transmissive electrode or a transmissive electrode, and through the first capping layer. Light generated in an emission layer of the interlayer 130 of the light-emitting device 10 may be extracted toward the outside through the second electrode 150, which may be a semi-transmissive electrode or a transmissive electrode, and through the second capping layer.

The first capping layer and the second capping layer may each increase external emission efficiency according to the principle of constructive interference. Accordingly, the light extraction efficiency of the light-emitting device 10 may be increased, so that the luminescence efficiency of the light-emitting device 10 may be improved.

The first capping layer and the second capping layer may each include a material having a refractive index equal to or greater than about 1.6 (with respect to a wavelength of about 589 nm).

The first capping layer and the second capping layer may each independently be an organic capping layer including an organic material, an inorganic capping layer including an inorganic material, or an organic-inorganic composite capping layer including an organic material and an inorganic material.

At least one of the first capping layer and the second capping layer may each independently include carbocyclic compounds, heterocyclic compounds, amine group-containing compounds, porphine derivatives, phthalocyanine derivatives, naphthalocyanine derivatives, alkali metal complexes, alkaline earth metal complexes, or any combination thereof. The carbocyclic compound, the heterocyclic compound, and the amine group-containing compound may be optionally substituted with a substituent including O, N, S, Se, Si, F, Cl, Br, I, or any combination thereof.

In embodiments, at least one of the first capping layer and the second capping layer may each independently include an amine group-containing compound.

For example, at least one of the first capping layer and the second capping layer may each independently include a compound represented by Formula 201, a compound represented by Formula 202, or any combination thereof.

In embodiments, at least one of the first capping layer and the second capping layer may each independently include one of Compounds HT28 to HT33, one of Compounds CP1 to CP6, β-NPB, or any combination thereof:

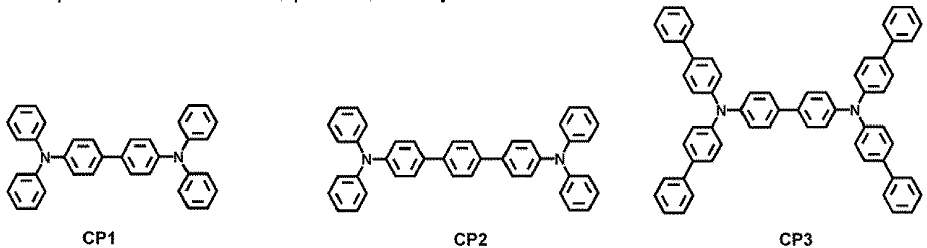

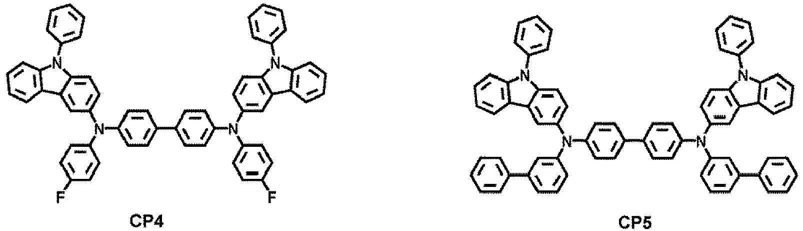

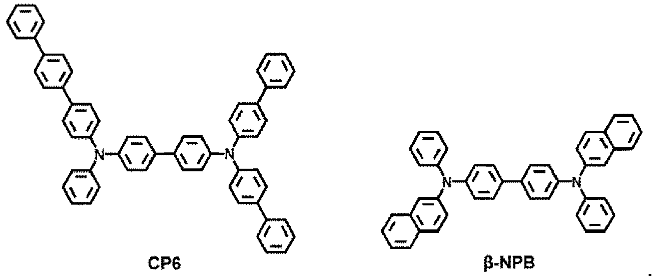

### [Film]

The heterocyclic compound represented by Formula 1 may be included in various films. Accordingly, another aspect provides a film including the heterocyclic compound represented by Formula 1. The film may be, for example, an optical member (or a light control means) (for example, a color filter, a color conversion member, a capping layer, a light extraction efficiency enhancement layer, a selective light absorbing layer, a polarizing layer, a quantum dot-containing layer, or the like), a light-blocking member (for example, a light reflective layer, a light absorbing layer, or the like), or a protective member (for example, an insulating layer, a dielectric layer, or the like).

### [Electronic apparatus]

The light-emitting device may be included in various electronic apparatuses. For example, the electronic apparatus including the light-emitting device may be a light-emitting apparatus, an authentication apparatus, or the like.

The electronic apparatus (for example, a light-emitting apparatus) may further include, in addition to the light-emitting device, a color filter, a color conversion layer, or a color filter and a color conversion layer. The color filter and/or the color conversion layer may be located in at least one direction in which light emitted from the light-emitting device travels. For example, the light emitted from the light-emitting device may be blue light or white light. The light-emitting device may be the same as described herein. In embodiments, the color conversion layer may include a quantum dot. The quantum dot may be, for example, a quantum dot as described herein.

The electronic apparatus may include a first substrate. The first substrate may include subpixels, the color filter may include color filter areas respectively corresponding to the subpixels, and the color conversion layer may include color conversion areas respectively corresponding to the subpixels.

A pixel-defining film may be located between the subpixels to define each subpixel.

The color filter may further include color filter areas and light-shielding patterns located between the color filter areas, and the color conversion layer may further include color conversion areas and light-shielding patterns located between the color conversion areas.

The color filter areas (or the color conversion areas) may include a first area emitting first color light, a second area emitting second color light, and/or a third area emitting third color light, wherein the first color light, the second color light, and/or the third color light may have different maximum emission wavelengths from one another. For example, the first color light may be red light, the second color light may be green light, and the third color light may be blue light. In embodiments, the color filter areas (or the color conversion areas) may include quantum dots. For example, the first area may include a red quantum dot, the second area may include a green quantum dot, and the third area may not include a quantum dot. The quantum dot may be the same as described herein. The first area, the second area, and/or the third area may each include a scatterer.

For example, the light-emitting device may emit first light, the first area may absorb the first light to emit first-first color light, the second area may absorb the first light to emit second-first color light, and the third area may absorb the first light to emit third-first color light. In this regard, the first-first color light, the second-first color light, and the third-first color light may have different maximum emission wavelengths. For example, the first light may be blue light, the first-first color light may be red light, the second-first color light may be green light, and the third-first color light may be blue light.

The electronic apparatus may further include a thin-film transistor, in addition to the light-emitting device as described herein. The thin-film transistor may include a source electrode, a drain electrode, and an active layer, wherein any one of the source electrode and the drain electrode may be electrically connected to any one of the first electrode and the second electrode of the light-emitting device.

The thin-film transistor may further include a gate electrode, a gate insulating film, or the like.

The active layer may include crystalline silicon, amorphous silicon, an organic semiconductor, an oxide semiconductor, or the like.

The electronic apparatus may further include a sealing portion for sealing the light-emitting device. The sealing portion may be located between the color filter and/or the color conversion layer, and the light-emitting device. The sealing portion may allow light from the light-emitting device to be extracted to the outside, and may simultaneously prevent ambient air and moisture from penetrating into the light-emitting device. The sealing portion may be a sealing substrate including a transparent glass substrate or a plastic substrate. The sealing portion may be a thin-film encapsulation layer including an organic layer and/or an inorganic layer. When the sealing portion is a thin-film encapsulation layer, the electronic apparatus may be flexible.

Various functional layers may be further included on the sealing portion, in addition to the color filter and/or the color conversion layer, according to the use of the electronic apparatus. Examples of the functional layers may include a touch screen layer, a polarizing layer, an authentication apparatus, and the like. The touch screen layer may be a pressure-sensitive touch screen layer, a capacitive touch screen layer, or an infrared touch screen layer. The authentication apparatus may be, for example, a biometric authentication apparatus that authenticates an individual by using biometric information of a living body (for example, fingertips, pupils, etc.).

The authentication apparatus may further include, in addition to the light-emitting device as described herein, a biometric information collector.

The electronic apparatus may be applied to various displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various measuring instruments, meters (for example, meters for a vehicle, an aircraft, and a vessel), projectors, and the like.

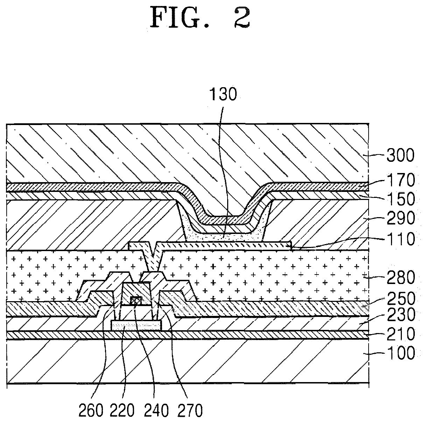

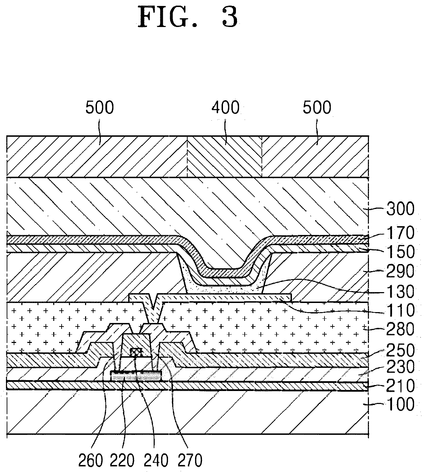

### [Description of FIGS. 2 and 3]

FIG. 2 is a schematic cross-sectional view showing an electronic apparatus according to an embodiment.

The electronic apparatus of FIG. 2 includes a substrate 100, a thin-film transistor (TFT), a light-emitting device, and an encapsulation portion 300 that seals the light-emitting device.

The substrate 100 may be a flexible substrate, a glass substrate, or a metal substrate. A buffer layer 210 may be located on the substrate 100. The buffer layer 210 may prevent penetration of impurities through the substrate 100 and may provide a flat surface on the substrate 100.

A TFT may be located on the buffer layer 210. The TFT may include an active layer 220, a gate electrode 240, a source electrode 260, and a drain electrode 270.

The active layer 220 may include an inorganic semiconductor such as silicon or polysilicon, an organic semiconductor, or an oxide semiconductor, and may include a source region, a drain region, and a channel region.

A gate insulating film 230 for insulating the active layer 220 from the gate electrode 240 may be located on the active layer 220, and the gate electrode 240 may be located on the gate insulating film 230.

An interlayer insulating film 250 may be located on the gate electrode 240. The interlayer insulating film 250 may be located between the gate electrode 240 and the source electrode 260 to insulate the gate electrode 240 from the source electrode 260 and between the gate electrode 240 and the drain electrode 270 to insulate the gate electrode 240 from the drain electrode 270.

The source electrode 260 and the drain electrode 270 may be located on the interlayer insulating film 250. The interlayer insulating film 250 and the gate insulating film 230 may expose the source region and the drain region of the active layer 220, and the source electrode 260 and the drain electrode 270 may respectively contact the exposed portions of the source region and the drain region of the active layer 220.

The TFT is electrically connected to a light-emitting device to drive the light-emitting device, and is covered and protected by a passivation layer 280. The passivation layer 280 may include an inorganic insulating film, an organic insulating film, or any combination thereof. A light-emitting device is provided on the passivation layer 280. The light-emitting device may include a first electrode 110, an interlayer 130, and a second electrode 150.

The first electrode 110 may be located on the passivation layer 280. The passivation layer 280 does not fully cover the drain electrode 270 and may expose a portion of the drain electrode 270, and the first electrode 110 may be electrically connected to the exposed portion of the drain electrode 270.

A pixel defining layer 290 including an insulating material may be located on the first electrode 110. The pixel defining layer 290 may expose a region of the first electrode 110, and an interlayer 130 may be formed in the exposed region of the first electrode 110. The pixel defining layer 290 may be a polyimide or polyacrylic organic film. Although not shown in FIG. 2, at least some layers of the interlayer 130 may extend beyond the upper portion of the pixel defining layer 290 to be provided in the form of a common layer.

The second electrode 150 may be located on the interlayer 130, and a capping layer 170 may be further included on the second electrode 150. The capping layer 170 may cover the second electrode 150.

The encapsulation portion 300 may be located on the capping layer 170. The encapsulation portion 300 may be located on a light-emitting device to protect the light-emitting device from moisture and/or oxygen. The encapsulation portion 300 may include: an inorganic film including silicon nitride (SiNx), silicon oxide (SiOx), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, or the like), an epoxy-based resin (for example, aliphatic glycidyl ether (AGE), or the like), or any combination thereof; or any combination of the inorganic films and the organic films.

FIG. 3 is a schematic cross-sectional view showing an electronic apparatus according to another embodiment.

The electronic apparatus of FIG. 3 may differ from the electronic apparatus of FIG. 2, at least in that a light-shielding pattern 500 and a functional region 400 are further included on the encapsulation portion 300. The functional region 400 may be a color filter area, a color conversion area, or a combination of the color filter area and the color conversion area. In an embodiment, the light-emitting device included in the electronic apparatus of FIG. 3 may be a tandem light-emitting device.

### [Manufacturing method]

The layers included in the hole transport region, the emission layer, and the layers included in the electron transport region may be formed in a certain region by using various methods such as vacuum deposition, spin coating, casting, Langmuir-Blodgett (LB) deposition, ink-jet printing, laser-printing, laser-induced thermal imaging, and the like.

When layers constituting the hole transport region, an emission layer, and layers constituting the electron transport region are formed by vacuum deposition, the deposition may be performed at a deposition temperature of about 100 °C to about 500 °C, a vacuum degree of about 10⁻⁸ torr to about 10⁻³ torr, and a deposition speed of about 0.01 Å/sec to about 100 Å/sec, depending on a material to be included in a layer to be formed and the structure of a layer to be formed.

### [Definitions of Terms]

The term "C₃-C₆₀ carbocyclic group" as used herein may be a cyclic group consisting of carbon as the only ring-forming atoms and having three to sixty carbon atoms, and the term "C₁-C₆₀ heterocyclic group" as used herein may be a cyclic group that has one to sixty carbon atoms and further has, in addition to carbon, at least one heteroatom as a ring-forming atom. The C₃-C₆₀ carbocyclic group and the C₁-C₆₀ heterocyclic group may each be a monocyclic group consisting of one ring or a polycyclic group in which two or more rings are condensed with each other. For example, the C₁-C₆₀ heterocyclic group may have 3 to 61 ring-forming atoms.

The term "cyclic group" as used herein may include the C₃-C₆₀ carbocyclic group or the C₁-C₆₀ heterocyclic group.

The term "π electron-rich C₃-C₆₀ cyclic group" as used herein may be a cyclic group that has three to sixty carbon atoms and may not include *-N=*' as a ring-forming moiety, and the term "π electron-deficient nitrogen-containing C₁-C₆₀ cyclic group" as used herein may be a heterocyclic group that has one to sixty carbon atoms and may include *-N=*' as a ring-forming moiety.

In embodiments,
the C₃-C₆₀ carbocyclic group may be a T1 group or a cyclic group in which two or more T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
the C₁-C₆₀ heterocyclic group may be a T2 group, a cyclic group in which two or more T2 groups are condensed with each other, or a cyclic group in which at least one T2 group and at least one T1 group are condensed with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.),
the π electron-rich C₃-C₆₀ cyclic group may be a T1 group, a cyclic group in which two or more T1 groups are condensed with each other, a T3 group, a cyclic group in which two or more T3 groups are condensed with each other, or a cyclic group in which at least one T3 group and at least one T1 group are condensed with each other (for example, the C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, etc.),
the π electron-deficient nitrogen-containing C₁-C₆₀ cyclic group may be a T4 group, a cyclic group in which two or more T4 groups are condensed with each other, a cyclic group in which at least one T4 group and at least one T1 group are condensed with each other, a cyclic group in which at least one T4 group and at least one T3 group are condensed with each other, or a cyclic group in which at least one T4 group, at least one T1 group, and at least one T3 group are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, etc.),
wherein the T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or a bicyclo[2.2.1]heptane) group, a norbornene group, a bicyclo[1.1.1]pentane group, a bicyclo[2.1.1]hexane group, a bicyclo[2.2.2]octane group, or a benzene group,
the T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
the T3 group may be a furan group, a thiophene group, a 1H-pyrrole group, a silole group, or a borole group, and
the T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group", "C₃-C₆₀ carbocyclic group", "C₁-C₆₀ heterocyclic group", "π electron-rich C₃-C₆₀ cyclic group", or "π electron-deficient nitrogen-containing C₁-C₆₀ cyclic group" as used herein may each be a group condensed to any cyclic group, a monovalent group, or a polyvalent group (for example, a divalent group, a trivalent group, a tetravalent group, etc.) according to the structure of a formula for which the corresponding term is used. For example, a "benzene group" may be a benzo group, a phenyl group, a phenylene group, or the like, which may be readily understood by one of ordinary skill in the art according to the structure of a formula including the "benzene group."

Examples of the monovalent C₃-C₆₀ carbocyclic group and the monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group. Examples of the divalent C₃-C₆₀ carbocyclic group and the divalent C₁-C₆₀ heterocyclic group may include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a substituted or unsubstituted divalent non-aromatic condensed heteropolycyclic group.

The term "C₁-C₆₀ alkyl group" as used herein may be a linear or branched aliphatic hydrocarbon monovalent group that has one to sixty carbon atoms, and examples thereof may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isoctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group. The term "C₁-C₆₀ alkylene group" as used herein may be a divalent group having a same structure as the C₁-C₆₀ alkyl group.

The term "C₂-C₆₀ alkenyl group" as used herein may be a monovalent hydrocarbon group having at least one carbon-carbon double bond in the middle or at a terminus of the C₂-C₆₀ alkyl group, and examples thereof may include an ethenyl group, a propenyl group, and a butenyl group. The term "C₂-C₆₀ alkenylene group" as used herein may be a divalent group having a same structure as the C₂-C₆₀ alkenyl group.

The term "C₂-C₆₀ alkynyl group" as used herein may be a monovalent hydrocarbon group having at least one carbon-carbon triple bond in the middle or at a terminus of the C₂-C₆₀ alkyl group, and examples thereof may include an ethynyl group and a propynyl group. The term "C₂-C₆₀ alkynylene group" as used herein may be a divalent group having a same structure as the C₂-C₆₀ alkynyl group.

The term "C₁-C₆₀ alkoxy group" as used herein may be a monovalent group represented by -O(A₁₀₁) (wherein A₁₀₁ may be a C₁-C₆₀ alkyl group), and examples thereof may include a methoxy group, an ethoxy group, and an isopropyloxy group.

The term "C₃-C₁₀ cycloalkyl group" as used herein may be a monovalent saturated hydrocarbon cyclic group having 3 to 10 carbon atoms, and examples thereof may include a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, and a bicyclo[2.2.2]octyl group. The term "C₃-C₁₀ cycloalkylene group" as used herein may be a divalent group having a same structure as the C₃-C₁₀ cycloalkyl group.

The term "C₁-C₁₀ heterocycloalkyl group" as used herein may be a monovalent cyclic group of 1 to 10 carbon atoms, further including, in addition to carbon atoms, at least one heteroatom, as ring-forming atoms, and examples thereof may include a 1,2,3,4-oxatriazolidinyl group, a tetrahydrofuranyl group, and a tetrahydrothiophenyl group. The term "C₁-C₁₀ heterocycloalkylene group" as used herein may be a divalent group having a same structure as the C₁-C₁₀ heterocycloalkyl group.

The term "C₃-C₁₀ cycloalkenyl group" as used herein may be a monovalent cyclic group that has three to ten carbon atoms and at least one carbon-carbon double bond in the ring thereof and no aromaticity, and examples thereof may include a cyclopentenyl group, a cyclohexenyl group, and a cycloheptenyl group. The term "C₃-C₁₀ cycloalkenylene group" as used herein may be a divalent group having a same structure as the C₃-C₁₀ cycloalkenyl group.

The term "C₁-C₁₀ heterocycloalkenyl group" as used herein may be a monovalent cyclic group of 1 to 10 carbon atoms, further including, in addition to carbon atoms, at least one heteroatom, as ring-forming atoms, and having at least one carbon-carbon double bond in the cyclic structure thereof. Examples of the C₁-C₁₀ heterocycloalkenyl group may include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group" as used herein may be a divalent group having a same structure as the C₁-C₁₀ heterocycloalkenyl group.

The term "C₆-C₆₀ aryl group" as used herein may be a monovalent group having a carbocyclic aromatic system of 6 to 60 carbon atoms, and the term "C₆-C₆₀ arylene group" as used herein may be a divalent group having a carbocyclic aromatic system of 6 to 60 carbon atoms. Examples of the C₆-C₆₀ aryl group may include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group. When the C₆-C₆₀ aryl group and the C₆-C₆₀ arylene group each include two or more rings, the respective rings may be condensed with each other.

The term "C₁-C₆₀ heteroaryl group" as used herein may be a monovalent group having a heterocyclic aromatic system of 1 to 60 carbon atoms, further including, in addition to carbon atoms, at least one heteroatom, as ring-forming atoms. The term "C₁-C₆₀ heteroarylene group" as used herein may be a divalent group having a heterocyclic aromatic system of 1 to 60 carbon atoms, further including, in addition to carbon atoms, at least one heteroatom, as ring-forming atoms. Examples of the C₁-C₆₀ heteroaryl group may include a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group. When the C₁-C₆₀ heteroaryl group and the C₁-C₆₀ heteroarylene group each include two or more rings, the respective rings may be condensed with each other.

The term "monovalent non-aromatic condensed polycyclic group" as used herein may be a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group may include an indenyl group, a fluorenyl group, a spiro-bifluorenyl group, a benzofluorenyl group, an indenophenanthrenyl group, and an indeno anthracenyl group. The term "divalent non-aromatic condensed polycyclic group" as used herein may be a divalent group having a same structure as the monovalent non-aromatic condensed polycyclic group described above.

The term "monovalent non-aromatic condensed heteropolycyclic group" as used herein may be a monovalent group (for example, having 1 to 60 carbon atoms) having two or more rings condensed to each other, further including, in addition to carbon atoms, at least one heteroatom, as ring-forming atoms, and having non-aromaticity in its entire molecular structure. Examples of the monovalent non-aromatic condensed heteropolycyclic group may include a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indeno carbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphtho silolyl group, a benzofurodibenzofuranyl group, a benzofurodibenzothiophenyl group, and a benzothienodibenzothiophenyl group. The term "divalent non-aromatic condensed heteropolycyclic group" as used herein may be a divalent group having a same structure as the monovalent non-aromatic condensed heteropolycyclic group described above.

The term "C₆-C₆₀ aryloxy group" as used herein may be a group represented by -O(A₁₀₂) (wherein A₁₀₂ may be a C₆-C₆₀ aryl group), and the term "C₆-C₆₀ arylthio group" as used herein may be a group represented by -S(A₁₀₃) (wherein A₁₀₃ may be a C₆-C₆₀ aryl group).

The term "C₇₋C₆₀ aryl alkyl group" as used herein may be a group represented by -(A₁₀₄)(A₁₀₅) (wherein A₁₀₄ may be a C₁-C₅₄ alkylene group, and A₁₀₅ may be a C₆-C₅₉ aryl group), and the term "C₂-C₆₀ heteroaryl alkyl group" as used herein may be a group represented by -(A₁₀₆)(A₁₀₇) (wherein A₁₀₆ may be a C₁-C₅₀ alkylene group, and A₁₀₇ may be a C₁-C₅₉ heteroaryl group).

The group "R₁₀ₐ" as used herein may be:
deuterium (-D), -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂),
wherein the groups Q₁ to Q₃, Q₁₁ to Q₁₃, Q₂₁ to Q₂₃ and Q₃₁ to Q₃₃ as used herein may each independently be: hydrogen, deuterium, -F, -Cl, -Br, -I, a hydroxyl group, a cyano group, or a nitro group; a C₁-C₆₀ alkyl group, a C₂-C₆₀ alkenyl group, a C₂-C₆₀ alkynyl group, a C₁-C₆₀ alkoxy group, a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof; or a C₇-C₆₀ aryl alkyl group; or a C₂-C₆₀ heteroaryl alkyl group.

The term "heteroatom" as used herein may be any atom other than a carbon atom or a hydrogen atom. Examples of the heteroatom may include O, S, N, P, Si, B, Ge, Se, or any combination thereof.

The term "third-row transition metal" as used herein may include hafnium (Hf), tantalum (Ta), tungsten (W), rhenium (Re), osmium (Os), iridium (Ir), platinum (Pt), gold (Au), and the like.

The term "Ph" as used herein refers to a phenyl group, the term "Me" as used herein refers to a methyl group, the term "Et" as used herein refers to an ethyl group, the terms "ter-Bu" or "Bu^{t}" as used herein each refer to a tert-butyl group, and the term "OMe" as used herein refers to a methoxy group.

The term "biphenyl group" as used herein may be a "phenyl group substituted with a phenyl group." For example, the "biphenyl group" may be a substituted phenyl group having a C₆-C₆₀ aryl group as a substituent.

The term "terphenyl group" as used herein may be a "phenyl group substituted with a biphenyl group". For example, the "terphenyl group" may be a substituted phenyl group having, as a substituent, a C₆-C₆₀ aryl group substituted with a C₆-C₆₀ aryl group.

The symbols * and *' as used herein, unless defined otherwise, each refer to a binding site to a neighboring atom in a corresponding formula or moiety.

Hereinafter, compounds according to embodiments and light-emitting devices according to embodiments will be described in detail with reference to the Synthesis Examples and Examples. The wording "B was used instead of A" used in describing Synthesis Examples means that an identical molar equivalent of B was used in place of A.

### [Examples]

### Synthesis Example 1: Synthesis of Compound 1

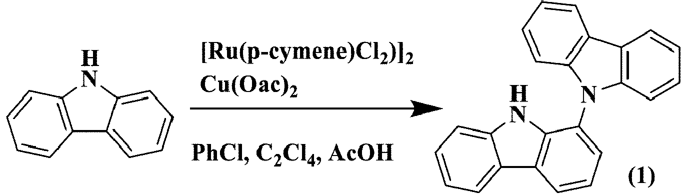

### Synthesis of Intermediate compound (1)

Carbazole (10 g, 59 mmol), [(p-cymene) RuCl₂]₂ (0.36 g, 0.596 mmol), and Cu(OAc)₂ (1.07 g, 5.9 mmol) were dissolved in 100 mL of chlorobenzene, 100 mL of tetrachloroethene, and 20 mL of acetic acid in a 250 mL round-bottom flask, and caused to react at a temperature of 140 °C for 24 hours. When the reaction was completed, the reaction product was cooled to room temperature, filtered through celite, and the solvent was removed therefrom through distillation. After separation through column chromatography, the separated solution was concentrated and dried to obtain Intermediate compound (1) (6.1 g, 31%). ¹H NMR (300 MHz, CD₂Cl₂): δ 8.35 (t, 3H), 8.22 (d, 1H), 7.93 (br, 1H), 7.68 (d, 1H), 7.52-7.28 (m, 8H), 7.22 (d, 2H).

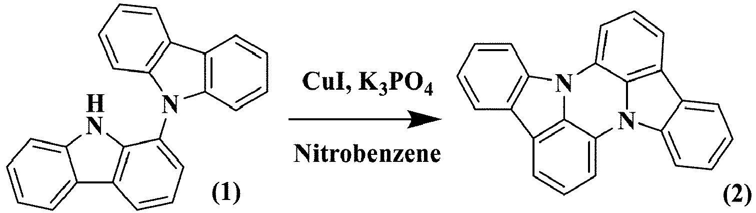

### Synthesis of Intermediate compound (2)

Intermediate compound (1) (20 g, 60.17 mmol), Cul (2.3 g, 12.03 mmol), and K₃PO₄ (25.54 g, 120.33 mmol) were dissolved in 150 mL of nitrobenzene in a 250 mL round-bottom flask, and caused to react at a temperature of 200 °C for 24 hours. When the reaction was completed, the reaction product was cooled to room temperature, filtered through celite, and the solvent was removed therefrom through distillation. Intermediate compound (2) (8 g, 40%) was obtained by separation through column chromatography. ¹H NMR (300 MHz, DMSO-d6) : δ 8.25 (t, 4H), 7.71-7.78 (t, 4H), 7.62 (t, 2H), 7.39 (t, 2H), 7.23 (t, 2H).

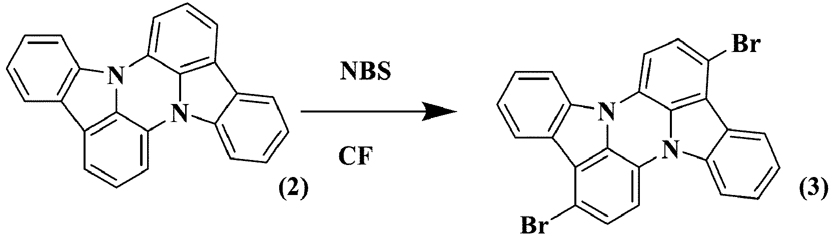

### Synthesis of Intermediate compound (3)

Intermediate compound (2) (1g, 3.02 mmol) was dissolved in chloroform in a 500 mL round-bottom flask, and NBS (0.9g, 4.8 mmol) was added thereto and reacted therewith at room temperature for 16 hours. When the reaction was completed and the Intermediate compound (3) is generated, the solid was filtered and washed with hexane, and the next reaction was conducted without purification.

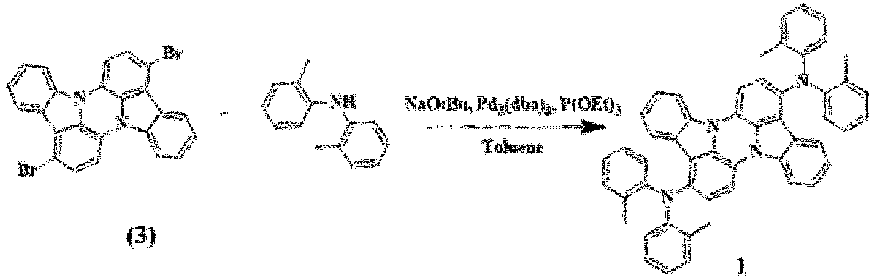

### Synthesis of Compound 1

Intermediate compound (3) (1 g, 2.44 mmol), di-o-tolylamine (1.9 g, 9.76 mmol), NaOtBu (0.47 g, 4.88 mmol), Pd₂(dba)₃ (0.11 g, 0.12 mmol), and 0.1 mL of P(t-Bu)₃ were dissolved in 20 mL of toluene, in a 250 mL round-bottom flask, and refluxed at a temperature of 80 °C for 6 hours. After the reaction was completed, the solvent was concentrated after cooling to room temperature, and separated through column chromatography to obtain Compound 1 (0.7 g, 47%).

### Synthesis Example 2: Synthesis of Compound 4

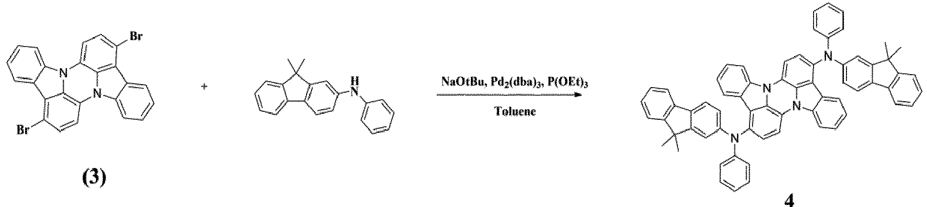

Intermediate compound (3) (1 g, 2.44 mmol), 9,9-dimethyl-N-phenyl-9H-fluoren-2-amine (2.8 g, 9.76 mmol), NaOtBu (0.47 g, 4.88 mmol), Pd₂(dba)₃ (0.11 g, 0.12 mmol), and 0.1 mL of P(t-Bu)₃ were dissolved in 30 mL of toluene in a 250 mL round-bottom flask, and refluxed at a temperature of 80 °C for 6 hours. After the reaction was completed, the solvent was concentrated after cooling to room temperature, and separated through column chromatography to obtain Compound 4 (0.8 g, 43%).

### Synthesis Example 3: Synthesis of Compound 8

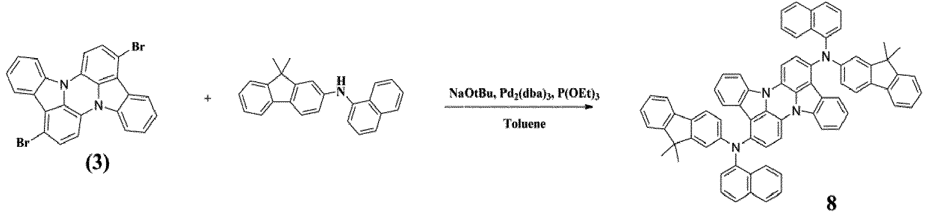

Intermediate compound (3) (1 g, 2.44 mmol), 9,9-dimethyl-N-(naphthalen-1-yl)-9H-fluoren-2-amine (3.3 g, 9.76 mmol), NaOtBu (0.47 g, 4.88 mmol), Pd₂(dba)₃ (0.11 g, 0.12 mmol), and 0.1 mL of P(t-Bu)₃ were dissolved in 30 mL of toluene in a 250 mL round-bottom flask, and refluxed at a temperature of 80 °C for 6 hours. After the reaction was completed, the solvent was concentrated after cooling to room temperature, and separated through column chromatography to obtain Compound 8 (0.9 g, 44%).

### Synthesis Example 4: Synthesis of Compound 35

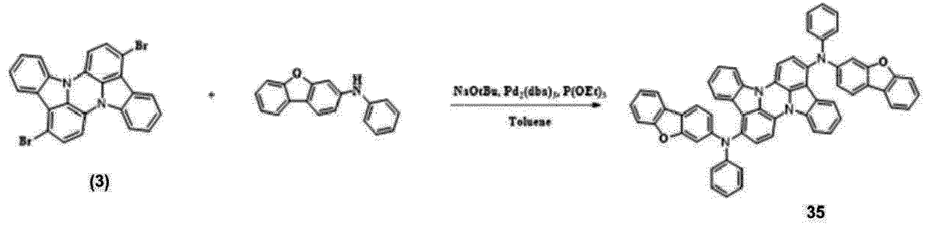

Intermediate compound (3) (1 g, 2.44 mmol), N-phenyldibenzo[b,d]furan-3-amine (2.53 g, 9.76 mmol), NaOtBu (0.47 g, 4.88 mmol), Pd₂(dba)₃ (0.11 g, 0.12 mmol), and 0.1 mL of P(t-Bu)₃ were dissolved in 30 mL of toluene, in a 250 mL round-bottom flask, and refluxed at a temperature of 80 °C for 6 hours. After the reaction was completed, the solvent was concentrated after cooling to room temperature, and separated through column chromatography to obtain Compound 35 (0.85 g, 49%).

### Synthesis Example 5: Synthesis of Compound 37

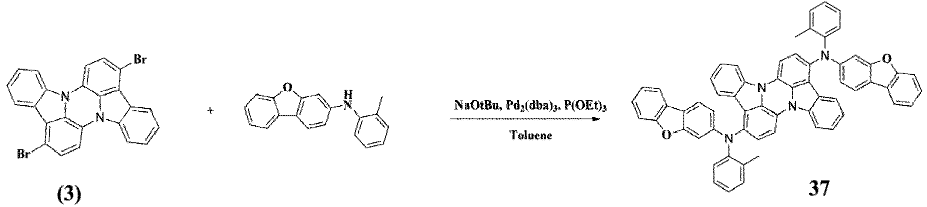

Intermediate compound (3) (1 g, 2.44 mmol), N-(o-tolyl)dibenzo[b,d]furan-3-amine (2.66 g, 9.76 mmol), NaOtBu (0.47 g, 4.88 mmol), Pd₂(dba)₃ (0.11 g, 0.12 mmol), and 0.1 mL of P(t-Bu)₃ were dissolved in 30 mL of toluene, in a 250 mL round-bottom flask, and refluxed at a temperature of 80 °C for 6 hours. After the reaction was completed, the solvent was concentrated after cooling to room temperature, and separated through column chromatography to obtain Compound 37 (0.8 g, 44%).

### Synthesis Example 6: Synthesis of compound 38

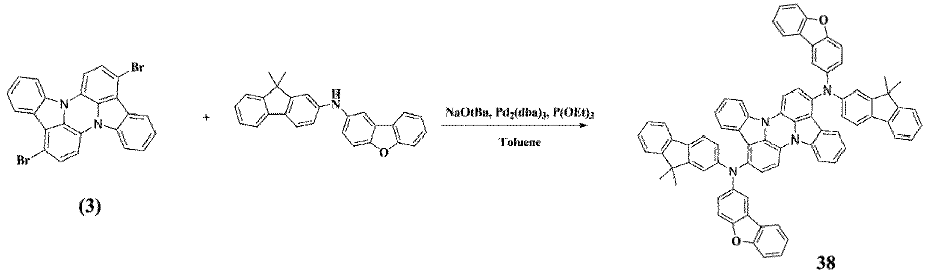

Intermediate compound (3) (1 g, 2.44 mmol), N-(9,9-dimethyl-9H-fluoren-2-yl)dibenzo[b,d]furan-2-amine (3.66 g, 9.76 mmol), NaOtBu (0.47 g, 4.88 mmol), Pd₂(dba)₃ (0.11 g, 0.12 mmol), and 0.1 mL of P(t-Bu)₃ were dissolved in 30 mL of toluene in a 250 mL round-bottom flask, and refluxed at a temperature of 80 °C for 6 hours. After the reaction was completed, the solvent was concentrated after cooling to room temperature, and separated through column chromatography to obtain Compound 38 (1.1 g, 49%).

¹H NMR of the compounds synthesized in Synthesis Examples 1 to 6 are shown in Table 1. Synthesis methods for other compounds than the compounds shown in Table 1 may be readily recognized by those skilled in the technical field by referring to the synthesis paths and source material materials described above.

**[Table 1]**

| Compound | ¹H NMR (δ) |
|---|---|
| 1 | δ 8.45-8.42 (m, 2H), 8.12-8.10 (m, 2H), 8.02-7.98 (m, 4H), 7.63-7.52 (m, 9H), 7.45-7.34 (m, 6H), 7.27-7.18 (m, 3H), 6.88-6.85 (d, 2H), 1.61 (s, 12H). |
| 4 | δ 7.69 (m, 2H), 7.36-7.50 (m, 4H), 7.05-7.08 (m, 12H), 6.78-6.88 (m, 6H), 6.70-6.72 (m, 6H), 6.50-6.58 (m, 6H), 1.19 (s, 4H) |
| 8 | δ 6.56-7.69 (m, 2H), 7.65-7.69 (m, 6H), 7.31-7.40 (m, 6H), 7.05-7.13 (m, 12H), 6.96-7.00 (m, 10H), 6.78-6.88 (m, 6H), 6.66-6.70 (m, 4H), 1.19 (s, 4H) |
| 35 | δ 7.69 (m, 2H), 7.48-7.53 (m, 4H), 7.30 (d, 2H), 6.89-7.01 (m,12H), 6.81 (m, 2H), 6.70-6.72 (m, 6H), 6.58-6.50 (m, 6H) |
| 37 | δ 8.50(m, 2H), 7.48-7.53(m, 4H), 7.50 (d, 2H), 6.81-6.96 (m, 14H), 6.63-6.70 (m, 8H), 6.41 (m, 4H), 1.62(s, 6H) |
| 38 | δ 7.69-7.72 (m, 4H), 7.48(m, 2H), 7.36-7.40(m 4H), 7.36-7.40 (m, 4H), 7.16 (m, 2H), 6.96-7.06 (m, 12H), 6.81-6.89 (m, 8H), 6.66-6.78 (m, 6H), 6.47 (m, 2H), 1.19 (s, 4H) |

### Example 1

A glass substrate on which a Corning 15 Ω/cm² (1,200 Å) ITO anode was disposed, was cut to a size of 50 mm x 50 mm x 0.7 mm, sonicated with isopropyl alcohol and pure water each for 15 minutes, and cleaned by exposure to ultraviolet rays and ozone for 30 minutes. The glass substrate was loaded onto a vacuum deposition apparatus.

m-MTDATA was vacuum-deposited on the ITO anode formed on the glass substrate to form a hole injection layer having a thickness of 110 Å, and NPB was vacuum-deposited on the hole injection layer to form a hole transport layer having a thickness of 10 Å.

Compound H125 as a host and Compound 1 as a dopant were co-deposited on the hole transport layer at a weight ratio of 97:3 to form an emission layer having a thickness of 20 nm.

BAIq was deposited on the emission layer to form an electron transport layer having a thickness of 10 nm, and Alq₃ was deposited on the electron transport layer to form a buffer layer having a thickness of 30 nm thickness, and LiF was deposited on the buffer layer to form an electron injection layer having a thickness of 1 nm, and Al was vacuum-deposited on the electron injection layer to form a cathode having a thickness of 200 nm, thereby completing the manufacture of the light-emitting device.

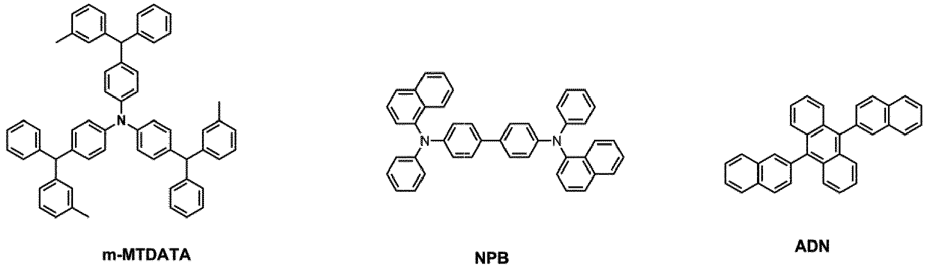

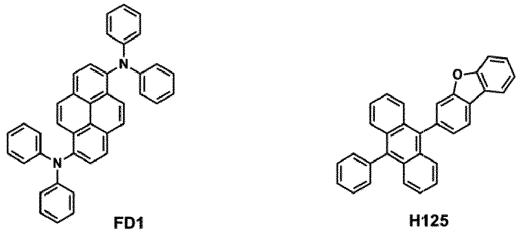

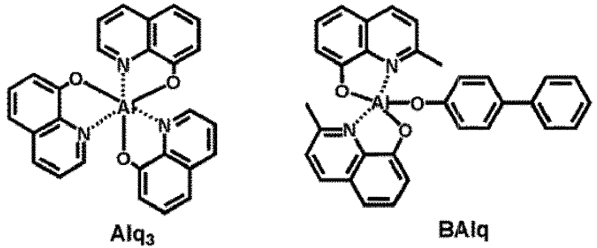

### Examples 2 to 7 and Comparative Examples 1 to 5

Light-emitting devices were manufactured in the same manner as in Example 1, except that, in forming an emission layer, corresponding host compounds shown in Table 2 were used instead of compound H125 as a host and Compound 1 as a dopant.

### Evaluation Example 1

To evaluate characteristics of the light-emitting devices manufactured according to Examples 1 to 7 and Comparative Examples 1 to 5, the driving voltage (V) at the current density of 15 mA/cm² and the luminance of 1,000 cd/m², color purity (CIEx,y), and luminescence efficiency (cd/A) of each of the light-emitting device were measured using a Keithley MU 236 and a luminance meter PR650. In evaluating the luminescence efficiency, the luminance/current density was measured using a luminance meter that has been calibrated for wavelength sensitivity, and the maximum external quantum conversion was conducted assuming an angular luminance distribution (Lambertian) which introduced a perfect reflecting diffuser. Table 2 show the evaluation results of the characteristics of the light-emitting devices.

**[Table 2]**

| | Emission layer | | Driving voltage (V) | Luminescence efficiency (cd/A) | Color purity (CIEx,y) | |
|---|---|---|---|---|---|---|
| | Host | Dopant | | | X | y |
| Example 1 | H125 | Compound 1 | 3.36 | 9.4 | 0.141 | 0.044 |
| Example 2 | ADN | Compound 1 | 3.76 | 10.0 | 0.140 | 0.047 |
| Example 3 | ADN | Compound 4 | 3.76 | 9.9 | 0.141 | 0.044 |
| Example 4 | ADN | Compound 8 | 3.76 | 9.7 | 0.142 | 0.048 |
| Example 5 | ADN | Compound 35 | 3.75 | 10.1 | 0.142 | 0.043 |
| Example 6 | ADN | Compound 37 | 3.75 | 9.8 | 0.140 | 0.047 |
| Example 7 | ADN | Compound 38 | 3.77 | 9.8 | 0.141 | 0.046 |
| Comparative Example 1 | H125 | FD1 | 3.36 | 7.9 | 0.142 | 0.043 |
| Comparative Example 2 | ADN | FD1 | 3.75 | 9.0 | 0.142 | 0.048 |
| Comparative Example 3 | ADN | Compound A | 3.75 | 5.0 | 0.136 | 0.053 |
| Comparative Example 4 | ADN | Compound B | 3.74 | 2.5 | 0.137 | 0.053 |
| Comparative Example 5 | ADN | Compound C | 3.76 | 2.2 | 0.136 | 0.0053 |

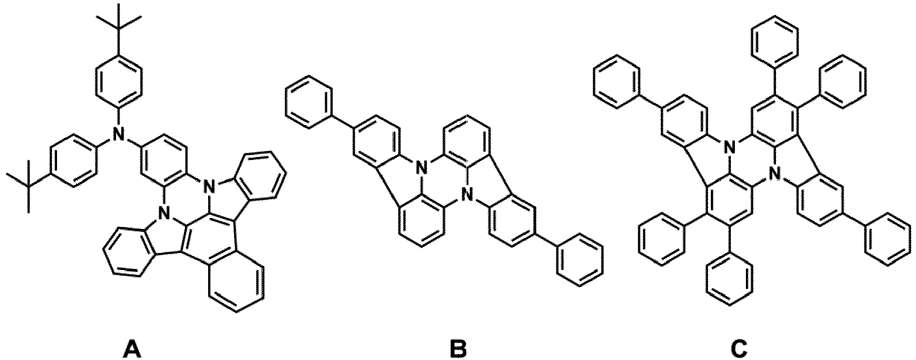

From Table 2, it can be seen that the light-emitting devices of Examples 1 to 7 have lower or equivalent level of driving voltage and higher luminescence efficiency and color purity compared to Comparative Examples 1 to 5.

Embodiments have been disclosed herein, and although terms are employed, they are used and are to be interpreted in a generic and descriptive sense only and not for purpose of limitation. In some instances, as would be apparent by one of ordinary skill in the art, features, characteristics, and/or elements described in connection with an embodiment may be used singly or in combination with features, characteristics, and/or elements described in connection with other embodiments unless otherwise specifically indicated. Accordingly, it will be understood by those of ordinary skill in the art that various changes in form and details may be made without departing from the scope of the disclosure as set forth in the claims.

## Claims

1. A heterocyclic compound represented by Formula 1: 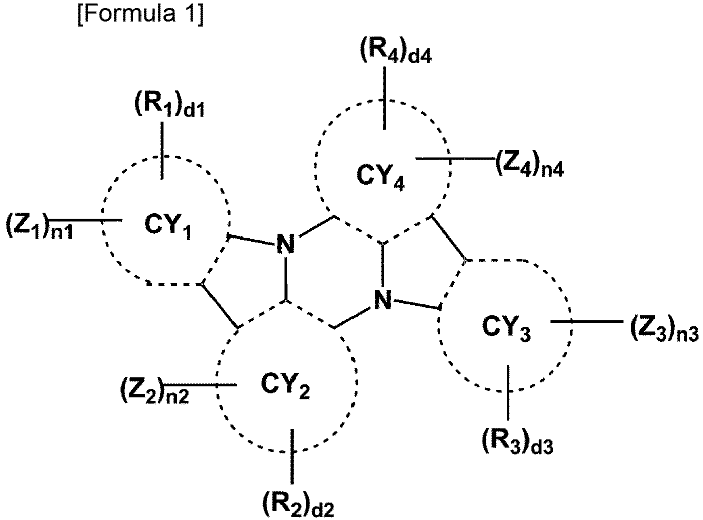
wherein in Formula 1,
CY₁ to CY₄ are each independently a C₃-C₆₀ carbocyclic group or a C₁-C₆₀ heterocyclic group,
Z₁ is *-(L₁)ₐ₁-E₁,
Z₂ is *-(L₂)ₐ₂-E₂,
Z₃ is *-(L₃)ₐ₃-E₃,
Z₄ is *-(L₄)ₐ₄-E₄,
L₁ to L₄ are each independently a single bond, a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
a1 to a4 are each independently an integer from 1 to 3,
E₁ to E₄ are each independently (i) a C₃-C₆₀ carbocyclic group unsubstituted or substituted with at least one R₁₀ₐ or (ii) a group containing at least one heteroatom,
n1 to n4 are each independently an integer from 0 to 3,
the sum of n1 to n4 is 1 or greater,
when E₁ to E₄ are each a benzene group, the sum of n2 and n4 is 1 or 2,
* indicates a binding site to a neighboring atom,
R₁ to R₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂),
d1 to d4 are each independently an integer from 0 to 10,
two or more groups of R₁ to R₄ are optionally bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ,
each R₁₀ₐ is independently:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, or a C₂-C₆₀ heteroaryl alkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ aryl alkyl group, a C₂-C₆₀ heteroaryl alkyl group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂),
wherein Q₁ to Q₃, Q₁₁ to Q₁₃, Q₂₁ to Q₂₃ and Q₃₁ to Q₃₃ are each independently:
hydrogen, deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof; or
a C₇-C₆₀ aryl alkyl group, or a C₂-C₆₀ heteroaryl alkyl group.

2. The heterocyclic compound of claim 1, wherein
CY₁ to CY₄ are each independently a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzotriazole group a benzoxazole group, a benzothiazole group, a benzoxadiazole group, a benzothiadiazole group, a 5,6,7,8-tetrahydroisoquinoline group, or a 5,6,7,8-tetrahydroquinoline group.

3. The heterocyclic compound of claim 1 or claim 2, wherein
L₁ to L₄ are each independently:
a single bond; or
a group represented by one of Formulae 2-1 to 2- 40: 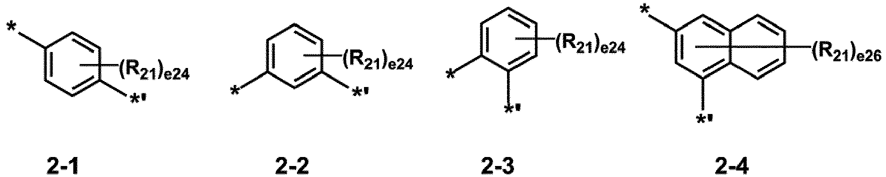 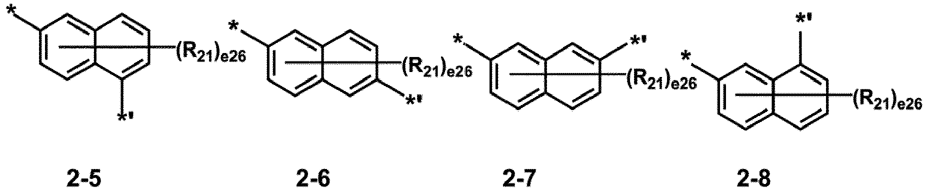 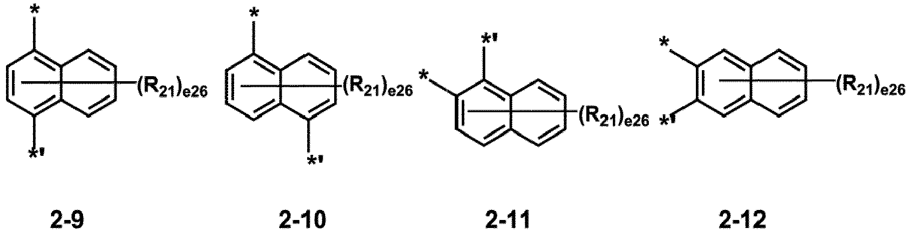 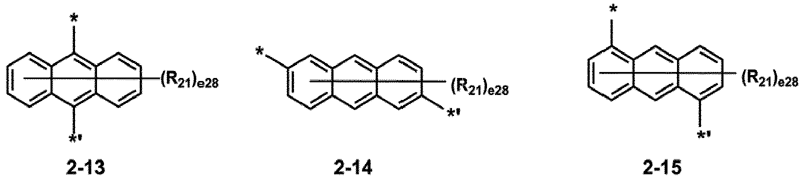 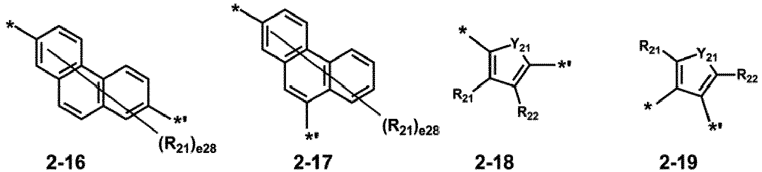 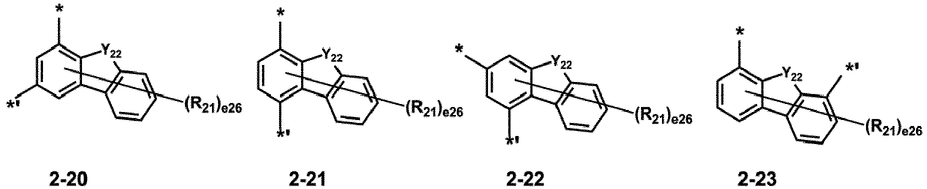 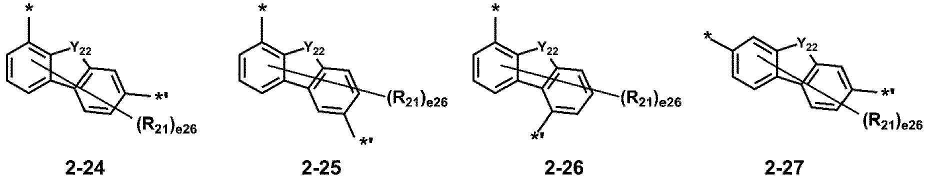 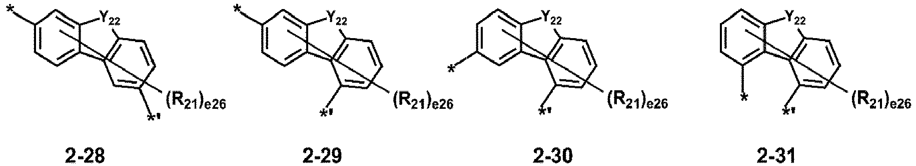 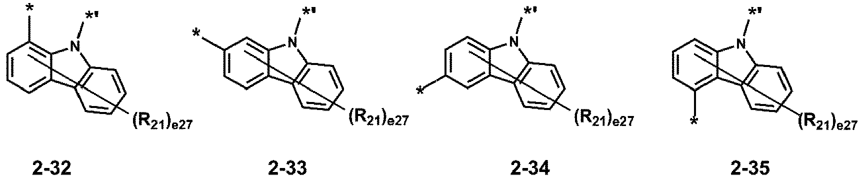 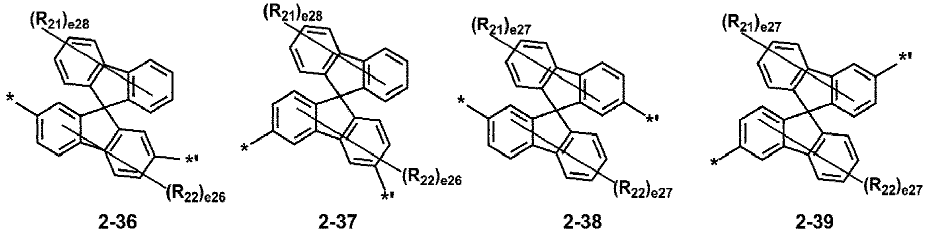 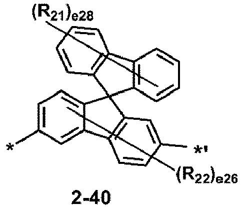
wherein in Formulae 2-1 to 2-40 ,
Y₂₁ is O or S,
Y₂₂ is O, S, N(R₂₃), C(R₂₃)(R₂₄), or Si(R₂₃)(R₂₄),
R₂₁ to R₂₄ are each independently hydrogen, deuterium, -F, -Cl, -Br, -I, a hydroxyl group, a cyano group, a nitro group, an amidino group, a hydrazino group, a hydrazono group, -CF₃, -CF₂H, -CFH₂, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a naphthyl group, a fluorenyl group, a spiro-bifluorenyl group, a spiro-fluorene-benzofluorenyl group, a benzofluorenyl group, a dibenzofluorenyl group, a phenalenyl group, a phenanthrenyl group, an anthracenyl group, a fluoranthenyl group, a triphenylenyl group, a pyridinyl group, a pyrimidinyl group, a pyrazinyl group, a triazinyl group, a quinolinyl group, an isoquinolinyl group, a benzoquinolinyl group, a naphthyridinyl group, a quinoxalinyl group, a quinazolinyl group, a phenanthridinyl group, an acridinyl group, a phenanthrolinyl group, a phenazinyl group, a carbazolyl group, a dibenzofuranyl group, a dibenzothiophenyl group, a dibenzosilolyl group, -Si(Q₄₁)(Q₄₂)(Q₄₃), -N(Q₄₁)(Q₄₂), or -B(Q₄₁)(Q₄₂),
e24 is an integer from 0 to 4,
e26 is an integer from 0 to 6,
e27 is an integer from 0 to 7,
e28 is an integer from 0 to 8,
Q₄₁ to Q₄₃ are each independently a C₁-C₁₀ alkyl group, a C₁-C₁₀ alkoxy group, a phenyl group, a biphenyl group, a terphenyl group, or a naphthyl group,
* and *' each indicate a binding site to a neighboring atom.

4. The heterocyclic compound of any one of claims 1 to 3, wherein
the group containing at least one heteroatom is:
a C₁-C₆₀ heterocyclic group unsubstituted or substituted with at least one R₁₀ₐ; or
-Si(Ar₁)(Ar₂)(Ar₃), -N(Ar₁)(Ar₂), -B(Ar₁)(Ar₂), -C(=O)(Ar₁), -S(=O)₂(Ar₁), or -P(=O)(Ar₁)(Ar₂), and
Ar₁ to Ar₃ are each independently hydrogen, deuterium, -F, -Cl, -Br, -I, hydroxyl group, a cyano group, a nitro group, a C₁-C₆₀alkyl group, a C₂-C₆₀alkenyl group, a C₂-C₆₀ alkynyl group, a C₁-C₆₀ alkoxy group, a C₅-C₃₀ carbocyclic group that is unsubstituted or substituted with at least one R₁₀ₐ, or a C₁-C₃₀ heterocyclic group that is unsubstituted or substituted with at least one R₁₀ₐ, and
each R₁₀ₐ is independently the same as described in claim 1.

5. The heterocyclic compound of any one of claims 1 to 4, wherein
E₁ to E₄ are each independently:
a T1 group, a cyclic group in which two or more T1 groups are condensed with each other, a T2 group, a cyclic group in which two or more T2 groups are condensed with each other, or a cyclic group in which at least one T2 group is condensed with at least one T1 group, each unsubstituted or substituted with at least one R₁₀ₐ;
a group represented by one of Formulae 3-1 to 3-9; or
-Si(Ar₁)(Ar₂)(Ar₃), -N(Ar₁)(Ar₂), -B(Ar₁)(Ar₂), -C(=O)(Ar₁), -S(=O)₂(Ar₁), or -P(=O)(Ar₁)(Ar₂), wherein
the T1 group is a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane group, a norbornene group, a bicyclo[1.1.1]pentane group, a bicyclo[2.1.1]hexane group, a bicyclo[2.2.2]octane group, or a benzene group,
the T2 group is a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
Ar₁ to Ar₃ are each independently a C₃-C₆₀ carbocyclic group unsubstituted or substituted with at least one R₁₀ₐ, or a C₁-C₆₀ heterocyclic group unsubstituted or substituted with at least one R₁₀ₐ,
Formulae 3-1 to 3-9 are: 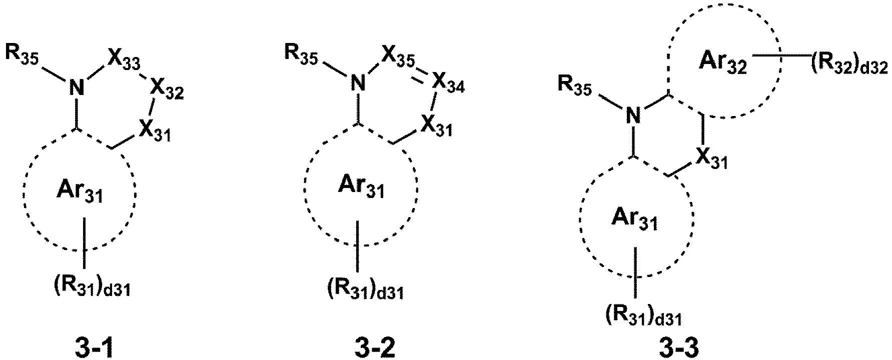 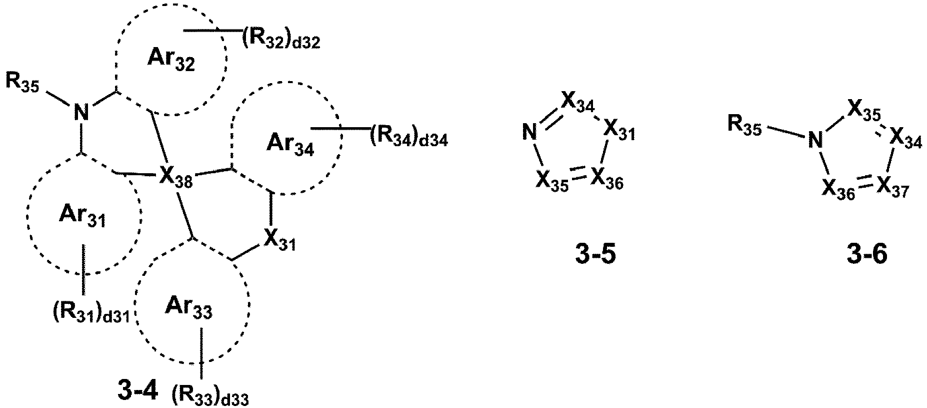 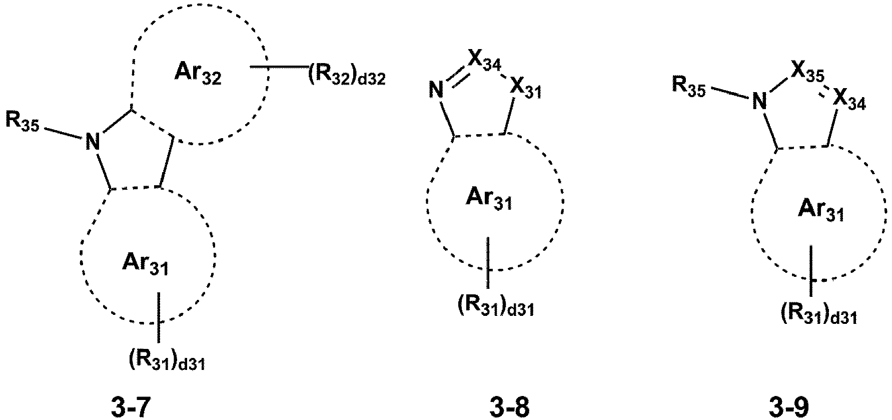
wherein in Formulae 3-1 to 3-9,
Ar₃₁ to Ar₃₄ are each independently a C₃-C₆₀ carbocyclic group or a C₁-C₆₀ heterocyclic group,
X₃₁ is N(R₃₁ₐ), C(R₃₁ₐ)(R_{31b}), Si(R₃₁ₐ)(R_{31b}), O, S, or Se,
X₃₂ is N(R₃₂ₐ), C(R₃₂ₐ)(R_{32b}), Si(R₃₂ₐ)(R_{32b}), O, S, or Se,
X₃₃ is N(R₃₃ₐ), C(R₃₃ₐ)(R_{33b}), Si(R₃₃ₐ)(R_{33b}), O, S, or Se,
X₃₄ is N or C(R₃₄ₐ),
X₃₅ is N or C(R₃₅ₐ),
X₃₆ is N or C(R₃₆ₐ),
X₃₇ is N or C(R₃₇ₐ),
X₃₈ is C or Si,
R₃₁, R₃₂, R₃₃, R₃₄, R₃₅, R₃₁ₐ, R_{31b}, R₃₂ₐ, R_{32b}, R₃₃ₐ, R_{33b}, R₃₄ₐ, R₃₅ₐ, R₃₆ₐ, and R₃₇ₐ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
d31 to d34 are each independently an integer from 0 to 10,
one of R₃₁(s) in the number of d31, R₃₂(s) in the number of d32, R₃₃(s) in the number of d33, R₃₄(s) in the number of d34, R₃₅, R₃₁ₐ, R_{31b}, R₃₂ₐ, R_{32b}, R₃₃ₐ, R_{33b}, and R₃₄ₐ to R₃₇ₐ indicates a binding site to a neighboring atom,
two or more neighboring groups of R₃₁, R₃₂, R₃₃, R₃₄, R₃₅, R₃₁ₐ, R_{31b}, R₃₂ₐ, R_{32b}, R₃₃ₐ, R_{33b}, R₃₄ₐ, R₃₅ₐ, R₃₆ₐ and R₃₇ₐ are optionally bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ, and
each R₁₀ₐ is independently the same as described in claim 1.

6. The heterocyclic compound of any one of claims 1 to 5, wherein
E₁ to E₄ are each independently:
*-N(Ar₁)(Ar₂) or *-P(=O)(Ar₁)(Ar₂); or
a group represented by one of Formulae 4-1 to 4-87,
Ar₁ and Ar₂ are each independently hydrogen, deuterium, -F, -Cl, -Br, -I, hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₅-C₃₀ carbocyclic group that is unsubstituted or substituted with at least one R₁₀ₐ, or a C₁-C₃₀ heterocyclic group that is unsubstituted or substituted with at least one R₁₀ₐ, and
each R₁₀ₐ is independently the same as described in claim 1;
Formulae 4-1 to 4-87 are: 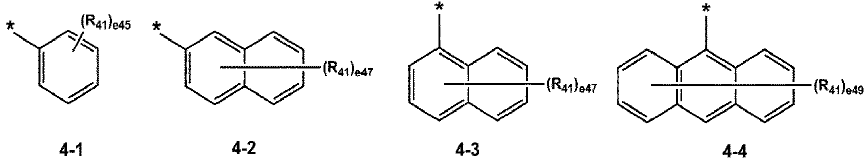 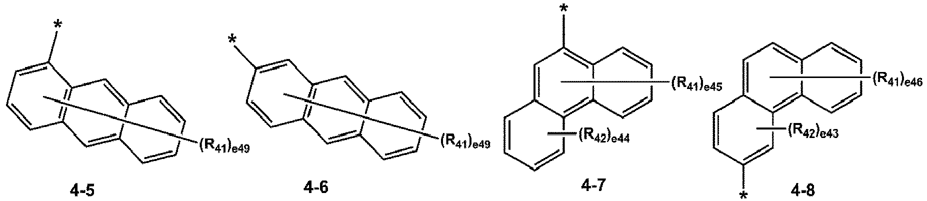 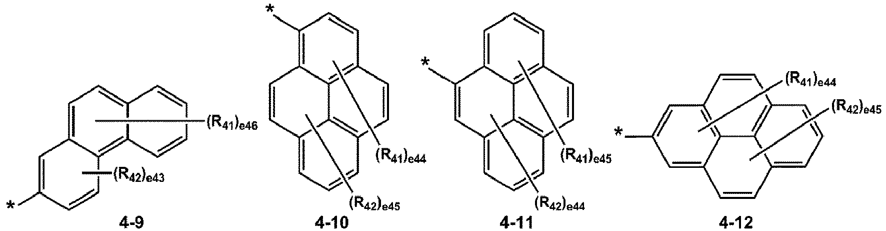 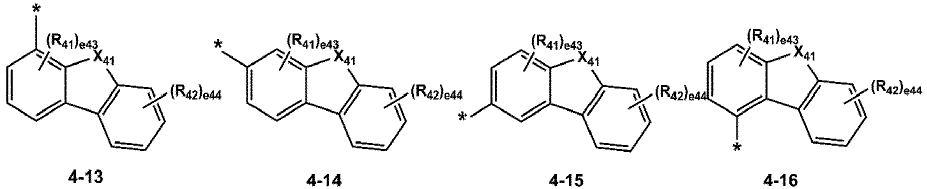 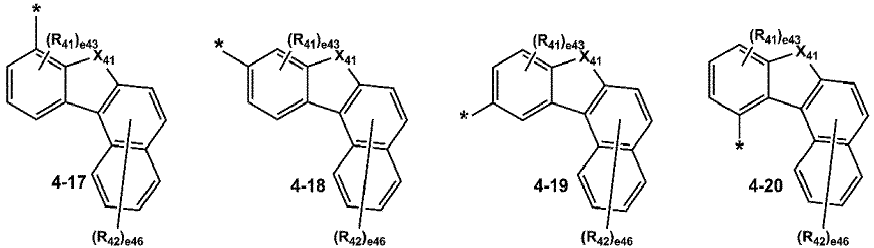 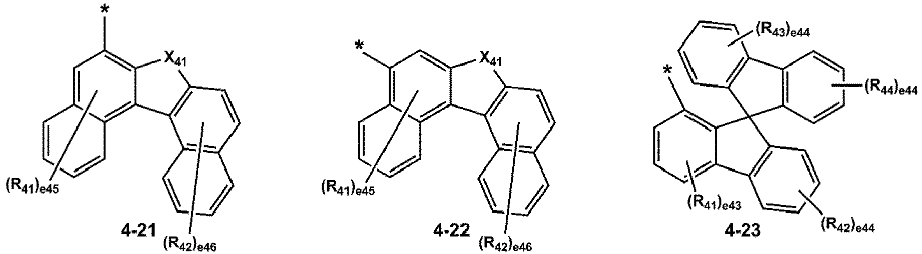 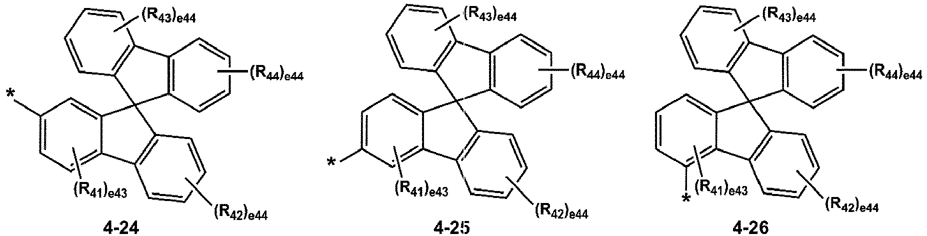 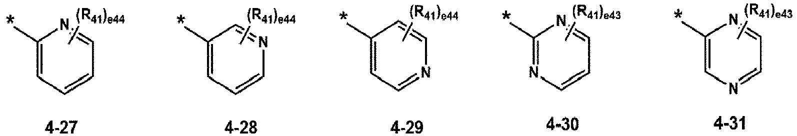 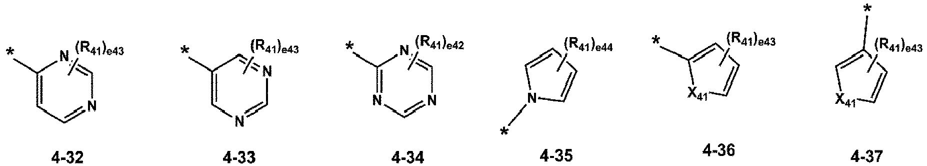 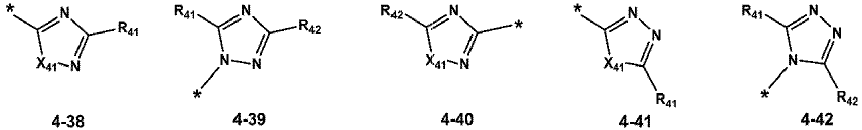 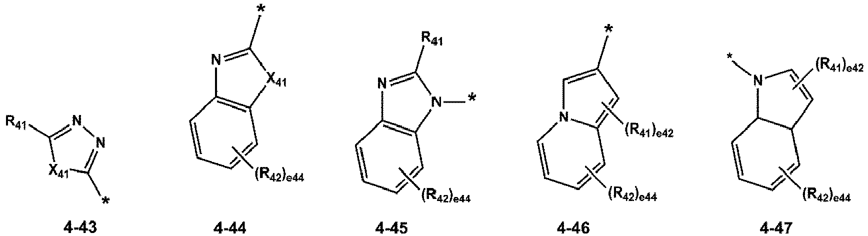 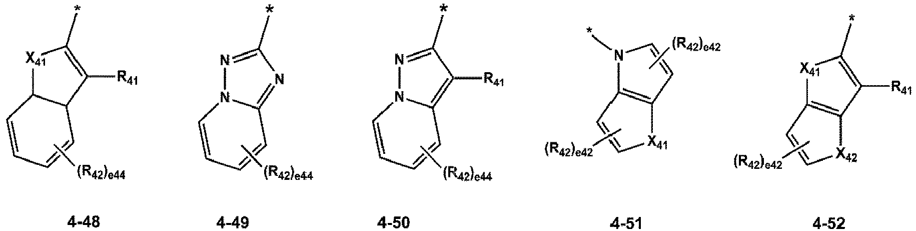 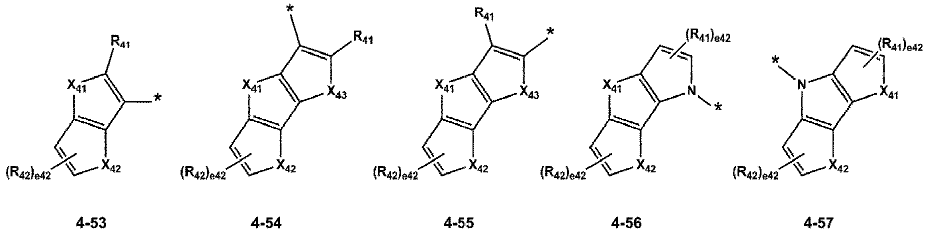 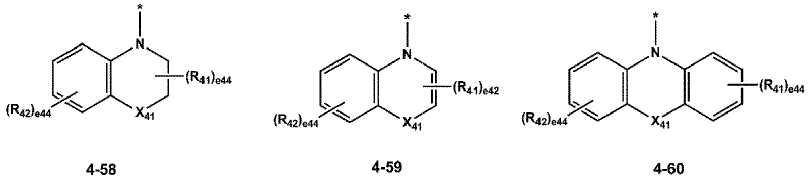 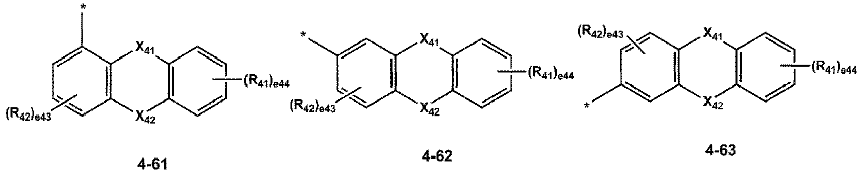 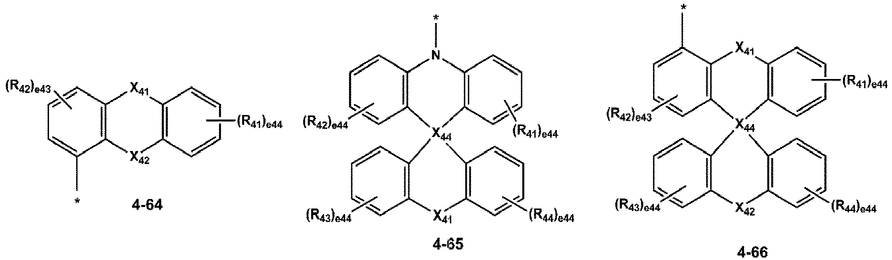 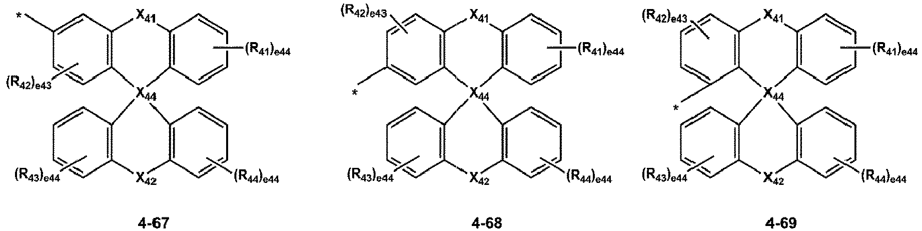 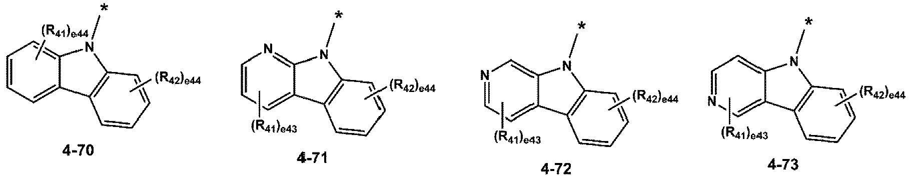 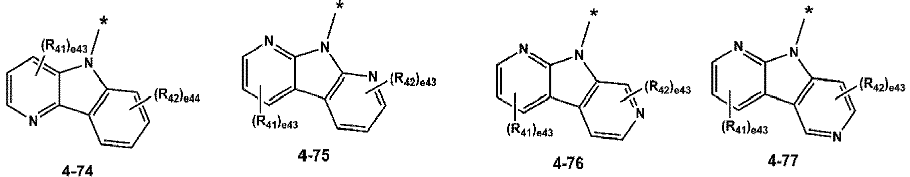 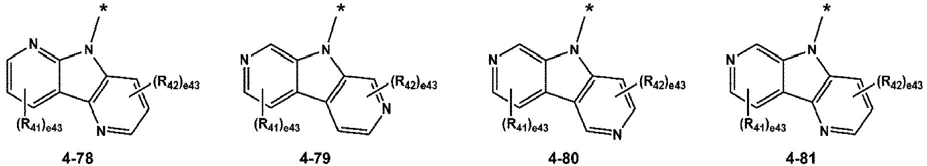 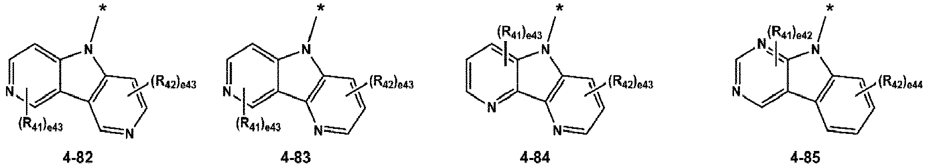 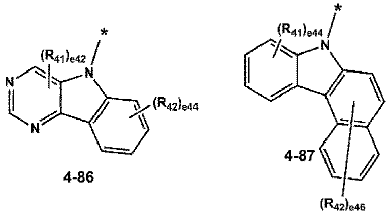
wherein in Formulae 4-1 to 4-87,
X₄₁ is O, S, Se, N(R₄₅), C(R₄₅)(R₄₆), or Si(R₄₅)(R₄₆),
X₄₂ is O, S, Se, N(R₄₇), C(R₄₇)(R₄₈), or Si(R₄₇)(R₄₈),
X₄₃ is O, S, Se, N(R₄₉), C(R₄₉)(R₅₀), or Si(R₄₉)(R₅₀),
X₄₄ is C or Si,
R₄₁ to R₅₀ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, a C₆-C₆₀ aryloxy group unsubstituted or substituted with at least one R₁₀ₐ, a C₆-C₆₀ arylthio group unsubstituted or substituted with at least one R₁₀ₐ, -Si(Q₁)(Q₂)(Q₃), -N(Q₁)(Q₂), - B(Q₁)(Q₂), -C(=O)(Q₁), -S(=O)₂(Q₁), or -P(=O)(Q₁)(Q₂),
two or more groups of R₄₁ to R₅₀ are optionally bound to each other to form (i) a C₅-C₃₀ carbocyclic group that is unsubstituted or substituted with at least one R₁₀ₐ or (ii) a C₂-C₃₀ heterocyclic group unsubstituted or substituted with at least one R₁₀ₐ,
e42 is 1 or 2,
e43 is an integer from 1 to 3,
e44 is an integer from 1 to 4,
e45 is an integer from 1 to 5,
e46 is an integer from 1 to 6,
e47 is an integer from 1 to 7,
e49 is an integer from 1 to 9, and
Q₁ to Q₃ and R₁₀ₐ are each independently the same as described in claim 1, and
* indicates a binding site to a neighboring atom.

7. The heterocyclic compound of any one of claims 1 to 6, wherein a moiety represented by 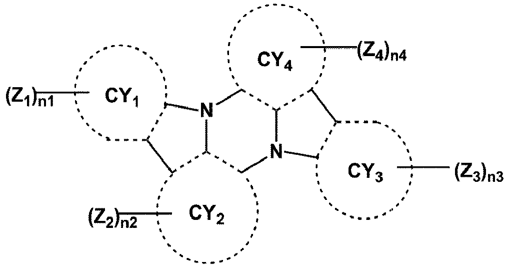 is a moiety represented by one of Formulae 1-1 to 1-27: 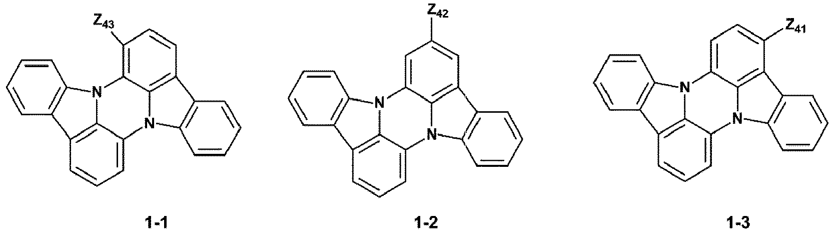 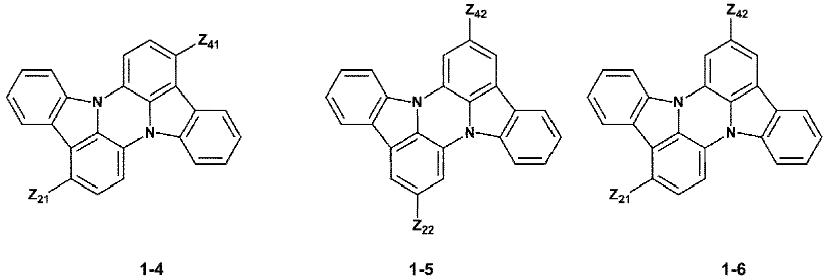 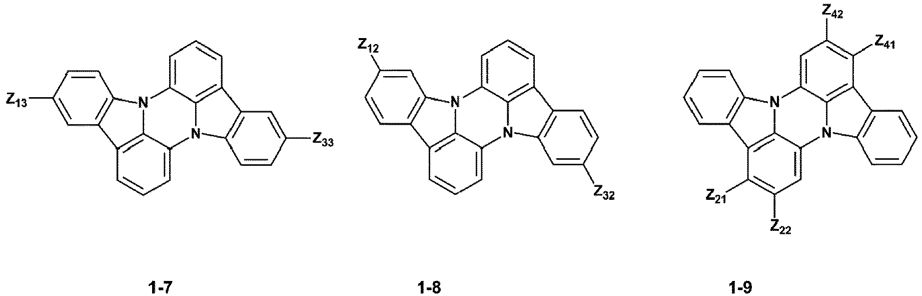 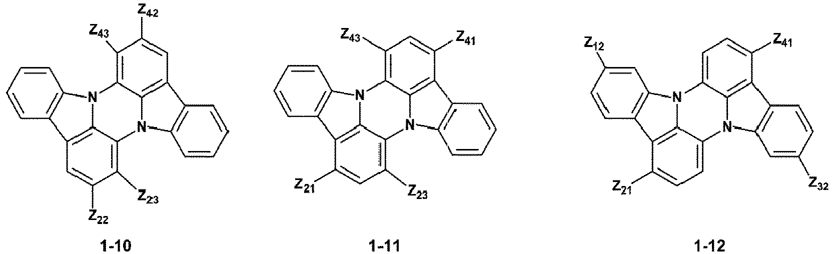 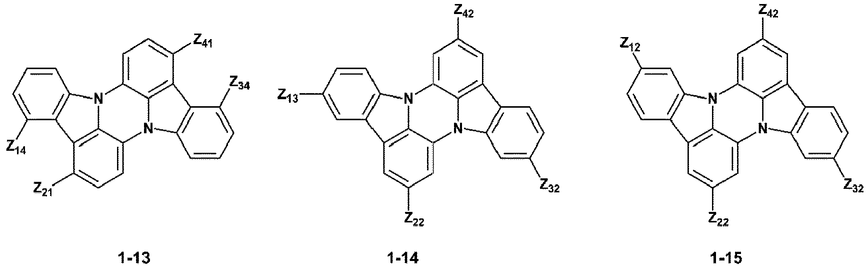 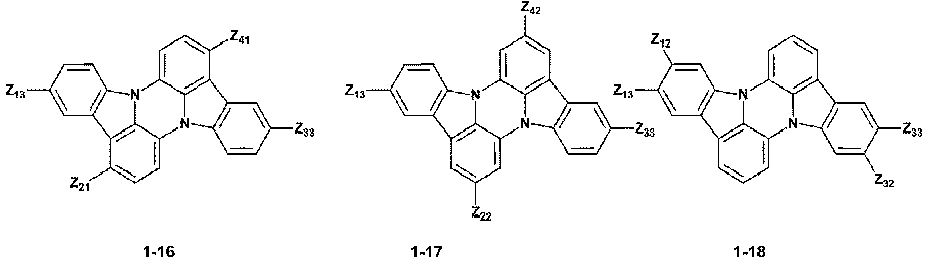 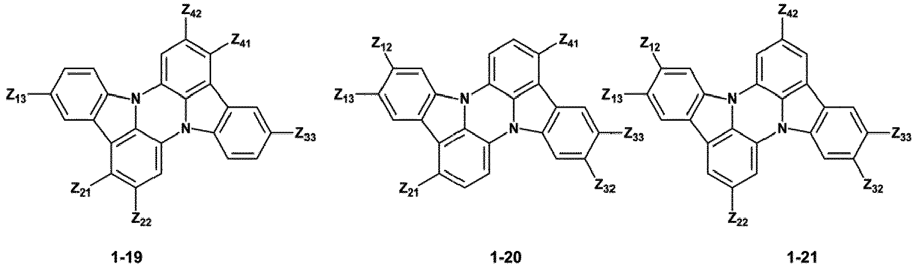 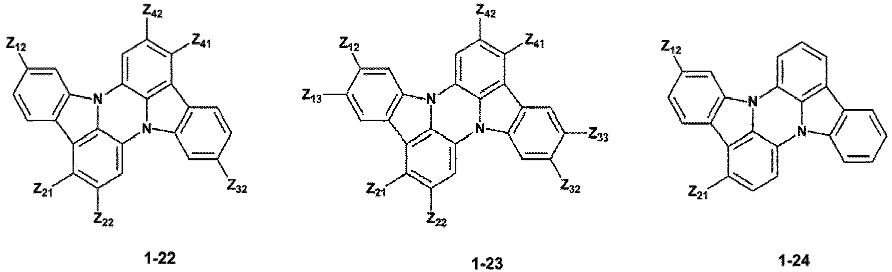 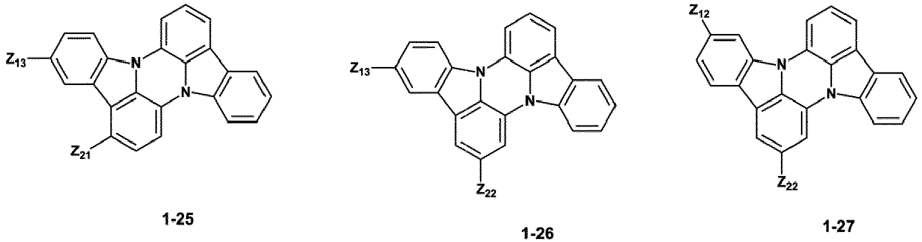
wherein in Formulae 1-1 to 1-27,
Z₁₂ to Z₁₄ are each independently the same as described in connection with Z₁ in any one of claims 1 to 6,
Z₂₁ to Z₂₃ are each independently the same as described in connection with Z₂ in any one of claims 1 to 6,
Z₃₂ to Z₃₄ are each independently the same as described in connection with Z₃ in any one of claims 1 to 6, and
Z₄₁ to Z₄₃ are each independently the same as described in connection with Z₄ in any one of claims 1 to 6.

8. The heterocyclic compound of any one of claims 1 to 7, wherein
the sum of n1 to n4 is an integer from 1 to 4.

9. The heterocyclic compound of any one of claims 1 to 8, wherein n1 to n4 satisfy at least one of Condition 1 and Condition 2:
[Condition 1]:
the sum of n2 and n4 is 1 or 2
[Condition 2]:
the sum of n1 and n3 is 0 or 2.

10. The heterocyclic compound of any one of claims 1 to 8, wherein
n1 is 0, n2 is 0, n3 is 0, and n4 is an integer of 1 or greater; or
n1 is 0, n2 is an integer of 1 or greater, n3 is 0, and n4 is 0; or
n1 is 0, n2 is an integer of 1 or greater, n3 is 0, and n4 is an integer of 1 or greater; or
n1 is an integer of 1 or greater, n2 is an integer of 1 or greater, n3 is an integer of 1 or greater, and n4 is an integer of 1 or greater; or
n1 is an integer of 1 or greater, n2 is 0, n3 is 0, and n4 is 0; or
n1 is 0, n2 is 0, n3 is an integer of 1 or greater, and n4 is 0; or
n1 is an integer of 1 or greater, n2 is 0, n3 is an integer of 1 or greater, and n4 is 0; or
n1 is an integer of 1 or greater, n2 is an integer of 1 or greater, n3 is 0, and n4 is 0; or
n1 is an integer of 1 or greater, n2 is an integer of 1 or greater, n3 is an integer of 1 or greater, and n4 is 0.

11. The heterocyclic compound of any one of claims 1 to 10, wherein
the heterocyclic compound is one of Compounds 1 to 111:

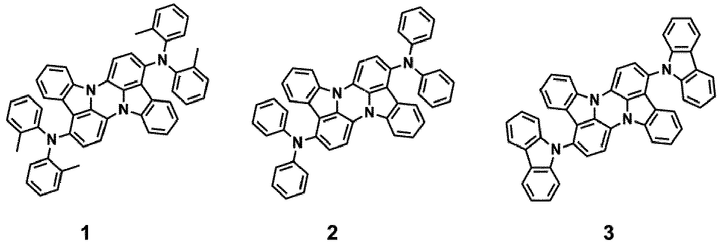

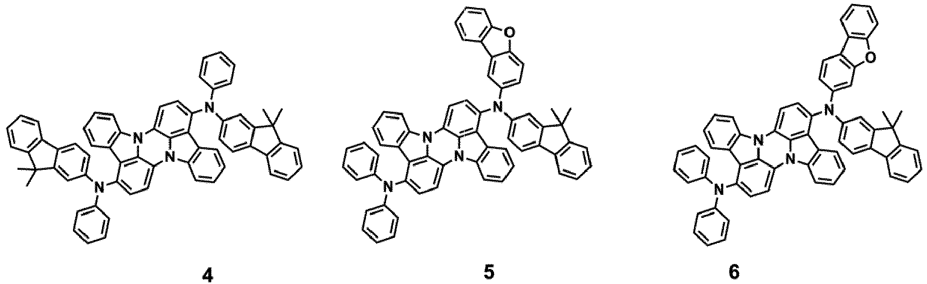

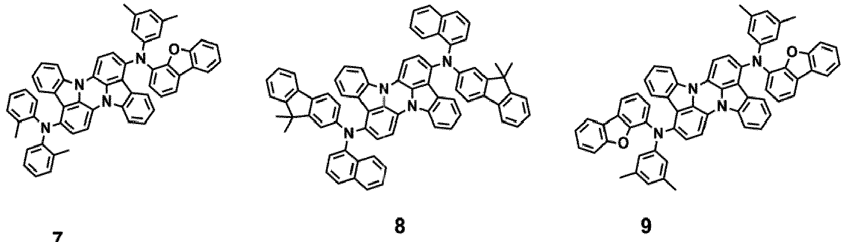

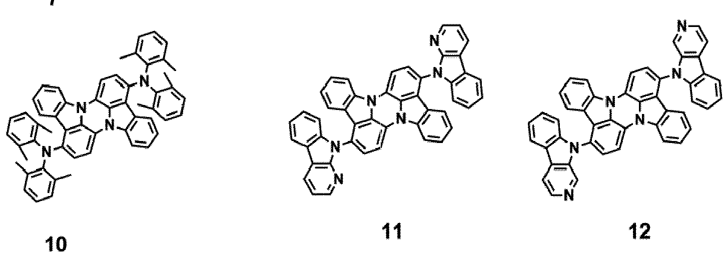

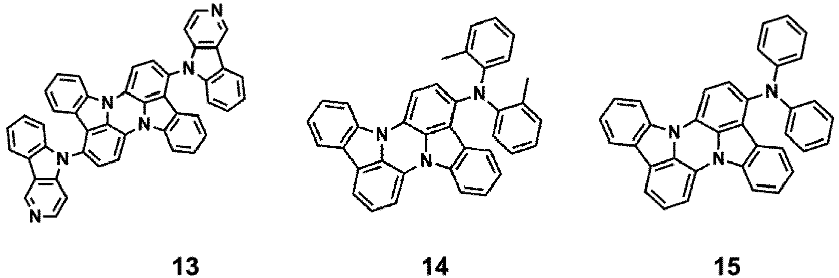

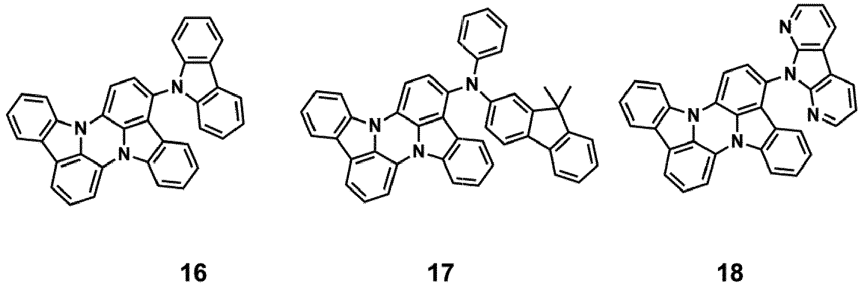

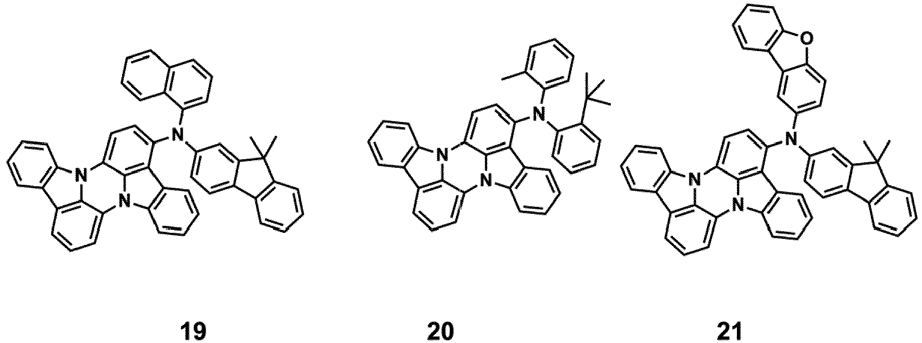

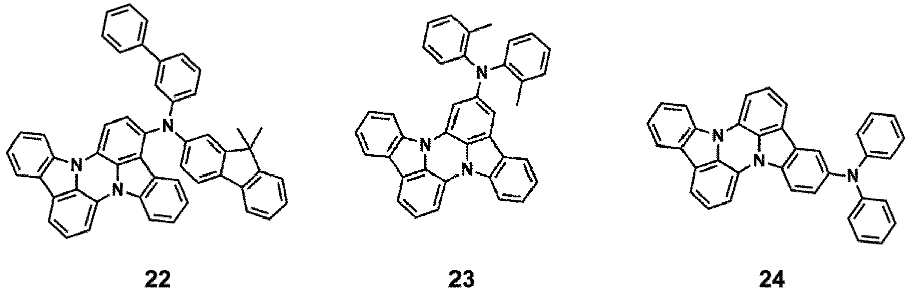

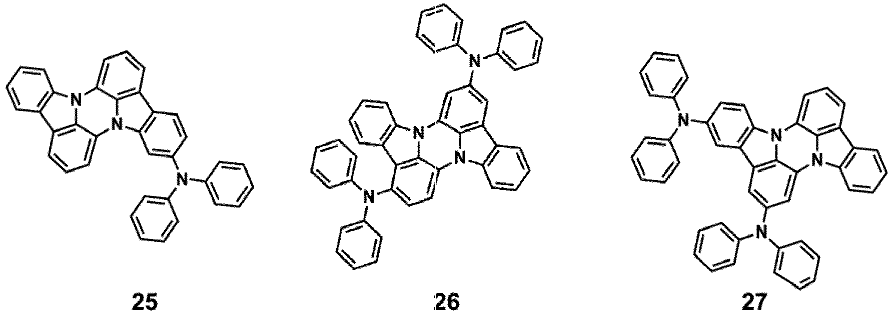

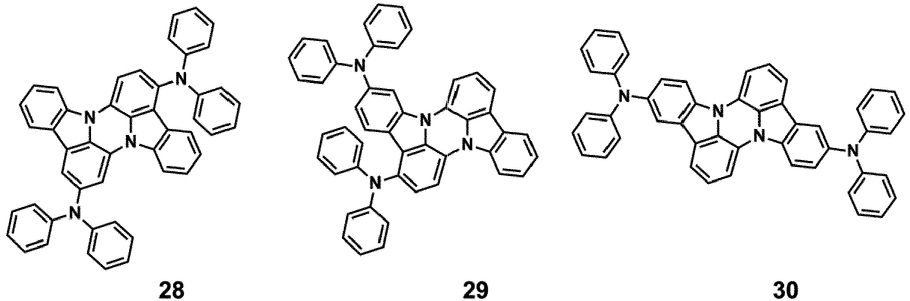

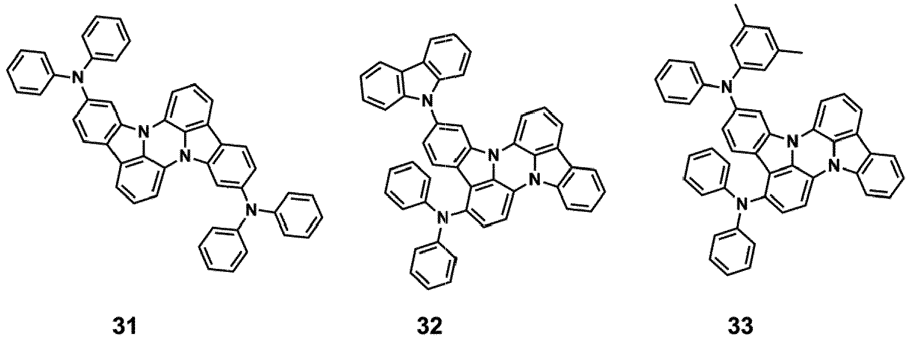

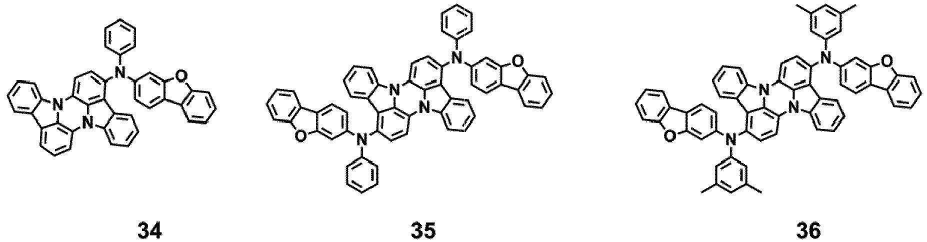

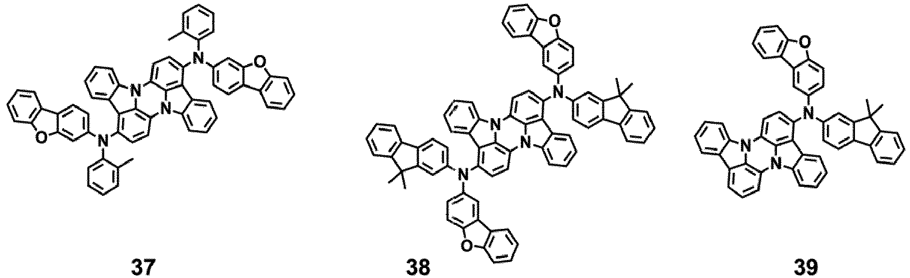

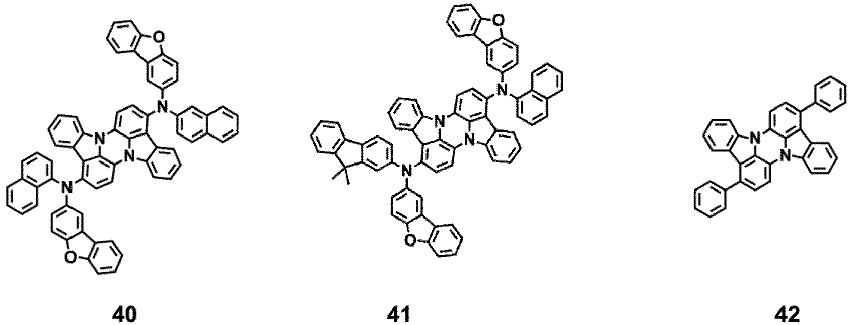

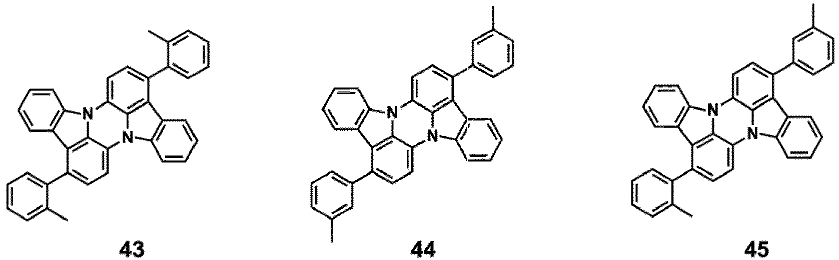

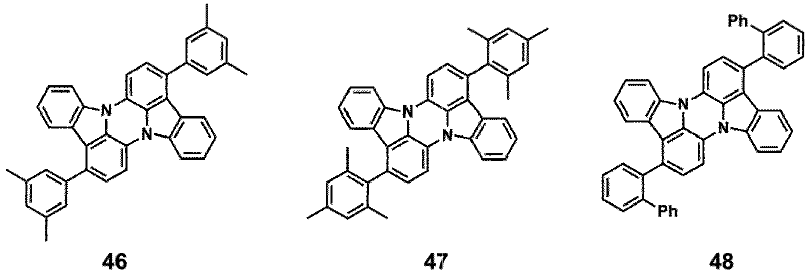

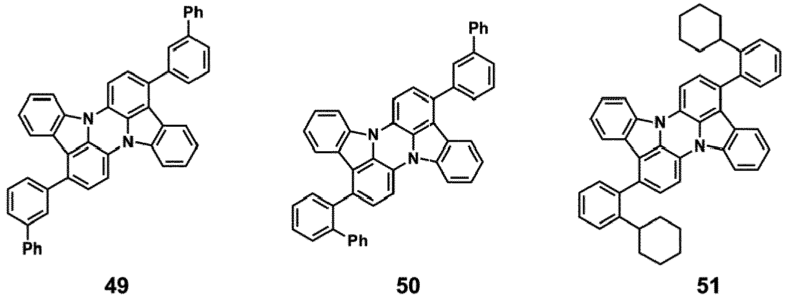

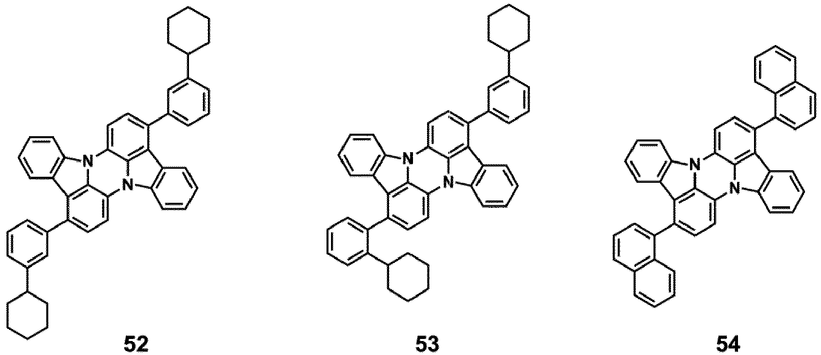

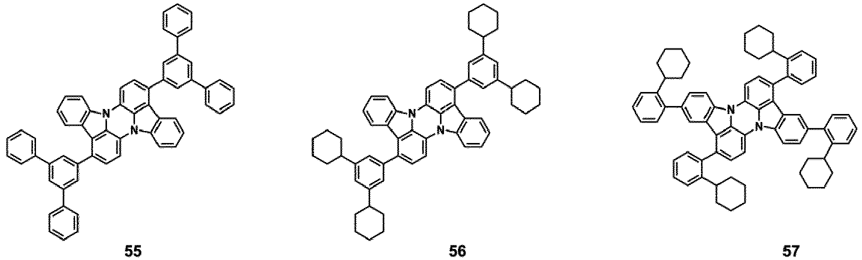

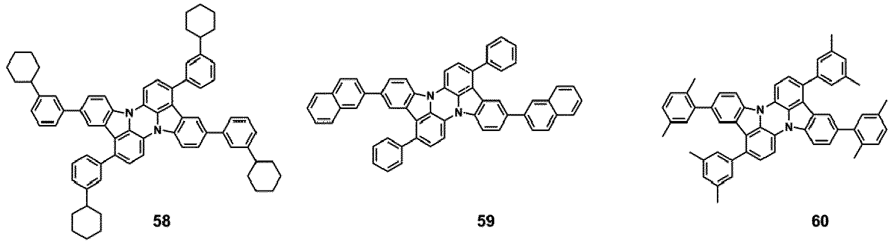

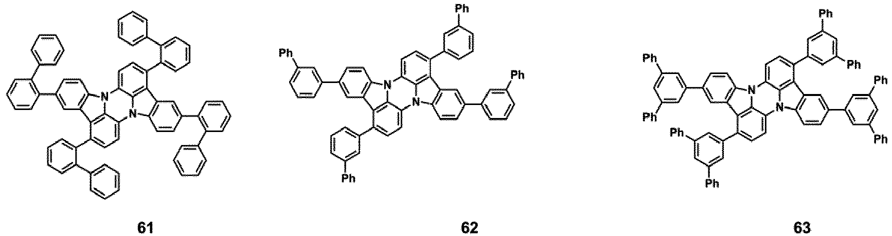

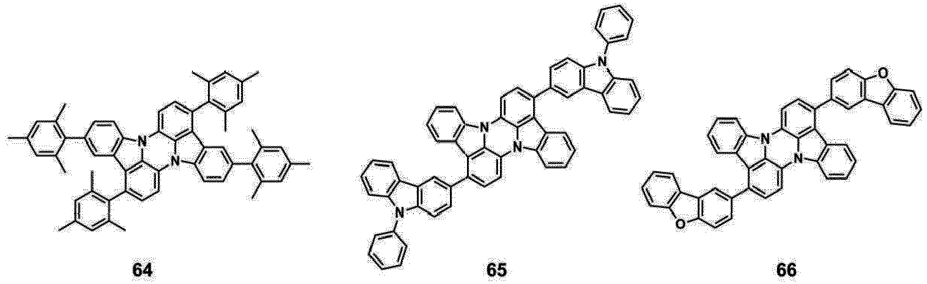

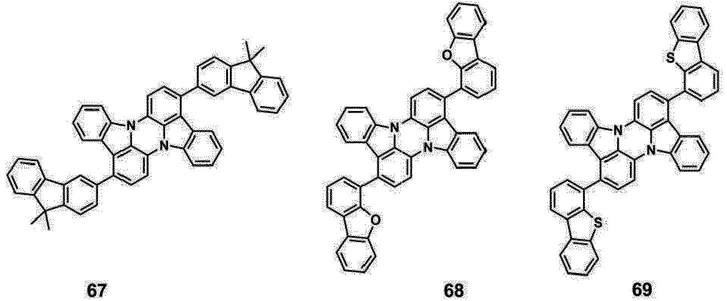

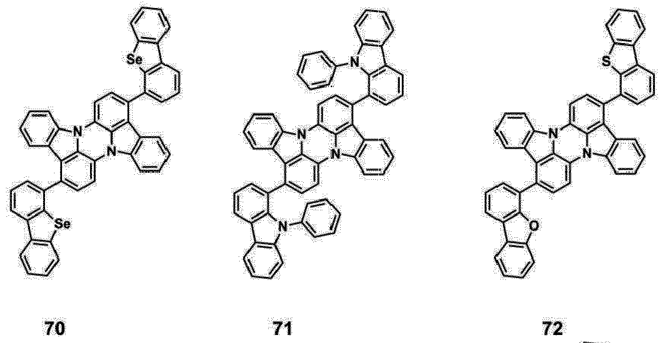

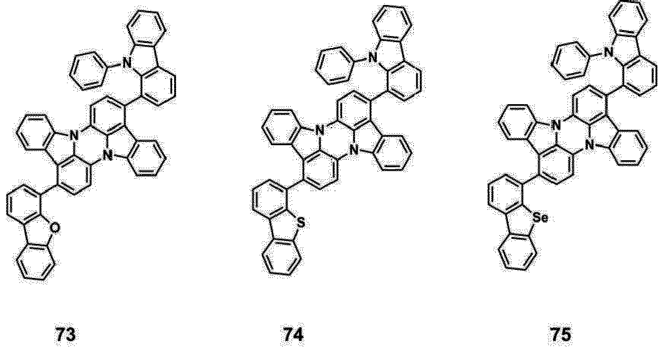

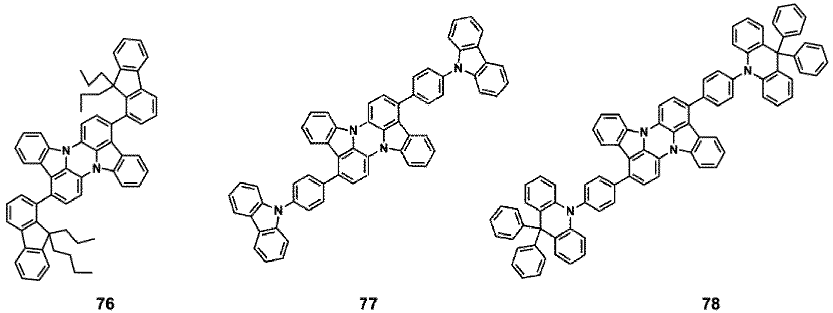

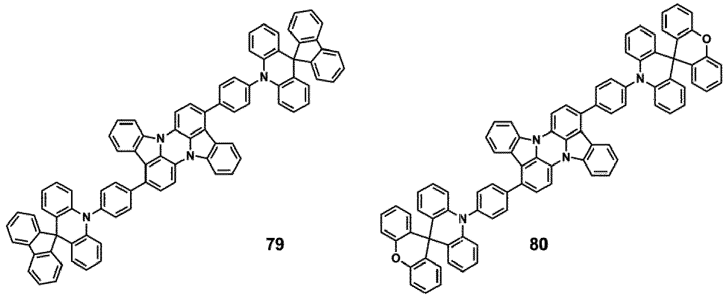

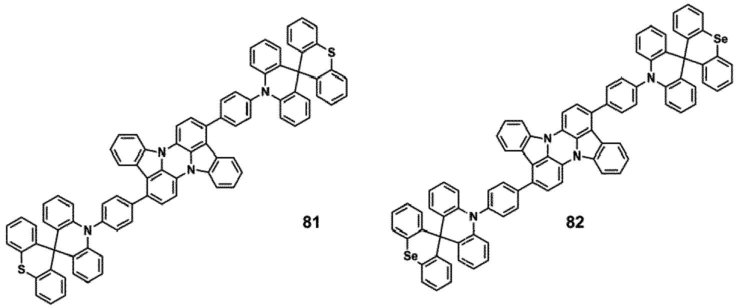

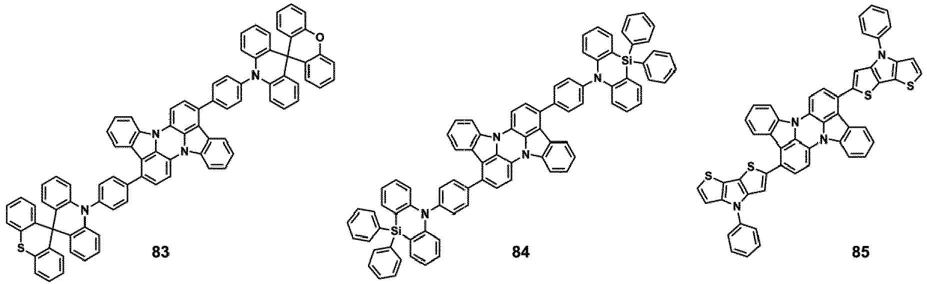

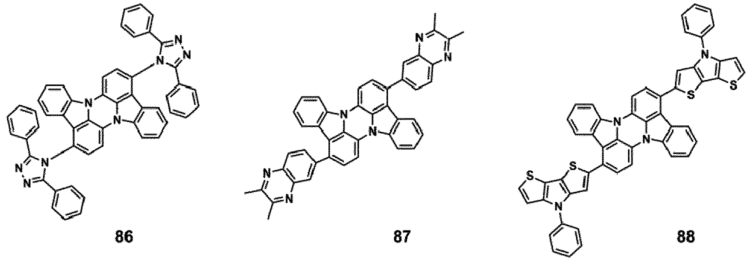

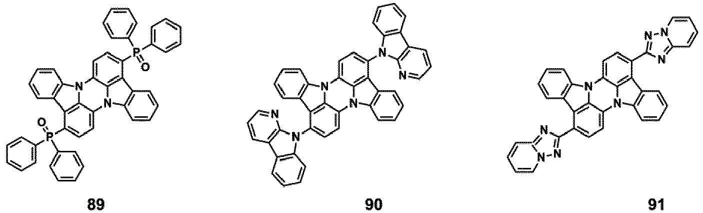

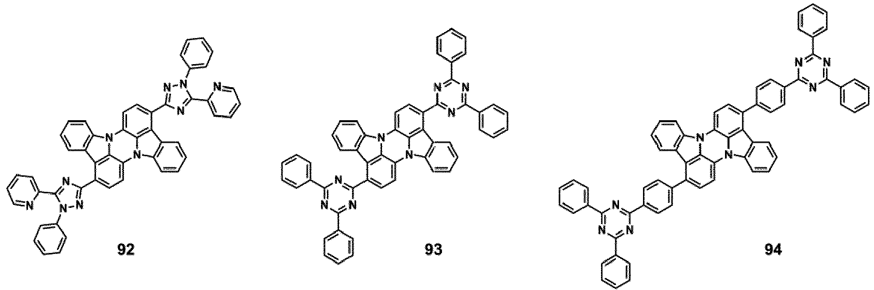

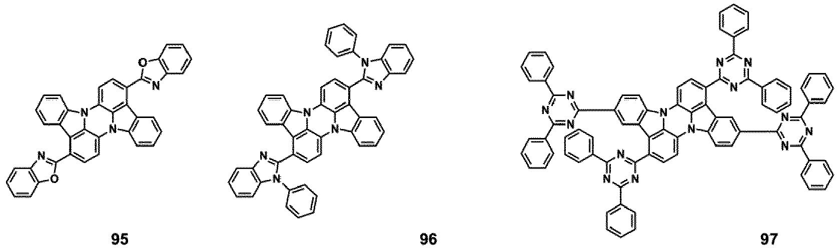

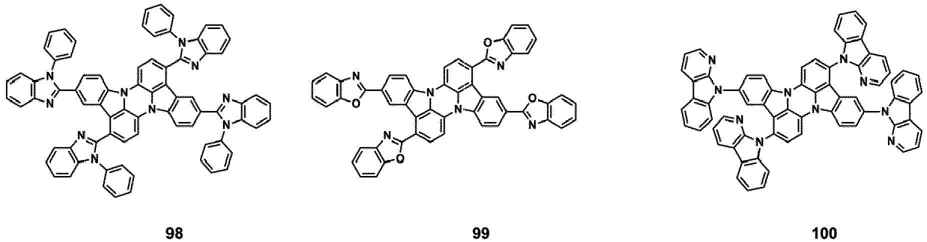

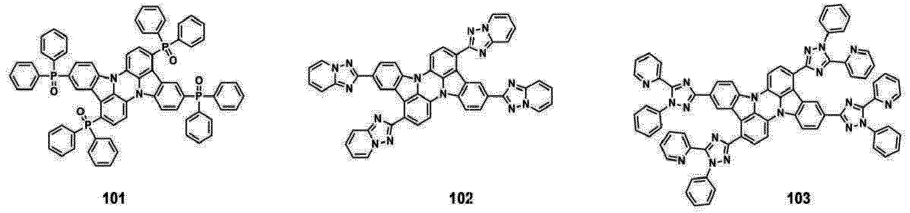

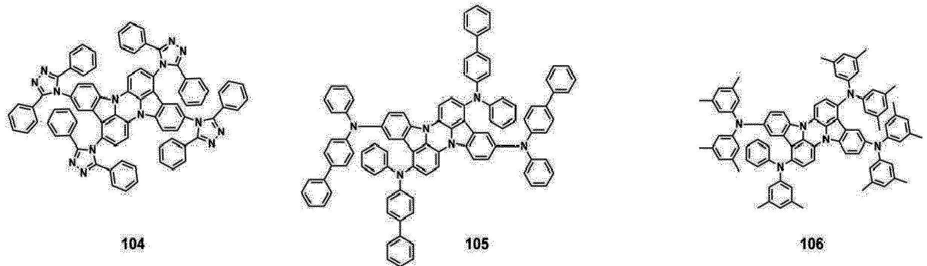

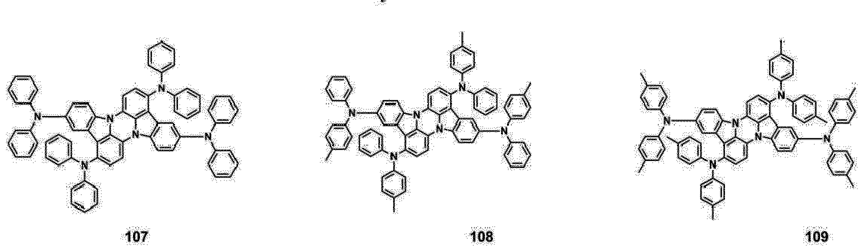

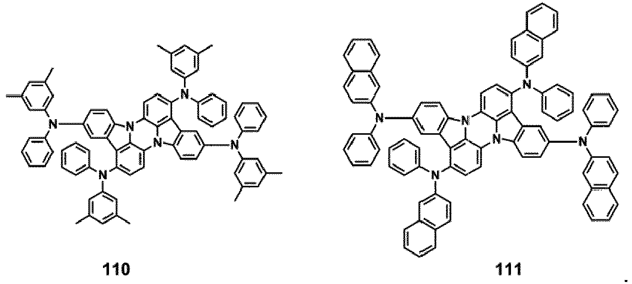

12. A light-emitting device comprising:
a first electrode;
a second electrode facing the first electrode;
an interlayer between the first electrode and the second electrode and comprising an emission layer; and
at least one heterocyclic compound of any one of claims 1 to 11.

13. The light-emitting device of claim 12, wherein
the first electrode is an anode,
the second electrode is a cathode,
the interlayer comprises:
the at least one heterocyclic compound;
a hole transport region between the first electrode and the emission layer; and
an electron transport region between the emission layer and the second electrode,
the hole transport region comprises a hole injection layer, a hole transport layer, an emission auxiliary layer, an electron-blocking layer, or a combination thereof, and
the electron transport region comprises a hole-blocking layer, an electron transport layer, an electron injection layer, or a combination thereof.

14. The light-emitting device of claim 12 or claim 13, wherein
the emission layer comprises the at least one heterocyclic compound; and/or
wherein the emission layer further comprises a host, and
an amount of the at least one heterocyclic compound is in a range of 0.01 parts by weight to 49.99 parts by weight, based on 100 parts by weight of the emission layer; and/or
wherein the emission layer further comprises an anthracene-based compound; and/or
wherein the emission layer is configured to emit light having a maximum emission wavelength in a range of 400 nm to 500 nm.

15. An electronic apparatus comprising the light-emitting device of any one of claims 12 to 14; optionally
further comprising:
a thin-film transistor, wherein
the thin-film transistor comprises a source electrode and a drain electrode, and
the first electrode of the light-emitting device is electrically connected to at least one of the source electrode and the drain electrode; and/or
further comprising:
a color filter, a quantum dot color conversion layer, a color conversion layer, a touch screen layer, a polarizing layer, or a combination thereof.
